# EUROPEAN PATENT SPECIFICATION

(11) **EP 3 321 374 B1**
(45) Date of publication and mention of the grant of the patent: **01.04.2020**
(21) Application number: 16198459.6
(22) Date of filing: 11.11.2016
(51) Int. Cl.: C12Q 1/6883

(54) **SHAR-PEI AUTO INFLAMMATORY DISEASE IN SHAR-PEI DOGS**
AUTOINFLAMMATORISCHE SHAR-PEI-ERKRANKUNGEN BEI SHAR-PEI-HUNDEN
MALADIES AUTO-INFLAMMATOIRES DES CHIENS SHAR-PEI

(43) Date of publication of application: 16.05.2018
(73) Proprietor: Stiftung Tierärztliche Hochschule Hannover, 30559 Hannover (DE)
(72) Inventor: Distl, Ottmar, 30559 Hannover (DE); Metzger, Julia, 30173 Hannover (DE)
(74) Representative: Gramm, Lins & Partner Patent- und Rechtsanwälte PartGmbB

(56) References cited:
- EP-B1- 2 397 565
- M. OLSSON ET AL: "Absolute quantification reveals the stable transmission of a high copy number variant linked to autoinflammatory disease", BMC GENOMICS, vol. 17, no. 1, 23 April 2016 (2016-04-23) , XP055369967, DOI: 10.1186/s12864-016-2619-0
- ANONYMOUS: "SNPs in coding region of Gene ID 609080", dbSNP at NCBI , 5 May 2017 (2017-05-05), XP055370864, Retrieved from the Internet: URL:https://www.ncbi.nlm.nih.gov/SNP/snp_r ef.cgi?chooseRs=coding&locusId=609080&mrna =XM_846283.3&ctg=NW_003726082.1&prot=XP_85 1376.1&orien=forward&refresh=refresh [retrieved on 2017-05-05]
- Anonymous: "Reference SNP (refSNP) Cluster Report: rs852257732", dbSNP, 13 October 2015 (2015-10-13), XP055370866, Retrieved from the Internet: URL:https://www.ncbi.nlm.nih.gov/projects/ SNP/snp_ref.cgi?rs=852257732 [retrieved on 2017-05-09]
- DATABASE Geneseq [Online] 10 July 2008 (2008-07-10), "Canine osteoarthritis related microarray probe, SEQ ID 21426.", XP055370982, retrieved from EBI accession no. GSN:AQF54186 Database accession no. AQF54186
- DATABASE EMBL [Online] 18 August 2008 (2008-08-18), "Sequence 1196 from patent US 7374927.", XP055370987, retrieved from EBI accession no. EM_PAT:EA781429 Database accession no. EA781429
- ANNA-MAJA MOLIN ET AL: "Genome-wide copy number variant discovery in dogs using the CanineHD genotyping array", BMC GENOMICS, BIOMED CENTRAL LTD, LONDON, UK, vol. 15, no. 1, 19 March 2014 (2014-03-19) , page 210, XP021182721, ISSN: 1471-2164, DOI: 10.1186/1471-2164-15-210
- JULIA METZGER ET AL: "Whole genome sequencing identifies missense mutation in MTBP in Shar-Pei affected with Autoinflammatory Disease (SPAID)", BMC GENOMICS, vol. 18, no. 1, 4 May 2017 (2017-05-04), XP055370019, DOI: 10.1186/s12864-017-3737-z

### SEQUENCE LISTING

<110> Stiftung Tieraerztliche Hochschule Hannover
<120> Shar-Pei auto inflammatory disease in Shar-Pei dogs
<130> 3064-008 EP-1
<160> 7
<170> PatentIn version 3.5
<210> 1
   <211> 70973
   <212> DNA
   <213> shar-pei dog
<400> 1
   agtggagggg aaccggaagt acgtcagaag cgcgcgactg tttggatgtg gaaagcggga      60
   cttgagaccc cagttgagct ccaaggagat ggatcggtac ctgctcctcg tgatctgggg     120
   agaaggaaaa gtcccgtccg cggcgggcgg ggaggcggag tcggccgccg agggtaccga     180
   gaagctgccc ggtaagcgcg ggactgagaa agagcgggaa cggcttgcgg ggaggggaag     240
   acattggaga tcgagtttct gtaagggagg gaactgttat ctcgagctct cccgggcatc     300
   ggcttctcta cggaggcggc ccagtaaggg ctgggaatgt cgttaccttc atggaaaaca     360
   tagggataaa ttcgtgaatg cagaattcca gcggtcagaa gttcttggca cagtagaact     420
   ttaacgggac ggtgggatgg aatgtcagct tggaaatgcg tgcgttcggg gagcgtttcc     480
   gcctcctttg tgttccagat gcgggtagtg cgcaagggaa cgcgaggccg aagggtctga     540
   ctctcttaga gtttacagcc tgatgggagg agacatccca cgcataaact aataaaataa     600
   acaaagctaa taaaaaaagg tgactttaaa acagttgtaa ccgtcggaga atacactcct     660
   tttcggtgtt tctgtagctg gtattgcatt ttgttttaat tgagcaggca tttttaaagg     720
   attttattta tttattgatg agagacacag agagaggcag agacacaggc agggggagaa      780
   gcaggctcca tgcagggagc ccgacgtggg actcgatcct gggtctccag gatcacaccc     840
   cgggctgaag gcaggtgcta aaccgctgag ccaccgggct gccctgagca ggcattttga     900
   ggagaaaatg gatgtggttg tattgcagaa tagagtttcc cttctccgct taaaattatt     960
   cttcctgggg agaattttga atttctgtta actggctagc atgtacaaac tgggcgcctt    1020
   tccggcttcc tctgattgca ttctgtttac catattgaaa ctagaagagc gtgtggttct    1080
   tctacataca acataacgtg cttgggagtc tcaaacggtg aaattgggat aggcctcgat    1140
   cacataactc ttgagcccgt tgagcgagtg tgtctttttt cttttccaga cctgacagca    1200
   gcaaatgttt atcatgtcct gaaaagaagc attagtgctt caatgaatcc agaagatagt    1260
   acttttccag gtgagtatga cgcaaacccc tttcttctct ggcactgcat ttgtattgga    1320
   aataaattta attagattga tcatttaaaa gtgtaagtac aaatatttgt tggtggtgca    1380
   caagtcactg ggctagggag gtaagttagg aaatacaaaa ctgtaaaaga ctcttcctac 1440
   tgttcgaatg taattaaatt atatgaaaat tattttatat tctcagcttg acttttgttc 1500
   cctttatggg attttcttgc ccacattacc tccgtataaa atagggtaat gtggcattat 1560
   aaggaggatt tacgaattct taccattttt ccccaccctt ccttcttaat gattctctta 1620
   aaagcacttc ataattattt tttttaagat ttttttattt ctttattcat gagagacaca 1680
   gattgagaga gagaggcaga gacacaggca gagggagaag caggctccct gcagggagcc 1740
   caatgtggga ctcaatcctg ggactcctga agtcgtgcaa aaccgctgag ccacccaggg 1800
   atccccagca cttcataatt attaaacaca gtgatctttt ctccatctaa cttaagatat 1860
   agagtaagca tctcttctat gttttggtga attgccatac tcctaagttt atattaggtt 1920
   ccggtgtttt atcctttgca ctttgaaagt catgaaatat ctctgagttc ctttaatgca 1980
   gaggtatttg ccagtgtcat tttctctgga gacctcttca tcctttttgt cacaatgttt 2040
   ttatcatagc aactatttat agaatccatt tacattagat ttaaatatca ctttcagtgt 2100
   tatcacttcc tggttttttg ctatactttc atcttttttt tttcttttgc ccattgcctc 2160
   ttcctattat ccatttttgt aaaatacatg gatttaccac cagaagacaa gaaggaaaca 2220
   caattaatta cctatttgtc agctattcac atgaagtgaa tggcatgcac aggaccactc 2280
   aacaaacttg gaaagcagta atgtgattgg tcactgatca cgatacacat ctttgattta 2340
   ggtagtgatt tgcgaagcta gcagtgacat tttactttag gtgattacag ttcattaata 2400
   aactttggta actggagttt gaaccttgtt ttgcggaatt attgaaacca tggtaattga 2460
   aatctgcata tcagaaccgt gcaaagaaag aattgcctgg ggcagcccgg gtggctcagc 2520
   agtttggcac ccagagcctg cctgatcttg gagacctggg atcgagtccc acgtcaggct 2580
   ccctgcatgg agcctgcttc tccctctgcc tgtgtctctg cctctctctc tctctttctg 2640
   tgtctcttat gaataaatga gtaaaatctt aaaaaaaaaa aagaaagaaa gaactgccca 2700
   tattgaatta attactcagt gaacattatt gctttcagta atatggcaag tccgggaaac 2760
   tggaagtgca atatgattat aattgcgatc aggctggaga ggtaggcata gctatatcga 2820
   ggactttgta aatcatatcg gagtgtttat actttttttg taataataat tagccattaa 2880
   aggctttaat gaaggaaggg acctggtgac atctatgtta atgacatatt ctagctacag 2940
   tatagcagac aggttggaag aaagtataga tatgaggggg ctgcttcaaa agaccagcta 3000
   agaaataaga gaggcaatca ggatagagaa gaatagatag atctgaagga aactataaag 3060
   gtaaaagcta cctgacttag tgttgcattg gtgtagttta ggagaaggga agagtgagaa 3120
   agcaatcagg agtgacatta gatttctggc acggttatag tgtagttggt ggcatttagt 3180
   gaggtaagag acttgaattt tgagggacta tcacatgtcc agtttagaac atataagcac 3240
   actaaacagt tccctgtaag atgtcaaagt gaaaatattt tataaaagtt cgatacatgt 3300
   gtccagaact caaaaggctg gagattaaaa aaaaattaca aaattatcaa taactagaag 3360
   ccataaagaa aatattttat aaaagttgga tacatgtgtc cagaactcaa aaggctggag 3420
   attaaaaaaa ttttttttaa attatcaata actagaagcc ataaagaagg atgagattat 3480
   ttatggacca tgaacagagc agaaaatctt gtactgaatc ctagtattta aagaaactga 3540
   actgagcact ggttgttaaa ggcatgagga gcagctggaa agataggttc caccgaagcc 3600
   aagtgtttta aggagaaaga tgacaactgg gtcatctttg attcctttct tactcttctg 3660
   ctgagagaga ggacaggtac aacagcagta agagctgtca catttagtaa catcttaatt 3720
   attggtgcct ttagggagtg tagttatagt ggcatgttgg agataaacag gtggaaatga 3780
   aggaggagag taggaaatgt gatgaaatag aaacagtcaa gtgtagacag tactttcatt 3840
   ttacccttga agaagagtaa agtagtagta gtgcctagaa gagggaggtg atttcaagga 3900
   gacttttttt tttttcctct actaatttga ggaatatttg tgcaaattta agtgctaata 3960
   ggaaggagct gacagaagag gagagcttga agatgcaaga aagatagtct aatcagggcc 4020
   ttggggtccc agtggaagtc tgaaggatga ctttggattg aaagggcaat acccctcatg 4080
   tgctaacaga aaggaatgag gaaggactta atgggaatgg gaatagtgtc ctatatttgt 4140
   tgacaggatg tttgggagta gttacctaat aatggtttct attttcttaa ggaagtagga 4200
   ggtaaagtct tttgctgaga ttgttggcag aaagtgataa tgaagcacgt agcatattac 4260
   ctagctttca tgagttaagc ttcagttacc atatttctct tccctccttt gaattcaatt 4320
   tctttacaca gttctacatt ctttgtggct ctaacctgac atgcactcta gggatgaccc 4380
   ttaacttcat atcctcatat aactttccgg tctccaagaa gagaatcttc tctatgtctg 4440
   agctgtggtt ttatttcctc ctctcagtcg ccaggatgaa ccagacattc atggtcttta 4500
   aagagggaat cctttttcct aaaatgactt acagttttgt gtcactcagt agtaattgag 4560
   tgagtatatt ttttttggta ggttttagtg gttaaaatga tgaataaaat agtacagatt 4620
   ttgattttga caaaattatt cattgtaagg gaataatttt gctctttagg cagattgttc 4680
   tgtttgaatt tttagagatt ttaaaaagat ctctctatat atgtatatgc atatatgtac 4740
   acacacatat ctggaggact gtataatatg taacaaaaat tatgtaattc tctaagtata 4800
   tatgctataa catctgctta tttgtggtat gtcttctaat aagcatggtt ttgttttcaa 4860
   gcatgttcgg taggcggtat acctggttcc aagaagtggt tctttgcagt acaggcaata 4920
   tatggatttt atcaggtaac agaactgaaa aaataggtgt tagtgaatgt ctttgttaat 4980
   ataaataatt tcaagaatcc attatttaat gcaatcttct gatgtattag ttttgcagtt 5040
   ctgactggga agagatatat tttggtacag aaaaagatga aattgaagat gttcttcaaa 5100
   caaatattga agaatgtttg aatactgagt gttttgagga agaagatagt aatagcagag 5160
   aatcattatc tttggctgag tatgcttatg atatgtactt tctatgatat ctttctataa 5220
   tatctttaaa atatttaatg ttagtatttt aaccacaaga gtgaagatta aatgttttaa 5280
   cagctatcag aaagaaattt tagttaattg aaattttcaa cccttcatct tggaagacag 5340
   gcaatttgaa attggaactt taatttggaa ttctatgtga gctatgatct caagttactg 5400
   gctctttcca acttaaaaat ttatattgtt aaagcacctt ggcttagaaa attttaagtg 5460
   tttataccta caacaactgt ctaaaaaaaa tgaactgtgc aattcttgtc attaaaaaaa 5520
   aaaatctgaa ctctcccaaa tatgacttgt tagtttctgt gtatttcctg ccagtgtaaa 5580
   tgtgaaaagc tttgcttgca ttacgtttta gaaatgcatt ttgcacactt gaattttgct 5640
   gaagctccat gaaaaggtta gatctaagta gatgaatgaa gctacacaca tgttttaaaa 5700
   gttaaaaaaa gaaacaaaac tagttgaata aaactattta aaatggaaaa attggcatct 5760
   gaaaacgttt agttttttaa gcttacagaa ttgcaggaat cggaaaaagt gatactccaa 5820
   ttaatacgtc actttcttaa agataaattc tacttgatta tgagttttaa gagcttgaat 5880
   aaaaacttta tttaagatta ataactggta ttcaaactca gaccctaaaa gtatgctttt 5940
   aattttgctg tttgaggtcg aaaaacacga tttctgcaat ttatgtatat cttgaacatt 6000
   tagacacttt taaaatgcat agtttctgag gattatatag gtgattggat tcactcagat 6060
   tttttgaatt gtggtacaat atataagttg gtctaggtga tataaattgt gattattttt 6120
   ttcatgacat atcccgtctt tgactcaaat taataccttt cattttagtc tctcatagta 6180
   ttttattaca aaaacctact tcggtttatt gtagtattca catattatgt ttttataaat 6240
   tactgcagac tttaaaaatt cttttttccc gttttcaaat tacatatgtt tgtataatta 6300
   atgtaagaag acattgtaga actgaggaaa aggtattgaa gggtcattaa aaggtagttt 6360
   ctagtttctt gatcgtgagc cagtcacttc atttctccac tcgtttattt tctcatcagt 6420
   aaagtgttga gatcctttca ggtcatttct aagctgcctt ctagccttag agttcttaaa 6480
   atcccatgtt tgcccaccat tatttaaaga tgacaagtgt aaaggtaatt atttctagta 6540
   agttacctaa taattatttc cacaagaggg agcttggaaa ctataccata agatgcctta 6600
   agtttcctag acaatggcag gttaataaca aaggatttaa gtaactttat gttacataat 6660
   aacattttga gggaaagaaa tcatcatttg gataaaatga aaagattttg aagaattgga 6720
   ggacaccatt ccagccagag ctgttttatg ccatacagag aaaagcagta tccttctcta 6780
   ttaaaccttt ggcaatactt ttagggttta taaaaatgtt tggtttatta gttattattt 6840
   atgtttaatt acactaggta taaattagag tgttttttcc cccttttttg ttatttgttt 6900
   ttgagggtat ggctaaaacc ttggactcag tgtttgaaac ctagcttcta cacctaatta 6960
   tgtgactgac acaaatcatt taaaggatat taatagtagt gcctcatgga ttgtcatgaa 7020
   gaacaaataa gatgatatat ctaacataat agtgtctggc acataggtct gtttaatatt 7080
   actaattatg attatatgta atgtgtatgt gtgtgtattt ttctaacttt cccttttatt 7140
   ttaggttata tgaagaatct gcagaaaatt tgcatcagtt gtcagataaa cttccggctc 7200
   ctggtaatat tttatgactt ttttcaataa tttctacttt atcttatact ttctatgaat 7260
   gactgatgaa tttaatcttt atgctagtgt tctcatttct aattttgaag ttcaaaatga 7320
   aaaaaaaatt ttttttagag gaagggaagg gcagaaagag cgggagagag agattcttaa 7380
   gcagacttca tttccagtgt ggagcccaat gcaggactcc atctcacgac cctgagatca 7440
   tgaccagagc tgaaatcaag tgtcagatac tcaactgact gagccacaca ggcaccctga 7500
   aaaataattt tttaattgaa gttgataaaa aacacactcc actactttaa taaagtatat 7560
   tactgagaca ttacaattct cttaattgtt tttatttagt taggcaaaaa ttagaaatta 7620
   gaaaattaaa tacccatctc agtttcctta tttagttatt gaagaatgtt tccactatat 7680
   accattgttt tccacagtat attctataga acactagtcc tatgaacacc tcctggagaa 7740
   ataacttctt taacctagat catttggata acattcttgt aaggtgtgag gtatatatac 7800
   gttatcaaat agaaagttat atagttaaaa aagcccttta ctcagccttt tttttttttt 7860
   ttacagagcc catttttttt atataatgaa aagcatattt tgtgaaatat tgcatcatac 7920
   tatttttaag atgtattctg tctcaaattt atctttcttt gtgccaaaga gctgacatct 7980
   aaagcttggt atagtttggc atctataaat acttttgaag agctttcttt gaatatttgc 8040
   atcaccgaag gtcatttatt aagcagatac ttgttgcatc tattatatgt catgtattgt 8100
   acaagttgct taagtatata atgattggta aagcatacgt gattaccatt gaagtacttg 8160
   atacctcaca gtctaatcct tatattcaag atcctgtttt agtgtaaagg tcccatatgg 8220
   aaaataactg aatacataaa taagcatcaa gtttttatat tttatttatt tatttattta 8280
   tttataaatg aaatatgtca aattcaaaat attcactgaa atatatagtt gctgttttgg 8340
   cagattagaa tgctttcatt atcaaatttc tatttggttt ctgtttcatt tatattttct 8400
   tttttttttt tttttttcat ttatattttc ttggtccttt gataaattta taatttttta 8460
   aaatgtgaag ttttatgaag ttatactttt ttcttaggaa ttaatgatat tgccaatata 8520
   attgagtttt ctgagtgttt aattgctcta tataaagaaa aaatttattt gaacattaca 8580
   attttatgtt gtcattccct tttgtataaa ttaagtatag agaaatatac tgaaattgct 8640
   cagttttttt tttttttttt ttttttcctc tgtttggtat acagtagcat tgatgatctc 8700
   aagagcaata tatataatta agcactggtg catagttggc aagtaattat gatagagttc 8760
   attattttct ggcatatact atttcaggat aaaatggctt taagtcaaat gatgaacatt 8820
   taaaaatttt gaacatctaa agatgtgtat tattttgaag aatttcagaa attaagttaa 8880
   aatcctttat cttctgaata aatataaaat tcaattgggg gtactatttt gagatttctg 8940
   aaaaaaaatt atggaaatat cttattctct ttctaggtag agcaatgata gatataatac 9000
   tgttgccttc tgacaaagat cctcctaagt tgaaagactg tttacctact ataggagcat 9060
   taaaacattt gaaggaatgg tattcagcaa aaattactat agcaggaaat cattgtgaaa 9120
   tgtaagttgc ttcgttcata tttgctgtgt tatctgtttt tacagttaac atgctatctt 9180
   gttttttaat agatttccca tttgaagatc tttttaatgt gacaagatag ttttatgtca 9240
   tcctgttagt agaggtttat ctattaagaa gaatctagat ttttcttatt tcatactttg 9300
   tttctgagat atttaagttt ttaggaaata tcaatgtttt ataaccatta ttgagcatta 9360
   attatctaaa tacactgtgt gcttttaaat aggtttgtat tgtccattga gcactatgat 9420
   gaatactatg gaacatggat acactggatt tatttttttg ttttctgtgc aatgtagaaa 9480
   caagtataca ccattgtgtt ttgtgctatt ttagtaagga tcaattgtat attttgatcc 9540
   ttaaagaaat actcatttgg atttattaac ttcatgattt tcttcattta acaaatgttt 9600
   atgaagtgct aactttaggc caagcacagc atgatggatt aagaaagtga gatttgatct 9660
   tgttcatgga aatacaagga gaaataagaa tgctgttagt catgatcatc tatgataagt 9720
   gctattatgg aattccatat gaaactgtgt gggaaaaatt taggtgagca gtcctttaaa 9780
   tataaaggac tgatactata tgtgtatgta tatatgtgca tatctgacac atttaagtca 9840
   gtaaatagta ataggaatat ctgttccttc atctgtatta ttaatggtaa ttcctctttt 9900
   cagggatatt gagattgaat ggacatataa tagtgagtcc ctcagagact gacttaagga 9960
   atgttctata atgttagtag ataaaagcag caatagaaac tgcatttttg cttgttagga 10020
   ttaaatttaa ggaagatata aaaccctgta tttaatcatc atagtctttt atttatagaa 10080
   attgtcagaa aattgcagaa tacctctctg ctaatgttgt atctttagaa gatctcaaaa 10140
   atgctattga ctcaaaagaa ttatggaggg gaaaaattca gatatgggaa agaaaggtaa 10200
   gtggatttct gttatcactg tttgcaaagc aggcctttca actttattta caacactgat 10260
   agtttaaatg aatttgaatg aaaatgtacc ttataaagat gttctagaat agcactaaga 10320
   attttctgcc ataatggaaa tgttttgcct gtcctgatag tcactggcta ttgagtactg 10380
   agttatattg ctactgagca cttttttaaa aactattttt tttatttgag agagagagtg 10440
   agtgagagaa agagagcagc atgagcaggg ggcaggagaa gcagattccc cactaagcag 10500
   ggagctcaag atagatgtca taatagttcc tatctcatag atttttatga aggttaaatt 10560
   atgtaaagta gaaagtaacc actcattaaa cattagtttc attgttatca ttactattaa 10620
   cacccctaat atttctgctt ctgcagaaat tgatcttttc atcagcgact tattttgaaa 10680
   gcaaactatg tgtggggtct tgtcctaaag atgtgtttaa ataagacaga caagctctgt 10740
   tagtgcagaa tttgcagatt ggtgagaagc cagatattga acaagtaaat gtaaaggaga 10800
   ttgtttggct ctatggttac acagaatgag cttattaata cactcttgtg gggtggggta 10860
   ggaccaagga aagtttttca tgaaaagatc catgggtaag ttgaaatagg aaggatgtgt 10920
   acataaatgg aagaaccaca ttttgagctg aggaatcaaa aatatatttc cttgtggttt 10980
   tcttctcaac tttttcattg cagtcagatg tattcatctt tgtaacttta ttcatgctgt 11040
   ttatacttgt gttctcttgg tttcttttca gtttggagtt gaaattagtt ttcctgaatt 11100
   ttgtttaaag ggagttacac ccaagaattt tagtacatct aatttaaatc cttgcattct 11160
   aaccaacaag gtaataccat caaaggataa gaatattttg ccaaaggtaa actatgttta 11220
   attcattttg caatcatcca ttcgtgcata tttttgttct tataagatgt ttgaatcttg 11280
   aaactaatat atatacttaa atatgctcgc ttgcataatt tttataatta taattaagtg 11340
   cttcaattat attatgttac cagcattgaa tgttggcaaa actatcatta ttttagatta 11400
   ttttttaaat ggtggaatga tcctaacttt agatcagtaa acttcttttg gttaggtctt 11460
   tgctatgaga cctttctaag actggattgg agggaatatg atacactgga aagggaatag 11520
   gctttgatgt taggtagacc tgggtttgaa atctggcact gtcactgaca aggtattgta 11580
   acctgggtag attgaataac tcatttgtga aatgaagaca ataacatatt taccttacga 11640
   agttattaca aaagttaaat gaagaaagag gttacagtat ctgacatttg cttcattcac 11700
   agtttaggaa gtgttagttt gtgtcccata tcttcattca ttcttaaaat gcaaataatt 11760
   atgaaatgca atttaaaagg gtttagttgg ctttttttta acattggtaa aaacaacata 11820
   agggggtatt tgtataaaag cactttagaa attcacttta aaagatacaa ttcaagaatg 11880
   tgcaaaaaca acagtcattg aagttacaag gatgtcagat tgatgggtaa tattttctga 11940
   aaagggggta ctaccaaaag gacatatttc tgatgcaaac acatttccca gtagttgttc 12000
   agaattgctt acttttaagt aacctgccat ggccatcttt cacaggaata ttacatattt 12060
   tctaaagatt ataagattga tgacctcagt gtctcttctt tcatagtata ccatgaataa 12120
   tttatttaaa ttttgctgct gttgacagga atcttagggt acaacctctt caataaatgt 12180
   ctctctctgc gtatacaggt atacacacac acatacatat actgtatatg tgtgtgtatg 12240
   tatttttgtc tatatatata cacatgatca catatggtga aatcactcca ttttctgccc 12300
   aaatctttgg gctgtcttct caaaatgtgt tagcttttct ttatcctttt tgtgaagtac 12360
   tgtggtctca actcctaaag cacctgtcct ccttcactag ctcagtgttg gcttagtttt 12420
   aggctctgcc caaagactgc aatctcattt ctctgagtgg agctcttggc ttcactagtt 12480
   ctcttaactt tttacctttt ttttcaaggt gtttttttgt ttgtttgttt tttaaatttc 12540
   agtataccac tcgatataag tttatcctgg agagttttct ttctcggtct tacaaaccct 12600
   tgaaatttgt ttcttcaaca tcttagcccc ttggatcacc tgtcatttta aagttttaat 12660
   ttgtaattta taaagtttta aattgtaatc actaatttgt taacttttat tttatttata 12720
   gaacttaaag tatcatgtaa tcttatttct ttcctaggtt taccattatt atggccctgc 12780
   tttagaattt gtgcagatga taaaattatc agatctaccc tcctgctata tgtcggactt 12840
   cgaatttgag ttgtatcctt ttctttacat gttcattttt agattacatt ttctgtgtca 12900
   ggtagactgt ttcctccata ttttgatatt tgactgtact ggggtttttt ctttaataat 12960
   attcagcata tttttaatta ccatggtttc aatggaaatc ctacaacttt caaaaagaaa 13020
   acctccttca agtactatac ttctgcttct aaaataggcc gaagactttc tggtattgct 13080
   attcattgct aatggggtag ggaggatgga gaaatgttag gaatatgtgc agaaaaacag 13140
   taatttatat acttctgaaa tgtttatatg gttctcttgt catcagcagg acagatatat 13200
   aagcaccagt ccactgtatc tttttatata tttaagtaaa tacaaatgct atctcatgta 13260
   tctgcaaata ttcaacattt ttaggctctc ttttaagaaa aggattttta gaaatacact 13320
   taacagaaca gaacattttg tataaaaaga aattgttgcc tggaaatgga acactcgcat 13380
   ggtgttcttt tgtgtagaaa ctacagtttt tacaaatctc tttttctgat agtttatagg 13440
   gctgctcttc atagaagcac tcttttcttg gtatattttc agcattagaa aaataattat 13500
   ctttaaggaa aggagtaatt atgaatttct acagttaaaa tttttaataa ataatctaag 13560
   aaccttgtga attagaggac caaacttgta ttagggagta gtattgtttt gtggttatca 13620
   ttcatcattt ctttaactcc ttagttcaga gggctgacaa aaaacaatac caaacagaat 13680
   tccgtgttgt tgttggagca gatttcctct ctgcgtggca aggtataggg tatttttggt 13740
   tttctttagt ttttaatata atgtttaaga atgagtgaaa aaaggaatat agtttgttaa 13800
   tctgttttgc ataataaata tacttcttta acataggatt ttggtcctaa ttgatattaa 13860
   gatactttgg acttcccata ataacaccaa agtcctctgc agacatgaaa tgatgttgag 13920
   tttttagaag agattttcaa aaataagctt catgactgac aatttcattt gttttaaaat 13980
   ttgaaaacac attattattg ttaatctact ccttcaacat ctcatttcag ttatgtgaga 14040
   agatattcat atgcttgcat tatctttttt gtggtaagac aggtgggtgg aagtaagaaa 14100
   aacttccaaa gccccccctg tattctcatt tggaagtttt cagtttttct ttcttaattc 14160
   tggtgatgtt tgtgagatat gtgtctattt cagtgtgtgt atatatatat attttaagtc 14220
   atgtatttgt ttttttgttt tttaaagatt ttatttattc atgagagaca cagagagaga 14280
   gaggcagaga cacaggcaga gggagaaaca ggctccatgc aaggagcccg atgtgggact 14340
   cgttcccagg tctccaggat cacaccctgg gctgaaggca ggcgctaaac cactgagcca 14400
   cccaggcacc ccaaagtcat ctatttgaag tcaagtatag ttgttaatgg gaaaaactaa 14460
   aagtttgctt ttctttctgg ttgtttaact actgtgtttc tatgaaaata attttttttt 14520
   ccataagaat tttatttatt tacttatgag agatacagag agagagaggg gtagagggag 14580
   aagcaggctc acatgaggga gcctgatgtg ggacttgatc ctgagagtcc aggatcatgg 14640
   cctgagctaa aggcagacac tcaaccactg agccccacct aggtgcccct atgaaaacaa 14700
   tttttttaaa agatttattt atttatttat gatagacata gagagagaga cagagacaca 14760
   ggcagaggga gaagcaggct ccatgccggg agcccgacgc aggatgctat cccgggaggc 14820
   caggatcacg ccctgggcca aaggcaggtg ctaaactgct gagccatgca gggatcccca 14880
   aaaacaattt ttaatgcaca gaatattcat tgacagtatt tacttaacat taccttactg 14940
   ttttaacaac agacaaacta tatcaaacaa ttctacaggg aggaaaatga ttattgtaat 15000
   tatagtttcc ccatggcact ataatcaaat attggttata attcccttag aaatttgata 15060
   cataaaggaa atatgatttt ggccttaaga tggttgacta tatggtaatt taaacttttg 15120
   actttacagt taagcatctg aatctcgatt ttggctcagg tcatgatctt agggttgtga 15180
   gagcaagccc tgcattgatt aggctccacc ctggaacgtg gagcctcctt aagattcccc 15240
   ctctcgccct tgcatgttct ctatcaaata aatcttaaaa tttgactttc agcctcttcc 15300
   ataaagttaa gttttgaata agtgtgccta agtgtttatt tgataaagta gcttttaaag 15360
   caatagcctg aagtataata ttagccaatg tttttatgct caaagtatat ctaatatgaa 15420
   atttaaaatt ttatgtttta tttgtctaac atgtatctta ttatgtgcta gacactgtgc 15480
   taaacattag ggatccagtg atggattgca cagatgagga ctgtcctcat ggaacttata 15540
   ttttggtggg aaaaataata aacactatga aaacaggagc ctgggtggct catttggtta 15600
   agtagccacc tcttgatttc agcgcaggtc atagtctcag gatcatgaga tccagcccca 15660
   tgtggggctc tgcagtaagt gtagagcctc cttaagattc tttcttcctc ttcttcaccc 15720
   tttacactcc cgctttctcc aaaacaaaca aaagaactct gaagaaaata agcagactac 15780
   aatagagtaa taacatggag gatctacttt aaacaaatta gcaagatcat acatctttac 15840
   aaaaatatgc acatagaagt cttgtgaagt tgtatagtta aaaaaaaaac aaagaattaa 15900
   taaaaatctg ttcctatttc aactcaagta actagatagt tctctgtgta accttatcta 15960
   attcacctaa ttttgggggg cctctttttt ctagaaaatg agaatatcat ttatcattag 16020
   tcacctcacc tgtgttatca gatagggaaa gggaggagta tgatagaata gaaagtctca 16080
   agtaagagtg tatgtacttc agaaaactta gatttaagtc ctagctagct ctaccactca 16140
   ctggctttaa actttttaaa attgtaaact ctaagtaata atccctgttt tattcatctc 16200
   aaaagttgca atgaaaacca gattggaata tttatattgt attttcaatt gtttcgtttt 16260
   aaatttgata tattagatta tcatttttat tgcttcttgg acaatgacaa tacaacattc 16320
   ttgggggcat ctgggtggct catttggctg agcacctgac tcttgatttc gactcagctc 16380
   atgatctcag tcatgggatt gagccctgcg ttgagttctg tgctcagcag agtcggcttc 16440
   gggttctctt tctccttctc cctctgtatc ctgcccctgc ttgcatgctt tcactctttg 16500
   tttctctaaa ataaataaat aaaacattta aagaaatatt agaatattct ttatgaataa 16560
   aagacaaata agtgatggaa ctttatatag atgtcctact ttaatgcagc aaaagggatg 16620
   atttttggaa acataattta gctaacatag ctctaaatgt gaagcactgt atcttccttt 16680
   tatattgggt cataattagt cttttacatt tttgaaaaag tacttttgat ttgtgtgctc 16740
   attctttgaa atacttctaa tgctttataa cttgagcttc acttatttat tcaacaattt 16800
   tgtgtatgat gctaggacag agactgcctg ttgtgtgtgc caggtacttg ttttagatcc 16860
   tggagagaat aaaatggaca aggcccctcc ttaggaagct ttcatagtac ttaaagtcta 16920
   gtgattatat gatcttaagt taaattctga ctttaaaaaa aaaaaaaaac tttgattagc 16980
   cccagaataa aaataaaaac cttttatagt aggttttaaa aataaaacct tttattagct 17040
   tactaaaaaa taagaggatt gttaagggaa catgatatct actaatcaat tgtatgacca 17100
   ggaagttttt tttattttgt ttcatttgtt taaaaaaaat ttttttttga gaaaaagagc 17160
   attagtggcg tgggtgaagg gtggtagtga tgagagggac agagggaaaa ggagagagac 17220
   tcttaaggag gctctacacc tagcttggaa cccactgcag ggcttgatct cacaaccctg 17280
   agatcatggc ctgagctgaa atcaagagtt ggatgcttaa cacactgagt catccaagca 17340
   ccctaggaaa tattttttta aggaaagagt ttgcaaagtt agaaacatga tcgccttagt 17400
   gcctgcatgt ttgggttgtg cctactgaaa agattgaatc aaggtttcaa aattcaggtt 17460
   tgaaatggta tttgattttt aaattttagg gatagaatga taacctagaa gccttatcct 17520
   caagtctcag agtgtatcta atgataagtg cgtgagaaca aattatcaga atcgaagttt 17580
   gttatgaacc cattataaac aattgacata aatgtattat aggaaattgt agcttatggg 17640
   atttagaaat atttattttc atatttatag caaatatctg ttcaagaata agctgtctca 17700
   gacatttggc tatttctttg gctgggtaag acatttataa tatcaggctt taagttgtgc 17760
   attgaactgc atttagcatc tctaatgtat taagtaataa ctataataat tatataatta 17820
   aggtataact ggccctgatc tagtatacag ctgtctatcc tttgtgctaa acacctttgt 17880
   tatgcatttt aggctatata gtcttactca tgcgggtatg aagaactaga atttagtgtt 17940
   tcaaaagtta gatcttttta tacttaatga ttatttactg ttgctaatat ctttcatttt 18000
   tagtgtcttc tacgttgtga aatacatttg acttagaata aaaagacatg gctgtaataa 18060
   caaagaaaaa taaacatatt taatttttcc tttttttgct cctaatatat ttatttgtat 18120
   atctaagaat acagcagata ataaatagca gtaaaactaa tatattaaag tattgactta 18180
   gattcatata tgtcatgaga ctcattcatc tacaaaggac aattaaaagt gatttttcat 18240
   tagatttaag atctataaaa tatcatactt aagtttcccc agagttaaaa aatgatattt 18300
   attgggattt gctgattgtt gaagcttaca tttctgacca aacaggcaaa atgtagcttt 18360
   caaagtatac tttgttttat ttcattttgt aatcgaattg agaatttacc taatataata 18420
   tgcgggatcc ctgggtgggc gcagcggttt agcgcctgcc tttggcccag ggcgcgatcc 18480
   tggagacccg ggatcgaatc ccacgtcggg ctcccagtgc atggagcctg cttctccctc 18540
   tgcttatgtc tctgcctctc tctctctctc tctctctctc tctctctctc tctctctctg 18600
   tgactatcat aaataaattt aaaaaaatta aaaaaaataa atattatatg ctggctttgg 18660
   gaaaaagaaa tatttattaa acatcttatg cccagccctg ggaatgcaaa gatgaatgag 18720
   ctgaattttc tcataaatca gttggaagct gctgtatgtt ctctggcata ggattcatgg 18780
   tttctttgca ttttatactt tagtagcatt aaatctctga tttctaggaa cagcaatttt 18840
   gaaattacct acaaatgtat tgtaattttg aaaccagaac aataatccat ccagtcaaaa 18900
   ggttataaaa ttatggctaa gtatgaaaaa atcttttgat gatggcattc ccagcctctc 18960
   tcactgtttg cagatgtaca aagaaatgac actacaacct gaacttaaag caaatatcgt 19020
   gaataacaag gttttcagaa atacataatt tttttttaaa gattttattt atttattcat 19080
   gagagataga gagagaggca gagatacagg cggagggaga agcaggctcc atgcagggag 19140
   cctcgtggga cttgatccgg ggtgggactc aatctcgggt ctccagtatc atgccctggg 19200
   ctgaaggcag cactaaactg ctgagccccc caggctgccc agaaatacat aacttaatgg 19260
   aaaacgctgt ctgtgtctga ctacctttgt ctggtttttt accttttttt ttcctaacag 19320
   tttgggtatt gtttttggtt ttccagtttt tacagtggac ttgtgagaga gtatgaaaaa 19380
   gtacctgtta tataggtgaa atttgagtgg aaaattgtca ggtgacacta tttaagaatt 19440
   aatttcagtg cgttaagtca ctaaaaatac gttctgtttt atctaggttg gtgcactttt 19500
   tgtattgccg tgtactatta gtaacataat tattccacct cccagccaac tcagctcaag 19560
   aaaatggaga gaatacatag ctaaaaagcc taagacaatc agtggtaagt agtaaatttt 19620
   taaggtgttt tataaaatgg agatgacatt ctttatgaga aaatattatt tattacttag 19680
   ttttaaaaca aagccacagc attttaagat tttttatctc taataaccta ctgttagtga 19740
   caacaaagta tatgctacct tatatattca aaaaaataac cagctatcta aaggcaattt 19800
   tgattaagta gatacctgat tatatcaaac atgggtttct tagagacatt tcaggaatga 19860
   aatttatgaa ataaagtagt taggtgtgcc tgggtggctc agttggttaa gcatctgcct 19920
   ttagctcagg tcaagatcct ggggtcctgg gatcgagccc tgcgttgggc tccctgctct 19980
   gtggggagtc tgcttctccc tctccttctt ccctgacctc cactcatgct cacatatgca 20040
   ctctctctct ctcactctgt caaataaata aatacaatct aaaaaaaaag aaaagaaagg 20100
   gattaattta tcatatggta cataaaacct tgtgattata gttttctgaa gaaatagaag 20160
   ttatttaaca attatatgct atgcagtgtt ctatattggc tcatttaatt cttatgatag 20220
   ccctataaat ttatttttat atccatttaa cagattcaga aattgggatt tgaagaagtt 20280
   acagagctgt taattggcag aatcttggtt caaatcctgg ctccgacagt taatagttct 20340
   gtaacttctc caaggatttg aacccaggct ttgttcttaa tcactgcact gtattgcatc 20400
   taatgcttta ttacattgaa aatgtcatta taaagatgaa acctgttgta aaatcacacc 20460
   atttatatcc aaactttagt attttttgag gtaactatgc ctcaaaatgt tagttgcttg 20520
   ccccagtcgt atagctaaat ggtgcttctt catagtttgc ttatatgtac ttggtttttt 20580
   ctgttgttgt ggttggacaa taattttttt aaattatctt tgagcttata gttagttttc 20640
   gtgtcccatg gtatgaaggg actaagatct ggaatggaaa cattgacctg gatcgttcta 20700
   gaagaagtct ctagaaagat tccagagcca tagaatcctg aggagtacaa attttatcgg 20760
   tagcttacac agtattatat aggtgagatg ctgaaatcca tcttagcaaa tgttgaattt 20820
   gaaatggaga tgatatctat atggaaattt aaatttagag tttgaaatta ggtctattat 20880
   gcctagagaa tggtcagggc tagagattta gattttgagg ttaccacctg ttttatttat 20940
   tttgtaacaa accagcctaa aactaagtag cttcaaaaag caaatgcttt attttttcat 21000
   tttattttta tttatttttt taaagatttt atttattcat gagagacata gagggagagg 21060
   cagactcata ggcagaggga agagcaggct ccctgcaggg accctgttgc agacttgatc 21120
   ccaggaccct gggatcatgc cttgagccaa aggcagacac tcaactgcta agccatccag 21180
   gtgtcccaaa gagcaaatgc tttttgaact aggtttacct gggtggattc tttttttttt 21240
   tttttttttt ttttaagatt ttatttattt tgagggggtg ggcaggggag agagcatgag 21300
   ccagagaagg ggcagaagga agggagaagc agactccccg ctgagcaagg agcttgctct 21360
   caggacacgg gaatcattac ctgagcgaag cctgaagctt gacccgctaa gccacccagg 21420
   cttccccacc tggatggttc ttctgccatt ctcaccaggg gtcactgatg cagctgcact 21480
   gagtttatgg attgttcaaa agttgaactc agcttgggat gcagtggctg ctatcttact 21540
   ttctccatat aatctcagag ccttttcctt gccccatgct ttcttcatgt gatctctagc 21600
   aggttagctg gaatttttcc atggaggctt aagactccaa aatgtacaaa aatcaaaact 21660
   tctaggactt ttaaggctta gtccctgatc tggcccagcg tcacttcgtt gttttttttg 21720
   ttttgtttta atatttttat ttattcattc atgagagaca gagaaagaga gagaggcaga 21780
   gacacaggca gagggagaag caggctccat gcagcaagcc agacatggga cttgatccca 21840
   ggtctccagg atcacgccct gggccgaagg ccaaactgct gagccatcca gggatccccc 21900
   agtgtcactt ctactgtatt cttttattta aagctactca caagaatggc ccaggttcat 21960
   agggggattg tgactagaca agggtgtgaa tgcagagaag cccccaatgt aaaagcctac 22020
   catgactgtg tgcaggtaat agttgatgcc ctaggagtgg ctagacaaaa ccacagcatg 22080
   actagagaag agagccaaag ctaggatctt cagaaatacc tgtgtttaag gtaaaagtga 22140
   aagaaaaatt aaaaagtagt ttagcagatg gtagttggaa gaccctaggg ggctataatc 22200
   attaacatca acaacaaaat accatttatt gcatattacc agtgtgggaa ataaagggga 22260
   gtcaaactgg tccttccact tgacaaagta gttgcacgat cccaatcaag tgatgaaggc 22320
   actttgaaaa atcatttttt actctgttta gtcaacagta tgaaggtgaa gtgaaagata 22380
   cttccagaat gtatgaccta acataattgt aagaattcag aacagcttcc tggaggaaca 22440
   ggtattgtta atatagtttg ttgcactgta ctagtacata aagaagtgcg gctttgaccc 22500
   tactcaactc ctcccaggga atagatgtat acatggtgat tttggcagat gctaaaacaa 22560
   aaacagaaat agatttgcta ggtggcagta gtcaatttca cttactttac atgaaatcct 22620
   aggataatat ttatccattt ctcaacatta cacgtgtgta aggcttacta tgaattgaat 22680
   attaatatct cctaattaaa cttgactgtg atctggacag cccattatta atttttcctt 22740
   tgtaactttt ataaaggtaa tttattgatt tagaatttat tcttaagtgg attttttccc 22800
   atgcttttca ttgcaacttt aagaaactac cttaaaaaaa gaaaaaaaga aactacctta 22860
   gcatatgact tgatttgatt tctttaaatt agcacctctt gaaatttgga gattaatatt 22920
   ggtaacttct gaataataag acatatatca catgacaatc ttccaaggca gattgttgat 22980
   tactgattag gctgtatcat taaatgctga ttatgctgct accttacagt attttttttt 23040
   ttatcttgga agtagaaaca atcaaattgg acagctgatt attaagttct attctaccca 23100
   tagccctttc tgtaggggaa attatttatc tcatgacaga actccttttg taaatgagga 23160
   gccaagtttt agctagcata aagttttaat agctcttact taggaaaaca tcgtatccca 23220
   ttatagtgcc actacaagta gcttctctca ctattctaaa gatatcaact attagatgaa 23280
   actcttaaat caatgttctg tgttcctgaa acttactaca ttatgtccag ctctgcttta 23340
   ctgttggctc tgcatctccc ctgtcgtcca tgctaaactg tatatggttc ctttaagcta 23400
   ttctctgatt ttaatccttt atgaccttat tgttcattca ttcattcatt cctttagtca 23460
   gtcacacctt tcattcattt ttttgagatt gagtaaatat tatgtatcag gcatggtaaa 23520
   ataatctgga gattagtaat gagcaaggta gacttggttc ctcctttcct ggaatttatt 23580
   tatttatttt tttaaagatt tatttattta cttatgatag acacagagag agggcgagag 23640
   gcagagacat aggcagaggg agaagcaggc tccatgctgg gaacctgacg cgggactcaa 23700
   tcctgggact ccaggattgc gccctgggcc aaaggcaggc gccaaaccgc tgagccaccc 23760
   agggatcctc tggaatttaa actataatag aaaatagata tcgaggggca cctgtgtggc 23820
   tcagtggttg agcatctgcc ttggctcatg ttgtgatctg gaagtcctgg gatcgagttc 23880
   tgcatcatgc tccctgcagg gagcctactt ctccctctgc ctatgtctct gcctctttct 23940
   ccatgtctct catgagtaaa taaataaaat cttaaaaaaa aaaagaaaga aatatcaaga 24000
   ggccattaca agtcttactt aattatacca ttgtattcaa tagcaaccaa ccataaatcc 24060
   aggagagata gtacttgtaa aaagttagat gtggaaagac tattatgcta acatttgtgg 24120
   taaaaacaac aatttggagt atgtgtaagc acttgtatgt atttgtttat ataaatagaa 24180
   gttttgcatt aaatatttgt ttatataaat agaagttttg cattaaatag cagatattat 24240
   tatcgaatat ccacagtaca gtaggcacta ttacacatgc tggaccaaca gtgaatagaa 24300
   caaagtccat gttctcagga agcttacata actgtagagg taatgagttt agactttacc 24360
   ccagaagcca tatggctagg cctctcagtt ttgaggggcc tttactccag tcattttctc 24420
   attcaggacc taatttgcca tcccatcgaa aattgcaaac agactcttct cctgacttta 24480
   tatccatttc tccaacttta ttttttttcc tgttagtact taccagacct aatatagtag 24540
   gtgtcttttt ctctcactca aagccagagg agacaagatg gagtttttta tttttattca 24600
   tttctttatt ttcagtgcct ggaagagtgc ctgatactta ttaggtactc aaaaatagtt 24660
   gttaagtaat acatgagatt tatatttcca ggtagtgata attgttgtct ggaaaagtaa 24720
   aaaaattttg aatggattcc acccatacag tgtgaatcaa aatatttgcc ctagtttttg 24780
   tcagttcctt aggtttaaca aatcatctct gatgaaataa catacatata tatattaatt 24840
   tgaaccatat ggtattgctg tacttgtata tcaaaaattg ttaggtatca gcagttgcat 24900
   atggtttgac ctaaaataga aaaacaatgt tccaactgaa ttatgcttaa attaaacata 24960
   gccaaagaat gatttttaac agtaattttc agtgaaatag ccagaaagta ttatgatact 25020
   aagaaaaatg caaaagaact atgtgaacat gggacgcctg ggtggctcag tggttgagca 25080
   tctgcctttg gctcaggtca tgatccctag gtcacgggat cgagtctcac atcaggctcc 25140
   ctgtgaggaa cctgcttccc cctctgccta tgtctctgcc tctctctgtc tctctcatga 25200
   ataaataaat aaaatcttta aaaacaaaaa cataactgta tgaacaaatg caacaattac 25260
   tctgaagaaa ggcttctata tgtaaaactt gtactgaaaa gccagtagtg taatgaaaag 25320
   tgcctggctt accatatgtt cacagagaag ttttacagaa ttgtcaaaag attcaatatt 25380
   gatcattgcc taactataca ttacaaatga ataatctgaa atatagagaa atcaagaaac 25440
   ttctctaata tcacagggta caatcagaat tttcacccaa gtctgaatgt atccacagac 25500
   catccagata attttacaat cttgagttct attgttgtct catttttttt aattggaatt 25560
   cttttgaaag aaaatatttt aaaggtactc ttgaaaaaag ttactgtttt gttgacagac 25620
   aagtgtcttc tagtaaaaga atttttaaag ggactagatt aataaggact aatgaaatgt 25680
   tttgaattga tgaatcatgt gccttcttga ttctcaaatc agtgtgataa aagtgtttca 25740
   aaataatgag ctgaaactaa taaggttaaa atgaaatgac actgcattca gggcattatt 25800
   gcagatggta ggacttctgg taatgaacac tgtcagccaa ctttactgtg ttaactttta 25860
   ttgaatacac cattcaatgg cgtcctcgtt gtctttaagc atatatagaa tgttccccag 25920
   gatagaccat gtatatatta agtgattaac taaggctaat aaatttaaaa gggtagaaat 25980
   aatgcccagt atgttcttaa attacaatag aattaaatta gaagttagca aataccaacg 26040
   tgaggaatac tttaaatatt tggaaattag actcttttaa ataacccata ggtcaaagaa 26100
   gaaaccaaag gggaaattag aaaatgtttt taactaatga aaccaaaaca tatcaaatta 26160
   tggatagcat gtaagtcagt actaagaaga tcataaatgc ttatcacaga agaaagatct 26220
   aaaattagta atccaaacat gaacctcagg aagctagaag actaattcca agttaattag 26280
   gaagaaggaa tgagaccaga agtcagtgaa taaaatgctg aagactacat ctaataaagg 26340
   aagtgcaaga tctgtacact gaaaactaca aaacttactg agagtaataa agaaaataga 26400
   agtaaatgaa aagatacacc gtgtttgtaa attgggaagc tctatattaa gatggtactt 26460
   ccctctgaat tgacccatag atttaattca tctctaacaa aatcccaaga ggacttttca 26520
   tagaaattga caaactgata caaaatttat atggaattgc aaaggtctta gaatggctaa 26580
   acaatgttga aaaggaagaa tgacttttgg gaacttacac tacctgattc acaacttcac 26640
   agctataaag ccacagtatt caagacagtg tggtattgaa tgaagaacat acatgtatca 26700
   atggaacagt atagaaataa agatatcaat gggaaaaagt ctccttaacg cagtgctgaa 26760
   agcactggat atccatatgt aagattttta tgtaaaaatt atctctaaat ggactatata 26820
   actaaatgca agcattaaaa ctataaaaca cctgtaagaa aatatatcaa ccaaaaggat 26880
   ctataaaaag aaaaaaatct ataaattcac ccaaactaaa aatgtctact ttcaaaaaaa 26940
   aaacactaag aaaaattatt tattttttta attttatttt ttaaaagatt tatttattta 27000
   ttttagagag agctcatgtg tgcaggagca gaggaagggg cacagggaaa gggaaacaag 27060
   cagtctccct gctgagcatg gagcctgacc tgggtttgat ccctgaaccc tgagatcatg 27120
   atgtgagtca aaatcaagaa ttggatgctt aacctcctga gccacccagg caccccagaa 27180
   aaaaatttta aaaactgcta agaaaaaaca tttcctattt ttttgttttt tattttcctt 27240
   ctttatttga gagagagaga aaacaagaaa tggggagggc cagagggaga gagaaagaat 27300
   gtgaagcaga ctccctgctg agggggagcc caatgtgagg cttgatccca ggaccctgag 27360
   attcatgacc tgagctgaag gcaggtgctt aaccaactgg ggcacccaga tacatcattt 27420
   ccagtcatgt caacagtgga cagatacata aaaaaaaaaa ttcttgcaac ccaattaata 27480
   ggtaagagac ttgagtagac atttcattaa gatacatgaa tagctaataa agtacatgaa 27540
   agatgtttaa catcattagt cttctgggaa atgcaaatta aaaccacaat gcaattctat 27600
   aatacgctta ctagaatggg tattattaaa aacagtgact atcaaatgtt gatgaggatg 27660
   tgaagaaact gcaaatctca cacatagctg gtgatggtgt aaaatggcat agatactctg 27720
   gaaaacagtg tagcggttaa acttacactt aactatgcaa ctcagtattt ctattcctag 27780
   gtatttatcc aggagaaatg agaacatatg ttcacacaaa cacttgcaag taactgttaa 27840
   taagagccat atttttagta gctaagaact ggaaactatg caaatatcca tgtgaataga 27900
   taaacacagt ttgttttgtc catacagtgg aatattgctt aacatattga ggcatgacaa 27960
   attttgggtg tacctaaata tggtaagttt atatggaatt tctataaaaa gctaaaggtc 28020
   cagaaggaag atcagtggtt gcctaaggcc aggaatagga ggggagattg gctgcagaaa 28080
   aataatggga gttttccaaa actggattat agtgatggat gcataattat ataatttcct 28140
   taagttaatc acatttatac tttgatatgt gtacattata cctcattaaa actttttgtg 28200
   agagagagat ggaaggaaag agagaggcaa ggaggtaagg caggaaggaa ggaaggtcaa 28260
   aaaggaagaa aatctagtgg ggtcctacct gaaaacaatt aaatacaaat ttctggggag 28320
   gaaaccaggc cctcaatagc ttgtaaaagc actcaagtgt ttcaagtgca tccagcatta 28380
   agaacttctg ccctagatgt gcttttttca aaaccctagg ctagagagcc actgttactg 28440
   aagagaaagt ccttaagatg ctctgagaca aaaaagaatc actatcttag gcttttaatt 28500
   ctttggttag aaaatcagga tagtaatact tgcttagtgt atctcacaaa cttgtttgaa 28560
   tcacagttgg tgatgaaggt agacactttg taaactctgc agtagctttg ttattctgtc 28620
   aatctttttt tttttttttt tttttttttt ttattttttt ttatattctg tcaatcttaa 28680
   aggatgtcta tatctatgga acagtaaaga aatccttgtt cttcatgatt aaaagatctt 28740
   gaaatatctt ctgaagtttg tctattcatt gtagctttca aattgcctat tttactatgt 28800
   tgtaactaaa gggaaatata taaagtttat gatcctaatg ttacttactc atttaaaata 28860
   gtcatggtgc aatttataat tttttctgca tttttggaat cttcacagta agaggtgtca 28920
   tacagctatt gaacaatact atcgtttctt aaaccatttc agatttattt ttataattga 28980
   gcctggaggc attttcttgg ctagtctccg tgaagctaag ttttatgtta aattgctcaa 29040
   agcttagtct gatgaagaca gtggggaatc aagctaggca ataaatattt tgaaaatgct 29100
   ttgtaaatat gattagattg ttgttgttgt tttccttatg attccctagc tgaccctaag 29160
   gaccatttca aagatttgag tctagaattg tatgaacaat gacattgttg aaaagtgtgt 29220
   agcttcataa aatccctaca ttgaagacta aattttattt tattgtttaa tttttagcat 29280
   gattatcaaa ataattgtgt atatcaattg gttgtttctt aagatatgta gatgtagata 29340
   tataatattt gtcagtcaga ggatttactt cagaatatct tataccctaa tcatgctgag 29400
   atatttttca ttatatcata tggaatatta tttcctcctt atagtaatac tctttagtat 29460
   tttatattgt taaactttta aaaagagagg gtgttacaag attttaagaa ccactgagga 29520
   acatatttgg tataaaatat gactttattt ttattttact agagttttgt ttgctaatga 29580
   aactaagaat gatctatctc ttcattatag tttatatatt atttgtacaa tttgttacat 29640
   ttaaagggca ctgtagtttc tacctctttc attttaccta tatagtaggt ccaaaatgga 29700
   tttttgttgg agaggacatg caagccaatc aatctcttaa aaattgatag tgtaaaaata 29760
   actgaagaaa atagagggta catgagtgcc tgaagtccat taagtatctg actcttgatt 29820
   ttggctctag tcatgatctc aaggtcataa gatagagccc cacactcggg agagtttgct 29880
   tgagattctc tgtctccctc tgcccctcct tctgatgctc actcactccc tctcccttaa 29940
   ataaatcaat ctttaaaaac aaagaaaata ggtagaagtt ttctgttgtg atcatttccc 30000
   cttatttctt taggaactgc tatgagaatt gaataacaat gctaattgta tatctattac 30060
   tctctgtagt atttacataa aaactactta ataactcttc agttaacatc tgccaaacct 30120
   ggaataacat ttttttaaat gactgcatag tcattatgat tagggaagtt ccttaaaata 30180
   aatatttttc ttttgagaaa aataaaattg ttgtaattac aggcttccat gattaaaatg 30240
   ctcataaagg tgatggaatg taaaataaat gtggaaaggg tgctatggct tgaagggaca 30300
   cccataaatt gaattggcaa agtgctaagt ttgtaaatga attaaagtaa ctataactct 30360
   tagcaaactt gagtttcaaa tgcaactggt aacaaggatt tttccttcct aaaaccttta 30420
   ttgaaactcc ttttctaaga ttgatattta taaacagtat taatggctct attgaataaa 30480
   tttattttgt actagatggt tgtttagaga gatgagcttt aagtctcagt atattcatct 30540
   gtgaaataaa ccgttttaat aaaattacag ttttctttgt gtatgtctgt atacatacac 30600
   acacatatat atgtatgtaa gtgtacatat atatatgtat gtatcatatt tgtgtaaatt 30660
   ccaaaatgct aagaactttt ttatgagcag aatcttacat tcctaagaca attacatatt 30720
   aaaaagttta taattcctac accatctctc cagaaaacaa ctccatagcc tttagcagtc 30780
   cctccccatc cccagtgcct gctcctcctc cagcctttgg gccactacgg atctgtttct 30840
   ttctggatag atttgcctat tttggacatt tcatataaaa gtaatcacac aataaatatg 30900
   tcatctttaa tgactgactt ctttcacata cagcataatg tatttaaggt ttatctttgt 30960
   tttatgatat accaatactt ccttcctttt cattgctgat tatagctctg tcatttggat 31020
   gtatcactta ttatttattc acacatcagt tgacggacac tttagctgtt ggctctttgg 31080
   ggctattgta aataatgttg ccatgaatat ttgtgtacat gtttttgtgt gaacatttgt 31140
   ttaaaattat cttgaatata aacctgaaag tggaatttct cagtcatata ttaatttata 31200
   agttttactt ttttgaggaa ttttcagact tttctaaagt agctgtaccg ttttatattc 31260
   ccaccaaaaa tctataagag ttccattttc tctataccta taccagtacc tgtttattgt 31320
   ctatagtttt tgttatattc ttctaaggga ggtgaagtgg tatctttgtg gttttggttt 31380
   gcacgacctt gttggctaat gaagaacatc tttacatgtg cttttggcca tttgtattta 31440
   tctttggagg aatgtctatt cagatcctgt actcagtttt aatagagttg tctttttatt 31500
   agtgttagag ttgtggagtt atttatatat tctagataca aatcctttat aagatatata 31560
   attcacaaaa actttgttct gtgggttgac ttttctttaa ttgattagta atatttattg 31620
   aatagttagt atatgtcagg caatgatcta agtaagcact ttccctgcag catctcacat 31680
   ttaatcctca tcatagcaac cttgtcatat ggatactttt tttttttttt aaagatttta 31740
   tttatttatt tgagagagat acagaggcgg ggcgaggggc agagacacag gcagagggag 31800
   aagcaggctc catgctggga gcctgatgtg ggactggatc ctgggtctcc aggatcacgt 31860
   cctgggccga aggcaggctc caaaccgcta agccacccac gctgcccttt ttaaaattaa 31920
   taatttttat tttttagagc agttttagag actcagcaaa attgagcaga aagtacagaa 31980
   agtttccata tagcccatgt cctcacatat aatctcccct gttaccaacg ttccacccca 32040
   atggtacatt cgttaaaatt gataagcttg cattgacata ccattataac ccgaagtcca 32100
   tagtaggttg acttttcatt ctcttgaggg tatactttga aacacagaag ttctgaattt 32160
   tggtgaaatt tagttttcgc tttttttact tttgcttttt ccttttgttt ccttttgttt 32220
   ttccttttgt ttgggtctct tatttaacaa accatggcct aatccaaggt catgaagatt 32280
   tatgccttta agagttttaa agtttggttt gtaaatttac atctatgatc cattttgaat 32340
   taattatgat atatatatat atatattgtg aggtagatgg gtagggggtc cagcttcatt 32400
   cctttgcata ttggttgttc aattgttcca ggaccatttg ttgaaagact gttccttctc 32460
   catcagatcg tcttggcacc tttgttgaaa atcatttgac tgtaaatgtg agggtgtatt 32520
   tttgcactct catttctgtt catctgtatg tctgtccctc aatgtcttga ttatggtagc 32580
   tacatagtaa gttttcaatc agggaaatac ggatccttta agctcctttg ctctcatcac 32640
   acattcaaat tcttctcatt tctctatcct ctcagaatga ttatacaata attttaaatt 32700
   aatatccatt gttttaatta ttatgagtat gtaattgctc ttcaacgttt gaagtgttat 32760
   ttttcctttc ctgtacagtc ttttgtttta gctagagcta gtgaatcttt ttatatatct 32820
   agccatagag ccttctccca gtggtaaaac tcatttcagt accttcaggc acattaagta 32880
   ttctatcaat gtttgtgtaa gaaatctctt ccagatcttt atgacctgtc taatccagac 32940
   ttctttttct agatgtgttc catagctttg ggtttaaaag actatttatt catcattatg 33000
   gaaatttctt ctgcccttct tttgttggta tctttaatct tcctttttct tcctttagtc 33060
   ccctgtttta gtggagcaca ttctctaaaa tcttcctgaa attttgaagg tattgttcca 33120
   ttgtcttcca gcttttattt ttgtttttat tttttatttt ttaaagattt tatttattcg 33180
   tgagagacac agagaggcag agacataggc agtgggagaa gcagtctctt tgtagggagc 33240
   ccgatggggg actccatccc cagagcccag gatcatgccc tgagccaaag gcagatagat 33300
   gctcaacggc tgagccaccc aggccaccct gtcttccagc ttttagtgct gttgtttaga 33360
   attctagtgc tattttttcc taatttttta aaataggaaa cccatatttt ctctggaatt 33420
   ttgtagaatc ttctgaatct ttctgaattt acatgatcat gtaccttggt ggctctgttt 33480
   atattcttat attgggctct cagcattcct ggaagcttgt ctttttttct tttttaagac 33540
   tttaatttat tcatgagaga cacacagaga gagacatagg cagagggaga agcgggctcc 33600
   ttgcagagag cctgatgtag gactcaatcc caggactccg ggatcatgac ctgagccaaa 33660
   ggcagacgct caaacgctga ctcacccaga tgcctcagct ggtcttttta ttgtggaaag 33720
   tgttcttata attattttgt tggtaatttc cttgcctgtg cttttgcatg ttctcacttc 33780
   tgtcattcct gtgtagatgt tgttcctcct ggatcgtgtg tctgtgtctc tttcccttcc 33840
   tccactattt tccatttctt tgcctttttt gctatgcatt ctgtagattc actgaacttt 33900
   tttcttaatg ttcttataat ctgtttttat tgaacagatg caatatcttc tctgaggatt 33960
   ttaatgatct gattggtttg tttgttgtgt gtgtgatttc tttttactgc atattctttg 34020
   tttccttgca agtcactttt ttctatttat tttggcatct agttttcata ctagcaactc 34080
   tccttaagag cccataactt ttgcctgcct gtcatattta agagatggta tcagtaagct 34140
   aatgggaaaa tttgagtgca ggattatagc ttgttgactg tgatcttcat tttagagtga 34200
   tctcattggg atcttagctg actctgatgt tgggatcctt cagtctttct tctaggactg 34260
   atcacatttc caaaagaaat ttcaattctg ttatctagtt tctaaaattc tagaagttat 34320
   gtaggtaaag aagagtaaag ggcctcaata ttcagtatat cagtatacaa atacacattt 34380
   taaccactga taattaactt taaccctcct ctctgtccag tatctctcag gaaggagaca 34440
   ccttgtttta ctttctgcaa agaataaaca gtagtcttct gggctgggga aaggacaatc 34500
   atgtgtctca gtagaataag gagtggtcag gatgccaaac tgcttctcgc agacttcaac 34560
   ctgttttctt atttcacctt cacattgctt ccagtgccac ctgggaccat catcccagag 34620
   tcttgtcagg gattgtagta taaattatgt tgcttctaaa ctttctctac tgtttttctc 34680
   agcttcctta gcagtctgtt accaattatt catctgcttt cctgctttca aaattagttg 34740
   ctattgtctc ttctgtcaga tattagtcct tgtagatttg tgctatttta aaaatctctt 34800
   tattctcatt ttgtggaatt tcagcagata gtgaaaatga atgcctatgt ccaatttacc 34860
   atcttcaaaa aaaagggact ctacttctat ttttaatctc aaaggcttca cagtttttct 34920
   ttgaccttca ctataaccct cacttgattt gtttttaaaa tatacaagaa gctgtcttaa 34980
   ctgaccttca tgtaactggc atgccagatt aatgagtact ctccattccc tgtaaaatac 35040
   attatgaatg cccactgcag aaccgaatag ctaccctaaa gtgtatctgc acacttctgc 35100
   tccactagtt gaattgtatg tgcttgcagg ggtcagtttc ttccatttta aagaaataga 35160
   agctggaagt agtagttgat atattatgga tgtggtcata caagaaagat accatggaac 35220
   tctagtgggc catttcaaaa gctaatgaaa agtacacata attttaaagt aaaatgtttt 35280
   aaagtaaaaa acaatcattt ttgttatgaa tttcagcttt cagttttctg agttaaatga 35340
   ataagtgcta cccaaagtgt attatataat aacttgtttt caaaaaacat agaaagtcat 35400
   tatagcctgt aggccaaata tatgattttg ccatgttgca tgttgaattt tcacatactt 35460
   aacaagataa ttgtttttgt ttagttccag atgttgaagt gaaaggagag tgttctagct 35520
   attatctcct cttacaaggt aatggtgaca gaaaatgtaa agccacattg attcattcag 35580
   ccaaccagat caatggctca tttgcactca ctttaattca tggaaagatg aaagcaagga 35640
   cagaagaagc caaactgagt aagtgttcat tttaggtgga aaatattttt atttttttta 35700
   tttttatttt ttaaaaattt tatttattta ttcatgagag acacagaggg agagagaggc 35760
   agagacacag gcaaaggaag aagcaggctc catgcaggga gcctgatgtg ggactcgttc 35820
   ctgggactcc aggatcaggc cctgggctga aggtggtgct aaactgctga gccacccggg 35880
   ctgcccagaa aatgttttta tatttgtttg tatatgcagt taacttaggt ggtttttaat 35940
   gaatttattt tccccaaact ccatttccct ttcaatttgc cttttatttc aaacatatta 36000
   tagcatgttt gggcagttta gagtccagat aattaatccc tttttcatta tacttggtaa 36060
   aatttagtgg gttatctttc gattccaaat aggaaatttt tagtattttc cacattaaac 36120
   atatttttct ataatttacc cctcattttt agttacttcc tctcaagcat cttgttccat 36180
   tttattgccc atcttgaagt taacttctct tagagcattg ttctaagaag ccaccaggtt 36240
   ttttgaccca gcctgaagtc atttactgtc atttactatc agtttcagag agctagccta 36300
   gattgattgt ggaaggacta cacaatgggt gaatggtagg tgtggttgat tggggcatct 36360
   ttagagacta gctctcatac tccgtctctg ctttgtttag gagtttgagt agctgagccc 36420
   gtaaaagaaa atgttatgga gggatttcta ctctgttact tgaattccct caatttcaaa 36480
   gggaggtcag agcttaaaat gcaaaataat ttagtatact tgatttctac atcttaatta 36540
   ttattttgaa ataagcaaga gcaagagaga tgggtaactg acttttattt tttaaaagta 36600
   tttaataaca ttttctgtga actattattg tcatagaaca tccttaatac tgactttttt 36660
   ctatttctca ggctttcctt ttgacttctt gtcacttcca catttttcgg gtgagcagct 36720
   tatacagaga gagaaacagt tagctaatgt tcaagctttg gctttgaaag aatgtctgag 36780
   taagtaatga tttgtacatt tcattctatg ttaagaatgt taactcgttc agacacaatg 36840
   ctttgttact gtcatacttt ttcttacaca ttttgtcttt taggtaaaat gcttatttgt 36900
   agaactatcc ttgatagagg tgatgcagag taggaaaggt ctgcaacctt tttccatcgg 36960
   attcagaatc ttcctatcta ttcgtacatt gccgttaaca tttataaaaa tatatatata 37020
   tatattttaa agattgagtt atttatttga gagagagaag acggggagag ggaccgaggg 37080
   agagggagag aaagtcttaa gcagattcta cactgaatac gaagctggag cctgatggaa 37140
   ggctcaatct catgaccctg agatcatgac ctgagccgaa accaagaaac tgggctatcc 37200
   aggtgcccca taaaaatatt tctttaggaa aattttgtat tccattcttc catttttaaa 37260
   aaaattctga ttaatataga tctttttata aaatttccca tatatttttg gttgtaacag 37320
   taggatagag actgcagaga gaagaaaata caaatacaga taaattaatg tgctaacttg 37380
   actattgatt cttagacttt tactattact aacaacgcat ttcatatata tggcagaatg 37440
   aagttagcat tatggatttt tccttattca ggaagctaac actttttcca agagtcatag 37500
   tttaaggtct tacctctatt actaaatctt aacttggggg atccctgggt ggctcagcgg 37560
   ttgagcgtct gcttttggct cagggtgtga ttccagagtt ccgggatcaa gtcccacatt 37620
   tggctccctg catggagcct gcttctcaaa aacaaaaata aatcttaact tggtatcagt 37680
   cttaactttt ttaaacaaat aatcattaat tactacctac tatgaactta ttttaaggca 37740
   attgatagag aatttttatt tgattcaaat tgtgaagcct ctttagttgc atataaaaaa 37800
   agatgataat taaattgagt agatacatta taaaataaca actgtctatt ttttctttaa 37860
   aagcatttta aaactatctt gagttcagta gcctatttta ttcaatggaa aataatttta 37920
   gaatacttgg tctcaattat tcaagaatta cctacttttt caattgtgca aatcagggta 37980
   attgcgtagt cttgagcttc tccagccatt gatttttttt ttaattgtat cgatattagt 38040
   gcaaggaaaa tagttagaaa ggcagatcgg tttcaataaa aagtagtaca tgttaaaacc 38100
   aagaaaaaga ataaatactg aaataatatt tattggctag aaaactctta tttcaaatat 38160
   caacctgcag tattattcac ctcttgactt tgttccgtcc ctgctacact cgtcttcagg 38220
   ccctgcttcc ttgccttaag ccttttgcag ctccttgtaa caaaattccc tcaatatctc 38280
   ccctttcctt catttcacct ctcttcctaa cccttccaac cagaaaaact gccagagtag 38340
   aaaaataggc tgctctctga agctgacaca tttctccttt gagtatgaag agttaaccct 38400
   cctagatagg tgggaaggga aatactaaca gctataacca gactgctgcc tgcaggtgtg 38460
   gttggaaact ctactgggga aaaagggaaa caggagtggt agataaatgt agaagcacca 38520
   gtagctgagc tgtaccacag ttgtaaggat gccacagctc tgggcaaggt gcaaggacaa 38580
   gtagggggcc aaaagttgag ggaaagctta cagctagtag ctgctagaga taccagctct 38640
   gggcatatcc atgaaatgac cagtttgtaa tccaaagcat taaatcttga tgaaaattag 38700
   tgattcaatt aatcaatttg tgatggaaat atttgctgaa ccaatctctt ctttacccag 38760
   agagtcaata ctggatttaa aatttggggt aaagttatat cagttaagtt cctgtatctc 38820
   agaagacaaa gtaacagcaa aacaaagttt cttatgtaag aatttctgaa tgtataatta 38880
   atttacacat ggtgaaaact aatatattaa gaacaacttt ctttggaaaa aaatcgacaa 38940
   ttcagaatca gtacaaagtt tatagtcttt gctatgaaac aaaaagttgt tcaagacgat 39000
   tttttcaaaa ttatcaacaa gacatggagt agacgtacta tctttccact gtttctctga 39060
   ccagtactga atcagaaaga gcaactggaa aagtttttat gaaaatgtac atacattatt 39120
   gagacacttt gttttttaaa atacacaaaa ttcaaataaa atttctggta tggttttatt 39180
   tttagaataa ttacttccaa ggggacctgg gtggctcagt tggttcagca tctgcctgca 39240
   gctcaggtca taatcccggg gtcctgggat caagccccac atagggctcc ctgctcgatg 39300
   gggagcctgc ttctccctct ccctcttgct ccacctgctt atgctctctc tccacctctc 39360
   aaatcaataa ataaaaaatc gttaaaaaaa tagaataatt acttccagga gttttattta 39420
   tttttaaaaa ttatatttaa tactattata ttagaattat ttttataatt ttatagtttt 39480
   tttacttgat tttttggtag ttcataactt cttatatttg aaaatgttaa tatcttgttc 39540
   actgattatt ttaaagaaaa ggtttaagaa tgctagatta aaaaaaaaaa aaaagaatgc 39600
   tagataagtt ttaaataaag actatttatt atgatgcaag ataaatccaa aacaacagtg 39660
   ttgtcatttc actccagcat tgcggtcact actctgagtt tcccacatct tttttttttt 39720
   ttaagatttt atttatttat tcatgagaga cacacagaga gagaggcaga gacacaggca 39780
   gagggaggaa caggctccag gcagggagcc cgacgtggta ctcgatcttg ggactccaga 39840
   atcacaccct gggctgaagg cggcactaaa ccgctaggcc acaagagctg cccatcccac 39900
   atctttttta aatttataga acctaataca aacctgattg acactcagtc tctggtactt 39960
   ttagatattc tcctctatat ttttctttaa taaatgctaa gttcttgcta gctttctcag 40020
   gaatagcatt cttttgctga ttcattttta gactttagtc caatatttgt ctcccagaat 40080
   tttgccacca tgtgcaaact catttattta tagattattt tttgcctcag tgaattattc 40140
   gttcataacc ctgttaaact tcatctattc cctatttctt tttctgacat cagggccagg 40200
   aggaagtctt actccactct ccagatagcc caatgttgta atatagttgg ctgttttaat 40260
   aagcatgagt taggttcatg atttgtttat ttttaacatt aataaaagtg tagaactaag 40320
   ttctaaagca cattacctat tgtgcgtctt tcagataaaa acaaatcatt gataactttt 40380
   tgttttagaa tggcacccag cagattgtgc ttcacataaa aagcttcact gctttttatt 40440
   ttctcacttg taataacaac agtatatttt acaatcttgc tttaccatta ctaatgttct 40500
   tgagtgcagg aaccatgtgt tacttatctt ggatcacaac tggaatgacc ctaccaatat 40560
   ccacactggg aaggtgggat tttgcagagg cattgtcgta aaactgatgt tttaaagtag 40620
   gacctctagg ctggctgctt tcttctcagt ctactctctt gcaatattta tgtattgccc 40680
   tcatgtagta atgggctcta attctcagtg cctaagggca caacacagtg ctttgtatac 40740
   aatacataat aaatgtcgaa ttgaattctc tatgcattta caatttacta ttttatttac 40800
   tcatgttatt agtccagaag tgttttaaaa atgtcatact tttattaaaa taatacatgg 40860
   gtctagttta agatgcaaat ggtattgaca aacttgatgt ggacactagc agtaccctac 40920
   tgtctcctct ccatctttca gcctctctct gcagcagcta cctgcagctg tttggctggt 40980
   tttcttaaca tttcctccag ttctgttaat agtatctgaa tgctgctgtt tcttaatttg 41040
   ccagttgaag acattatttt agcatctatc tatggaagat taagactcag ttttctcata 41100
   tcatcttcac tctccctttc caccctttac agtgaagtct tgaattttgg caaaatcagt 41160
   ggttagtttt aacattgtta tccatctctc tctctctctc tctctctctc tatatatata 41220
   tatatatata tatatatata tatatatata tatatatata taatatcacc tctcttgtat 41280
   aatctttgtt tttcttatgg ttgataatca tttttaattt gcccattctt ccatacaggc 41340
   aaaatattat tatgtttttc cagaggcccc aacatttttc tacatacttc ccattaaatt 41400
   cattgtatgt tcaaagacag cagttctttt tatttctctt tatttttttt tcagagtgtt 41460
   caatattttg ttctgatagc agaacatttg agacttctct ttcttctctt cttttggagt 41520
   ctcctttttt ctagacccct gttctccctc ctccttggat tactctctaa cttggttgga 41580
   gcatatattt cagtaactac ttgcgttaaa ctttgtggag cataaatttt ttttaagctc 41640
   ttgcatatct gaaaatatct ttacttgatt gatagattgt gaagaatcct aggttgaaaa 41700
   tcatctacct tcagaatttt gaagacattg ctttattatc ttctgtgtga ctgtgttcct 41760
   attgagaaat gtaatactat tcttatcctt tgtagtgttt tatttttggc ttgtttttac 41820
   ccctttctgg aatgttttat ctccagaagc tttactctga atcgtgctgt gtatgtattt 41880
   tccattctct ttctctgccc tatatttatt aagctatgta tttgatgagc catttcaata 41940
   gaagattcac attcttaaat ctgaaaactt ttatctttaa taattttgtt tctttcctgt 42000
   tttttttttt tttccttttt agaacttttg ttaagcatat aaaaatacct gaacagaata 42060
   ccaaattttc tttcctattt ccatattgtt ttctttttgt tctagtttct gggcaatatc 42120
   ttgcattttt ttaatagatt ttatttattt attcatgaga gacagagaga gagagggaga 42180
   gagaggcaga gacacaggca gatggagaag caggttccat gcagggaggc cgatgtggga 42240
   ctcgatccca ggtctccagg atcaggccct ggactgaagg cggcactatc tgctgagcca 42300
   ctggggctgc ctgcattttt ttttttttaa gcccttttat ggaatgttta acatttgagc 42360
   tctcatgggc ttaattttta aaagtctctt cttgttcatc aactgttatt taaggatcct 42420
   gtacttgttt catggggtgt attatcttct cttagttctc tgagaatatt aattatagca 42480
   aacttgcttt tgttccctgc aggttttttg tttgtttctg ctgggcttct ttttcctttc 42540
   gcttgcttta tttctctttt tcattttgga cttttctgat ttgagagtct caacaaataa 42600
   acccctcttt actctcctaa agttggaacc aattgagaaa taaacctaga aacactgatg 42660
   gagataaata aaaaataaaa gctgtattga aggatatgac attttagatc tatggccagt 42720
   tgagctaacc tccttgagtg aagaactgga caaatacata tcaacccaat gggggcaggg 42780
   cctttaccat atggtaggga gcatgcatcc tgaggacttg ccagctcgga ggatagttgg 42840
   agagaaggat ctgatcagag catgccttgt ttctactcca gactagctct gtgagtgatg 42900
   cgagggagac aggatctaga actgttgctg gatcaaagtc cttccttaca tgtgtaagca 42960
   taagaccatg gaaatgcaga gtccctcttt ccccaatacc tttttttttt attctttact 43020
   taaattccat gtagttaaca tatactgtat tatagtttcg gggtaggatt tcgtgattcg 43080
   tcagttgcat ttaacaccca gtgctcatta tgtcaagtgc ccccccttaa tgtccatcac 43140
   ccagttaccc catccccccc acctcccctc cagcaaccct caatttgttc cctagagttg 43200
   aagtctttta tggtttgcct ccctctctgt ttttatctta tttatttttc cttcccttcc 43260
   cctatgttca tctgttttgt ttcttacatt ccacatgtga gggaagtcat atatttgtcg 43320
   ttctctgatt cacttatttc acttagtatg ataccctcta attccataaa tgtcattgca 43380
   aatggcaaga tttcattctt tctgatgagt gagtaatatt ccattgtcta tacagaccac 43440
   atcttcttta tctgtccatt catctgtcaa tagacatcta ggctcttccc atattttggc 43500
   tattgtggac attgctgcta taaacattga ggtgcaggtg ccctttgaat tactatgttt 43560
   ctgtctgtac acttttaaat gggaagcttt acataccctt ctctaatata ctgtctggta 43620
   atctacaccg taaagatgtc gagtgacagg tcatcatttt ggttgagagc tcctatccct 43680
   acacctccac ttcaatatca gggtcaagct tttttttttc caggggcatt cagtgtttca 43740
   gaaggggaat cctttaatcg ttctagtgga gcttttgtag ctgctagctt tctggctacg 43800
   gggcttggag tcagaccttt cagtatgaac acttcttgtt agtccctcag ttttaacccg 43860
   agcctcagtg ttacctttca gtgtgcctga tgctcaatgg gctaggactt ctttcttgct 43920
   tctaccccag aaaaaaaaaa aaaaatgaga acagagtctc ctggctgtga gaggttgagg 43980
   gtacggtgaa gacctttact tctgactgct tcttccctat gcctccagtc ctgcgttagc 44040
   ctggacctca ccatcatctt cctaatgcct cacagtagca cctctgcagg ctcccagatg 44100
   acccttcctc tgctgtagca gctcagttac cactctcaaa ctgtcattat ttattgtttc 44160
   tgcttttctt ttccttttct taattccttg ctgtgagtgt aggtttgggg aaggagaaga 44220
   aaaaggtgga agtgtcccaa ctaccacatt taactgaaag tcttacaggt gttattttca 44280
   cataaacacc aacattctta gtgctctgca gtatatccta aaaacttgca aactcccctt 44340
   taccattttt cacctcttgg tgttaacaga ttactctatt tttgtttatc tcacataact 44400
   ggaaattcta tcattaccta caatggggtt aaaatgaaaa caggcctaca gcaataggaa 44460
   tgctgatgtg ctggagaaat tcacctattt tacttttgaa aatatttctc ctaaaagggt 44520
   agtagaggca tttcgtgaga gtctttagaa taatagtcat atggctaatc gtttttgttg 44580
   tcttacagaa aggagaaagt tggcaaagca gcctgaagca gtttctgttg atgaattcaa 44640
   aagtctattg atactcacaa gggaatcctt tttagagcat tttgatgctg ttattcctaa 44700
   aacaattcca ataaagacag acaaaattaa aacctccaat atgttaaatg gtaagttttt 44760
   tgttagttta aatatggatt ctttttgtat gtatggcatt tatgacattt cttattaaaa 44820
   ccaataaaat ggtgatcaaa aatattttat acatttaaac agtgatatat gtatttgttt 44880
   atacaagtat gtgtatgtat ttgatgaact atttctcacc atttaaacta aaaacttctt 44940
   tcattaagca tgtttaatat tgtttttgag cagagggaga atcattttaa ggtgattcct 45000
   cttactactt ttactaagct gctagatagg tggattttta taaagctatg ttttcataag 45060
   atgagaacag tagaacctac acataagttt atgattatta aaactaccta caataagatg 45120
   ctccacattt gccatacatt atagcaggaa cagtcagcaa actccactga aggagcagga 45180
   aatcatttct atattaactt gcatttctat tttctcggtt cctattcttg tgatatattt 45240
   gaatacaatg aagctttaca gaatctaaca agaaagcatt tgattattgc ctggcatttt 45300
   aaaagtacag ggtaattttt tgtttttatt ccaggagaat catttttatt ctataagttg 45360
   ataagaaaat gaccaaagaa tcaagtaaac agtaatgtct gttgaaagcc tttctctgtt 45420
   tgacaccata aagataaaca aagaagtaaa aatttggatc cctgactttc agcaggctta 45480
   tgatcgattt cagaagcaaa ataaaataga acacagagcc atttcaagag cccaggctct 45540
   ggagctcaag tgccagggct ctaatctgag ttttgtcact tgttagccaa atgtctttgg 45600
   gcagttattc tctctctcta tgccttcgcc tcctcgtgtt taaaatggcc ttaatactat 45660
   tacccactca ctagggttat tgtgaagatt agatgagctg acgtatgtga gaacataata 45720
   aatgcggaag agctgttagc tgttatttgt attatcatta tcatcatcat gacatatcag 45780
   taagaaagtt cagttaacag tacagagtaa tctgacaggt gttagagaca gtgtaaagtt 45840
   tcttgtaaat gggaaatgta ttcaataaat gagagacaca gtttcttgtt tgcagcatgg 45900
   actctggatc cagactaaca aggctggatg tggccctgca gcttacctgc tgtgtgacct 45960
   tgggcaagtt ttctaacctc tctgagcctc aagctttttt taatctttaa attagagatg 46020
   gcataggttg ttacaaggat taaaggaatt atttataaat tgcttagaaa gtcacttggt 46080
   atgaagtgct atatgtttgt ttttattttt atttatttta aaaatatttt atttattcat 46140
   gagagagaga aagagagaga gagagaagca gaggcacagg tagaggaaga agcaggctcc 46200
   atgcagggaa cctgacatgg gactcaatcc caggtctcca ggatcacgcc ccaggctgaa 46260
   agtggcacta aaccactgag ccacctgggc tgccctatat gtttgttttt aaataaatga 46320
   atgtgaattt aaaggagagg aatataagta gggtaggata gtcacagaac acgttgttag 46380
   gagtgaaggc actctgttgt aggtcttgag tgatacatag gatctaggtt gagtggagaa 46440
   cacattttcc agggagaagt tccataccca tacccagagt catgaaggca acaaactgag 46500
   atccagagag aagttggctt actcagagga cttgtggagg agataatagt tggaagatag 46560
   gaagggttga agtcattaca cagagttttt ctgtaagtat ggaaatttat ctcacagtta 46620
   cccgtacgaa gtcaagtcct aggacactgt taacacactc cgtttacagg ggtgttcatt 46680
   ttaaaatgta aatcagatta tatcactcct ttcctcaaac ctttgtcatt gctttccatt 46740
   gtacatagaa tatgtcatcc ttaatatggc ttacaaaaac cctaactgat gtctgattct 46800
   aaccttctcc agtgccacat ttctgtttcc ttggtggcac agagagtctt tccgttcctt 46860
   gagcacgtca catccctttt tacctcagga actttataca cagaggatcc tctttctgga 46920
   atacttttct cccaatcatt acctgattaa caggcattct tcatatcttg acttaaatgc 46980
   tacttcctca aagaagcttt ttttcccaga ccttcctcag tttaaattgt ttcctgttgt 47040
   actcattttc tgtatctttc tttaaattat ttttcacatt ttaagttata tactatagat 47100
   gtaattattt gttaactatt tgtcttcact ctgaatcata tgttctctga gagtaaagag 47160
   tgatgcctac tctccattgc cctagagtac tgtacctggc acataggagt tcattcctcc 47220
   caaaagtcat gttgaacaaa tgaaagaaac cttgaagatt ttttagagcc tgaagtgaaa 47280
   tttgagatga cttccaggaa tgaagttgtg atctttaaca tcctttgtag cttttgtttg 47340
   aataaataca tctaaatgca aaatacaggg cagcccctgt ggtgcagcgg tttagcgcca 47400
   cctgcagcct ggggtgtgat cctgggggcc caggattgag tcccacgtcg ggctccctgc 47460
   atagagcctc cttctccctc tgcctgtgtc tctgcctctc tctctctctg tgtctctatg 47520
   aataaataaa taaaaatctt aaaaaataaa taagtaagta agtaaatgca aaacacacag 47580
   caaaagaagc aacaatgcca gtcccattat ttaacaacca cactcatcag gaattattgc 47640
   tcaccatcag gattattgat tttttttttt tttttgtaga aactctagca tgggtaacat 47700
   gaaaatcagc atgaaaaaaa tgtcttgata taaccatttc atatatccat tgccacatag 47760
   ttgctaaaaa ctttcacagt gtcaaatgag atatctggac tcaaaatata gtaactgtct 47820
   ccttttacct cagaccattg gggtacataa gcacactgca caaatgagtt ttctaggtaa 47880
   ttttaaaaga tcgcagtttt attttaagta ttttaataag aatcttctgg tatgtactta 47940
   taattcttac tttattaaac agaatatgcc aattacaata taagttttct ttgtgattta 48000
   ttcatattta tgtacataag gctatgatgg cctatattac tatctacata ttatcatctg 48060
   caatattact tcctttttgt ttactgtgat ttgtgtaggg gaggttttga gcccctattt 48120
   ccttattgta tcattttcat ttctttgttt ctcctataaa gtaagacttt ttttttaaag 48180
   cagtgttctt ggtttctcca agaacaaaaa agaggccctt tttgtgcttt ttcatcgtaa 48240
   acatggtgtc ctgattgcca cataaataaa atattgtctt actttactgt aagaattgta 48300
   ttaaattatt ccccaaaagt acatgtgcat gtctccatcc attccttaat atttagccta 48360
   tgtcatttat acttatataa attgtatagt caacattaat aattaattat attgtaatat 48420
   ttaggaacaa ttaatattta tttttattta acatgacttg tgagttctca gcaagtaatt 48480
   gctcagatct gctaatgata tttgctttat acatttagta gaaagccatg tcaacattaa 48540
   acagtagatt attataaagc tgttgctgta taaaatagta tttcactatc cttatttaag 48600
   aaatgggaag tatggtatat ttggatggtg tttggtgggg gcaatccaat attgtcttta 48660
   aagatatttt aatattcaaa gttaaatagt ttaatgaata aaggggaaat aacttgcagt 48720
   tggcttcagt tttcatttat gtttcagttg tttctttttt gtataaaaat tcccttatca 48780
   aatcatcatt ttgtaagaat ccaaagaata aaatgggtat ttttgtcaga attgatggtt 48840
   agtcaaacaa ctaatgtcca gtaattttca gttactctat tagaatagtg tgtaagagtt 48900
   cacagtgata gtaaaccaga taggtttaga attcaattca accagatagg tttggaagta 48960
   ggacaataat tttgacagtg ttaaaattag tttggatgta ctaataattt ataatgcaaa 49020
   tggaagctgt ccctttatgt ttttgataaa gattgaagtg ttattagcaa tctcagttca 49080
   ttgctagaag gataagcaca ctgtgatata aaatatttta ctagtagtga aataacattt 49140
   aatgaagatg tgtatgttaa ggcatcttac attaatggat aagtgaaaat tctttctaaa 49200
   gtgaaaacct ttatctcaga atatcctgtg ttttttgcat tattgtactt ttgagaatga 49260
   agtaatctct tttagaaata agaacttttc tggataatgc tgctaattcc tgtttctggt 49320
   gtgaaaatca catagtatat ctaatatatt ttccatagag ttgcctcctt ttcatttaga 49380
   actaatttca caaaaaaaaa caggcaaatt atgcttttat tccacaggag tcctcataga 49440
   gctaattcat tttctgaatt ctagctcata gcttatgaag agacagtaaa gtaacgtttg 49500
   actttttttt ttttttttca tttccaaaaa cctttttaat ggcattttgc attgtttggg 49560
   ccatcagata tatccttaat aagtgcctga ggtaagtact gtgtagttaa tccaacacgg 49620
   ctttatatct atatctatat ctatatctat atctatatct atctatatct gtacctatat 49680
   atagatatat atgttttcat gtagaaaatt caatgttaat aatttttagt acttatgtag 49740
   gtttctttca ctttagatgc cagttcagtg gaatctactt cttcaaatct aatggaaacc 49800
   aatgctctgg aatggccaga aaggcatgtt cttcaaaatt tggaaagttt tgaaaaagct 49860
   aagcaaaaaa tgaggtaata tttaaatagc ttagtatttt ttaaatggat tttttttctt 49920
   aatctttaca ttgtgtttat gaaatataag tattttctag ttttattagg aaatgatttt 49980
   ttctttatta aaggaacata tgcgtattct aagtagacag gtagatggag cgttacagag 50040
   taggatgttg agaacatgct gttcttccct agtgtctact tgtactttct gtgtgtacat 50100
   aatcatagtt tttttttttt tttaagattt atttattcat gatagacaca gagaaagaga 50160
   gagagaggca gagacacagg aggagggaga agcaggcacc atgctgggag cccgatgtgg 50220
   gacttgatcc tgggactcca ggatcgcgcc ctgggcctaa ggcaggcgct aaactgctga 50280
   gccacccagg gatccctaca taatcatagt taacatttta gtgaatgcct ttccaggcta 50340
   cctttactgc tctatgtttt gggtattaga aggtggaggg aagacagtaa agtatcctcc 50400
   aaactgagaa ggcacttgag gatggaaaaa aagaagcagg ccattacata ccttctacat 50460
   tacatacatt cagagtaatt tactgatggt gcttcaggcg catgacagtc cagcactgtg 50520
   cagctattct ccacaaaatt cagaccttaa tccacagttt ttatacagcg aggggatggg 50580
   cataggtcat tgtagtgagg tcaaaggatt taacccacct ggttggagcc atctgtttgt 50640
   tagagaggag aaagggaaag aaactaagct atctctgcta gttacctaaa caactttagg 50700
   gaagatcaga acaagccttt tcacgtggtc agggcatgca ttagccccca aggggcctgg 50760
   aacatagtcc tgagagagtt cattacatga aggagatgga gaaccaaaga tggagtccgt 50820
   attgtcagca ctcctatagt tgggattttt ttttttttga cccatgcttt cttcttttag 50880
   ttttttgtta gtcatatttt aaatttctaa taatttcctt tgtgttctca actctttttt 50940
   ttttcattag caatctcttc ttttattaac aatgagaatt tggaagatac taaaaagatg 51000
   ttaattggaa tcttctttta acttacttta tctgttcctt agatgatctc attatccttg 51060
   gatctgttct gtttgttcct ttgctgtttt cctcgtatgt gtagctttcc tccaatgtct 51120
   ggtggtcctt gattgttact gggtcagaat ttattatgaa gttgctttat ataggtagtt 51180
   gatgtttatt ttctctccat atatgtagtc ctgtttcccg cctgttgtct tctgaatgtt 51240
   gtgggcaggg agatgcaggg tggagcatac ctggtagact tcactttggg gtacctctgg 51300
   gggtatcaga aggccagcag ccttagtcct tcactcaaga ctgaaataat ggggttttgt 51360
   tctgggggca tcaccactga ggctaggaca ggcatctagt tctggatctt cagagagtgg 51420
   tttctctatt ttcacttaga gataaaattg tggggataag gaagggtgtg ggagggtgat 51480
   acttaaacaa ctgttctagt agggttctta aaatacattg cctgatttct gccccttgcc 51540
   actcagccca agcgtagagt ctcattgtgg ctttgtggga tagaagacag attccacatt 51600
   tttttgtaag tttctctctc tagctctcat ttatgcactt gctctagttt atcccttttg 51660
   tttgttccac catttttttc atctttcatt tttcaacctt cttatgaatt tagtgtctgg 51720
   tacttcatca ttgtgatttc catttatttg agtggagagg ttaatatgta tgtttaatgc 51780
   accatcatct tgaaccagag gcttgtttgt cattcaaggt ctagatcaaa tatcaccttt 51840
   ctcttaagta ttttataaat gttgcagctc agtgttctct ttcttcatta agcacccaag 51900
   gactataaca ttacttcttt tcatgaagct tattttatta cacctttttt tttttacctc 51960
   attctacctt ggtatatctc ctttcactgt ccttaacctc ataccttcat actaaattac 52020
   atacagtgat tatgagtcta tacattatca tcagtgaact cattttagta actcccaatg 52080
   gtatttgcaa agtcaatact caaatacttg tgagagtgta acaatatggg gagatactat 52140
   tgaatttttg gttgggaaca aaagttactt tgcatattta cagaatttgt attgtgttaa 52200
   aataagctta actggagaat taaaggttct ttacatagtg aaattcagaa gtcataagaa 52260
   tttggttttg ggctaactca catgtgtgac tttgttgcct catctctaag atggggataa 52320
   caatcccagt tctacttaaa gtttatcaat gtttaatgag attatatatg aaaaagtact 52380
   ttgtaacctt gaaaacacta gacaaaataa agcgctatta ttttattgat tgattttagc 52440
   ctaagacata tcttgatggt ataatttatc acaaactggg cttttaaatt gctcagatat 52500
   ctttgtgttc ggggctatat aaatgcaaat tataaacatc ccaaatctag cctataaaat 52560
   gattgggatt aatattcatt aagaaacgtt tgtgagtcat aattagagaa tagctatgta 52620
   gaaatttagg ttaaactcat aatgtagttc ctactctctt ggagtatgga attcagagaa 52680
   aatatttgaa tattttcttg tttgttttta ttaggtttat gttttcagct taaaaataaa 52740
   ggggatagga ggaaggggca cattgttggt atattattga tctttaatgt atagaaatag 52800
   aagtctaagt ccataatgtc aacactttaa tcatataatc accaatatat tggtaattaa 52860
   atattcttga ttggagtgat aattgggaga tagctgaatt aatataaagt catgaagtta 52920
   atttcctgtg gaggtttcag aagattttat ttatgctttg atgatagagc aatagcatgg 52980
   tctgtgactc agtgcttgat aaaactgcta ccattctctg aaactaagtt tttttccttt 53040
   ttctggtttt taaacttcct gtgagctttt taggtttata gagtgagagt gtaggaatag 53100
   tacttaattc agagtcagtc tagttctgac tatcatttta aatgactgtg tcaccctggg 53160
   cttgtaatgc aaactaatgt gtgttagtat ctttatctga aaattagtaa taaggattct 53220
   tttctaggtt tggtatgcta actttcactt tagaaagtac ttatcttctt tttttttttt 53280
   tttttgtata ttccctatgc tatccttttt attccttttt tttttattta tttttattgg 53340
   tgttcaattt actaacatac agaataaccc ccagtgcccg tcacccattc actcccaccc 53400
   cccgccctcc tccccttcta ccacccctag ttcgtttccc agagttagga gtctttacgt 53460
   tctgtctccc tttctgatat ttcccgcaca tttcttctcc cttcccttat attccctttc 53520
   actattattt atattcccca aatgaatgag aacatataaa tgtttgtcct tctccgactg 53580
   acttacttca ctcagcataa taccctccag ttccatccac gttgaagcaa atggtgggta 53640
   tttgtcattt ctaatagctg aggaatattc cattgtatac ataaaccaca tcttctttat 53700
   ccattcatct tttgttggac accgaggctc cttccacagt ttggctatcg tggccattgc 53760
   tgctataaac atcggggcga ccactctctt tcaccataca caaagataaa ctcaaaatgg 53820
   atgaaagatc taaatgtgag acaagattcc atcaaaatcc tagagaagaa cacaggcaac 53880
   accctctttg aactcggcca tagtaacttc ttgcaagata catccacgaa ggcaaaagaa 53940
   agtacttatt ttcaggtgtt cattttgaga taagatgtgt tctgtctgtc agggaaattt 54000
   gtgagaatta agaacatatt ttatgtaaag tagtttgaga agtataaact cctgtatcta 54060
   tttcaggtat aataatgatt cagtacttat tcataatgtt ataataatgg ctaggagaca 54120
   ccttgaacag atttctgatt actgtctcat ttcatggttg tgcttcaagt aatctctcaa 54180
   gcccatagaa aattggaaca ttcagtctct accatcaaaa tttgagatta tggggtttca 54240
   gaacatagaa aaacatttag atttaaaata ttttttaaga ttttatttat ttatttgaaa 54300
   aagagagcag gagttgaggg ggaggtgcag agggaaaggg agaagcagat tccccgctga 54360
   gccaggaacc tgaagtggcg cttgatccca ggaccctgag atgatgacct gagctgaagg 54420
   caaacactta accaactggg ccacctaggc accctctaga tttaaacttt atagaattga 54480
   taacttcaca ctactaaata atcatgccat catttatctt ttcttgaaga tttgcttatt 54540
   ttagaaagag cagggggaga ggcagaggag gaagaggagg agttgagaga gagagagaga 54600
   gagaatctca agcagactcc ccactcagtg cagaacccaa ctaggagttt tgatcccatg 54660
   accctgagat catgacctga gccgaaatca agagttggat gttcaatgga ctgagccacc 54720
   caggtgctcc tcatttctca tgccatcttt tataaagaat ataattatgt ttattttagt 54780
   taagtagtag cattattgac atggttaact ataagtttca ttgtgaccta catttttaaa 54840
   aatatacttt tacactaaat ggttcacaaa gattttactt tattttcttt cctaaatata 54900
   tagttctgta gttttctttg ttttggtttc tgctcatatc accttttcct gactatgatg 54960
   aattctaatc cacttttaat aatctagata acattatttt ctcagaactg gttcattacc 55020
   tcgttcatct gagcagttgc tgggccacaa agaagggcca cgggactcga tcacattatt 55080
   ggatgctaaa gaattactga aattctttac ttcagatgga ttaccaattg gagatcttca 55140
   gcctttacag attcaaaagg ggtaagttat aaacttttag ttgccaattt tttaaaaata 55200
   attttaaaac acattgcatt tcatttcttt agttaattga tgccctatgc tacttgggca 55260
   aaattcacaa gtttgatccc aatgcttatc atttggactt aattttactc acattttacc 55320
   ttacccaatg tcctattttt ttgtggtatt gaatataaag aaaacaaagg caataagagc 55380
   aacctcagtt tattccctat agaagtcagt agcttctact ttgtatacaa aggacagtgt 55440
   tgtttaaaaa aaaaaattat aatttgactt gcaaaatatt tttaattctt ttttctgtgt 55500
   tattctctag ttttgatttt gcctttacct gtcatgacat tgtcagtcat gcctaatatt 55560
   agcaaaaagc acagcctttc aactgaagag aacattaaag tatagaaatg tttttgaaac 55620
   tataatatta ccgagtaaga actcggtatg aatatgaata gtttactact aatttgactt 55680
   ccaagacaac tttcactaag ttactagtta ataaatatga gatattgcta aagttgcttt 55740
   aattacaggt aaaatttgtg atgtcagtta cttgtgagat tgaatcattt gttcactata 55800
   tatatcatcg taattgttaa tctattattt tgttaatcta tatcttaatt gttaatccat 55860
   cattttgaag acctcccaga ggaccttaaa aattataatt cattttaatc tcttctacaa 55920
   aacttgtttt ggaaaatact gattttaagg gtaaactttg gaagggattt atttaaaggc 55980
   agttgtatct caaagataat cctggagcaa gcatattgcc ttcatgtgct gttcactaaa 56040
   gagagagaga aggaacacct ttgaaaattt gaggctcttc ataattctgg atttctggat 56100
   tttaatgact taataatctt aatgacatac aagttgtgta atttttcttt cctgatagct 56160
   ttgagagatc tgtaatattt ttttatggta atctggagca tgaagaagta tataaacatt 56220
   tttcacctat ttagctattg gattattgtc ctcccagatc attaagtgtg aagtgacaaa 56280
   gttgagtaga tttgccaaaa ctgaattata tagacatttt gaacagaatg tctttctagg 56340
   tgcaatgtta gggaagatat ctgtagggga gatacgattt tttctgtaat ttacaggtaa 56400
   tttatttagg aaaatttgat atagttcctt agtaaagaat gacatcatta tctaataatc 56460
   ttctattagc attgaagttg aagaatgagc tttcctaaca ataaatcaag aacctatgga 56520
   ctcttattac aatgtctacc tatacaagta gctattggaa tattaaaaag aatgcagaca 56580
   tgactcctat gtgtatatca aagaaatatt aagcagtgac aaaagacata tttattaaaa 56640
   cattttagaa agaacaggtg tgagtaaaat taaagaaaaa tacataaagt tgcagagagg 56700
   gttgaaatta gaaatggatg cagtatgcta aaagaaagaa gtgaaatgct aggtgcctaa 56760
   atcattttat attaggaaat tattcacttt aatagtgagt catcaaaata ctggagtcat 56820
   tgcagttaat tactattatt ttttgtggaa caccttaaat ggatgaatta gaatatatat 56880
   atttttttag gattcatact tagcacatgc cttatattaa aatgtaataa tttcaaatgc 56940
   ttattatagt acccttgata attttcagtg gcaagtgggc agttttcatt attaaagcca 57000
   taggggaaaa attgtgaaag cacatttatt catggaacta tactatctgt tgcttttcta 57060
   tataccattt ccaagtcttc tgaattcaga cccctggtat ttcattctat taactatgta 57120
   aaggccgctg ttacattatt cttccattcc catctcccct gtcctgagtt tcaggaacat 57180
   gatccagtgc attggaacag tgactttcaa ctcagggaga gaaaactagg ggttgtgggg 57240
   agtgggcatc tcttagaata accaggagag cctaacctaa ttgcagtgaa ctttttcttc 57300
   ctgagatttt gatatacccc tgtggatctg ggtgtaaaag agattgagca tgaatatttt 57360
   atgtaagctc accactatat gattagttat catacctgct gattggaaac ctacttcaaa 57420
   agcagaacac tgaagtcagc agtctctata tcctttgata tttaacaggt tgctaactct 57480
   caaagtaggt gtcaagatga ctacattcct aggcaaagaa aaatctggat ctctcatgtg 57540
   ttctagtagg aagtaaatac ctttggtact aaaagatttg aattgacttt aatttattcc 57600
   cctgcaaaaa aaagaactaa atgtataaac attagttcat tacaccaaga aaaaagaaaa 57660
   atccttcagg aataatgctt cttgaatttc cactacaaat catttttcac tcattatcca 57720
   tttctcagaa tagttttgtg aaataaataa cattgtaact tcagaaactt gaggcacagc 57780
   aaatatgtga caaaacccac ttatgcttaa ccttgtttga tttccttctt ctactttcta 57840
   atgttacatc atttctttcc catgtaagat tcacttaaaa agaaggaagc cagattttgt 57900
   tttttagata ctttcttgga ctttctcttt gtcactaaat aaactttaaa tacaaatgaa 57960
   aaactttttg ttttagggaa aagacttttg ttctgacacc ggaacttagt cctcggaaac 58020
   ttcagggttt accttttgaa aaagcctcag tgtgtcatta tcatgggatt gagtaagtct 58080
   ttatttacac tttactcttc tgcgtgttgc tggtgaagct tttctaatgt gagtgtgaag 58140
   agtatctgta ataaaatgaa gatataaagc caaagagaaa gtatctcaaa aagtctcaga 58200
   aaacaaagca gtgtgtacac aaataggaat ttgtcttcta ttgttaaaaa aataaaaagt 58260
   tatttgatga ttaatcagtt ttacagcatt taaacattca attaccagat attttaatca 58320
   aaattatttt gcccctatgg atttgtattt atactactgg tcacattaat attccctggt 58380
   cactaattac attgtaatta ctcagaaact attagatgaa atgtattccc atttgtaata 58440
   tataattatg tgtaaatttt tctagatttc taattaacca gtttgaataa aatttcaaag 58500
   aattctcctg cataaagtta ttagcagttt ctgaggttat gggcacatct accattttca 58560
   caagaacaag aaatgtaatg acggtgataa atttttaaga ccattttaag accaatgtaa 58620
   aaaattatgc atgcttaaag caaaacctta tttcatattg attttccata aatgaataga 58680
   aaaggacttg attttacttt aatgtttagg aagcacctta tcaactgaaa cctactattt 58740
   gattggcttg taaacttaat ctttatttca aataaacttc aaaaatactg aaaattgttc 58800
   actcagaagc taaaggtaca aaagatgatt atttctgccc ttaagcagca tatactctct 58860
   aggggaaaaa gaaaaaacac ataattttag cagattataa ataatgttaa aatacagatg 58920
   tctgcccagt agatagaaat aaatgccagg aactatttga gtgtttcgaa gaagaaatct 58980
   gaactaagtt tgaaagaatg aataggagtg cacctggcag aaaaggcaat gtttgcagga 59040
   ggaacaaagt acagagttac agattcttgg aagataaagt acatttttat ttttgaataa 59100
   gagtgacaaa catattatac tataatgaga ccaaatcatg aagtcatgaa ggtcaccggt 59160
   gacatgggag ggagattgat atttattttt gagaagtaaa agacctttat ttttaaagca 59220
   tgagattaaa tgcttgggtt tgtaccattg aagaaatctc attggctttt aataaattga 59280
   gttgaagtat ttcaagcaga tgttgaagga ctaaaatgct atcaaggcag tgaaaatagg 59340
   aaagagaata taaacttaaa gttaactaaa gaactttaga ttagttagat gttggggatg 59400
   agaagggagg atacaacaag aatttcttta gggtttctag cttaggcaca ttggaaagat 59460
   gatggtacag ttgagaaaat atggaaggtc actcaacaat cattctagga atgtttagtt 59520
   tccttgctat gtttaaataa gatttatttc attgaaagag tatgtaagtt tgacactaga 59580
   ggcaaaatat tcagctgttt tttttttcaa gcatcgaaat taaattccca ttaaagtccg 59640
   gtgcaagaaa aagataactg ctataaaaaa aaaaaaaaaa aaaaaaaaaa aagataacct 59700
   gctatcagta cttcttttct ttttcaccca ttgtgctgaa tatttctgtt ttaatcattg 59760
   ctttggctaa tcagttaaaa taaagaaaac tagagagttc aaaagagtta tacatattga 59820
   aaaagagacc ataagaaatt tttttgctgt ttttttgttt gtttgtttca tagagaagag 59880
   acctgaaaga ttattagaag tggtaaattt gaaaagtgac cagtgataaa atatatataa 59940
   aaagcctagt ttacctctaa accggccatt tccaatttta aacaccctga gaagagatct 60000
   tattgaaaat agcaatgaaa ccataaatat tagaactatt tttataagaa atgtagaatg 60060
   gaaaaaattg caaataatct atattagggg acataaaagc aacattgatt acttaaggga 60120
   aacatgcctt atttccctgt gcacagattc catattctga aggcattagt tcttttggaa 60180
   taaatataaa tatttaatat aattcttaac aactttgaac cttcagttga aatagttcta 60240
   taatttatct ggtaagaagc ctctttcctt ttacttaaag aatgagatga attttatcat 60300
   ataaccagaa agttttatta taaaggtatt ataataatag tataagagtg aaactaagta 60360
   ggaaatgcaa ggaaaaacca aatatacata ggaattcagt ctaatagggg aaaattttca 60420
   aattagagag caaaagataa aatattcagt aggtagtagt gagaatatga ttaaatatgc 60480
   aaataaaaat aattttaagt gattcttagt tcacactaaa taccaaaata aatcgcagat 60540
   gtattcagaa tttaaagtga aaaactgtga aatgtttaag tataaaatgt aaggaaaaat 60600
   ctgttttaag tgtagggaag atttttattt cattctgaca gaggaagaaa caaacaaaaa 60660
   aaaggaatga tagatttttc ctaatcttaa aacagcaatg tcagtaatca acagaaacaa 60720
   agaaagcttt aaagtaagga ttaagctgag gaaaatattt tttaacataa atgagaaggt 60780
   attttataat taaaaaatac ttgggctact gggtggctca gttgggtaaa catctgactt 60840
   ttgattttgg ctcattgtga tctcagggtc atgggatcca gcccagtgtc aggctctgca 60900
   gtcaacaggt gcagagtctg cttgcgatct ccctcagctc ctccccctgc tcatgcatac 60960
   ttgttcactc tgtttctgtc tctcaaaaat acatctttaa aaaaaaaaaa aaaacttaca 61020
   aatcagtaag aaaataaaat aaatgcccct atggaaaagc tggcaaaatc ttgaaccatt 61080
   tgtttaccaa agaggaaata caaatgactg attcacatga caagactgat aaatagtgac 61140
   aagtatatgg aacagtcaga actgtcatac attgcttgtg ggagtataaa atggtatctc 61200
   tactccagaa aaggatctgg gaggttctta taaaattaaa catactcaga tcctttggta 61260
   catcagttcc aatactagac attttcctaa gagaaatgaa aacacaactc cagggtcacc 61320
   tgagtggctc agtcagttga gccatctgcc ttcaactccg ggtcatgatc ccaggtcctg 61380
   ggatgaagcc cccagccccc cattcaggct ccctactcag tgaggaatca cttctccctc 61440
   tctttctgcc tctcccccag gcttgtgctc tctgtcttcc actctgtctt gcaaataaat 61500
   aaaataaatc ttaaaaaaaa aagaagaaga agaagaaaaa aaaacacaac tccgcaggaa 61560
   gagttgaaca agatcatatg gagcagtttt atccataata gccccaaact ggtaacagcc 61620
   taggtgtacc tcagcagaat agataaataa actggtgtat gtatacaaag cagtactcct 61680
   cagcagtaac aagaaacaac acgtcaatac atgtggtaac attacatttc tgtctgcatg 61740
   tggaacaaca gttgaagtaa gaatttgagg actttaaaat gagcaataag aactacctac 61800
   ccctataaac aaacaaatct ctctgtgact cagttttctt tattctaaat ttgtgctcct 61860
   taacctgaag gcacagatca aaccaaggat gtggtagagg ggtgagcaca gtgaatccag 61920
   gaacatgctt tttcttcaag aagatttgag catgtagagg gcagagggac tatttggttc 61980
   aacatagata atgaacctac ttgaaacaaa attctagtag aatgttaaaa taattatgaa 62040
   gctaaaggga gatttttctc tcttcctcat gttgctataa gcctttgtaa tccctgaact 62100
   cttcctgtac ccagacataa agaatatttg ggaaaaaata tatatataca taaactgccc 62160
   aagtgcaaat cagtgttttc ttaaggcaaa atgaaatatc tccactgttg gcttgcttga 62220
   tttttcacct attttatcct ggagtgttag ataccattga ttgtatggtg cactgttttc 62280
   tttaacaaca aattaaactc tactatcaaa ctgtgatatt ttgccatgcc agcctcctgt 62340
   tgctttcaga tttctttcat ctgctccaag agatttggca gtttcccctg ccttcacaga 62400
   cattgatgca tgctaggcag tatcttgcct attatgtagt taccaaacaa agatagcttc 62460
   atcttgaaga tacttttctt cctttggttg cataaagcat ttggttattg ttttgtaaga 62520
   aaatagagaa gtgtagtcat tcatccaacc atgaatgttt gctccactaa taccagatat 62580
   gcaccctgcc attctgtttc tgttcctttc ttatttgcaa agtacttttt gtttcagtgc 62640
   cagatcatag tataatcatt tgaggacatt taaaatggca aagtcaacaa gcatagtaca 62700
   cgcagttcag ttgaaggaat gacattatga acagctgtga tgaagaaagg ttagaactgg 62760
   aaggaaaata tctgcatgtt aaaatcaaaa tctcagagta gaaaaaaata ctcatttaga 62820
   gtgaaactgt atcttttttc tcaaaaattt cctataagct cattaaggca ataaacattg 62880
   cctcttgcct agaacctttc ttaacattca tggttcttga tttcattttc aattctctca 62940
   gttgcccagg agagaaatag gactcatcag taatccttag ggttactgct cctttttaat 63000
   gaaatgtgtt ttattaaata acgctagaga aagaatgtta cagaactgag aagcctgggt 63060
   tctagttacc attctgccac cactttctcc tctgttaaat taatataacc cctgggttaa 63120
   attagagatt tatcagtccc aatcacctgg acagcttttt aaaaaatgtg catactagaa 63180
   gatttgctgt ggtagaaatt aaacctaata ataataataa taataataat aataataata 63240
   ataataatag ccacgacagc agtagcagca tacatttgtt gagctttgct tgcatggtct 63300
   aggcactgat tctgagtgcc ttactttacc caaggaatat gtaaggtatc cttatactag 63360
   ctcttggaga tgactctata aataaagctc attttgcaga ggaggccaac agaagcatag 63420
   atcaactgaa gtgattgggc caagttcaaa ccacctaatc ggtgacagag ccagatttaa 63480
   atcctgggat tcagctctta gaaatcaaat atgaaagtgt cccaaaggga aagaaagagt 63540
   ccttaaaact tccatgaggc agtcagaagt tagggtaatg ataacactct ttgggagttg 63600
   agctaaaagc ttatggacgt aaagttctag ttttagggag caccttagaa ttttacaaat 63660
   caagaggaca gataacagag aaaggtttta attttgacag tcctcctagc acttctctga 63720
   agctgttgtg tttgcctgat gcagctgtag cagaggagac aatgaggagt tgtccagggt 63780
   cacatatctc atgggtgatt ttcctgatat gcttatttgt tgtccttaac aacatgggat 63840
   ttctttaaga ccagaggcat tcactgagcc aacatctcat tgattccttg aatcagatct 63900
   agctagaaag cagatgcaca gggacagcca aagcgtttga agaaaaatct gtcaatcaga 63960
   agtaagggtg gggagtgagt ttcaaagaga aattcagaat gttctgagct gtgtatggtg 64020
   agccagttcg aataccttta tacccttttc atctttgtgt tgaacataaa cttgcccttt 64080
   ctaaccgaag tcagcatttc aacttgttac ataccagcca tgctgaggaa tcagatgtcg 64140
   gtagagactt aaagagtatt aaagcagcag ctctattatt ttgaaaataa gccaggatta 64200
   acttaatctg ttgcccagaa agagtcaaag aggaatcaga tcatacttta aatattttaa 64260
   tcttttgccc tgtcatccag caggctagaa aaaaacgaac ttgtgaagga agccggccgt 64320
   tccctccgcc cttccaacag ttattaggaa gttcagtaaa aagttttcaa ggaaatgttc 64380
   cgctgtttcc tcactggcag ggaaaaactt gttgctgttg tgaagcctgt gattttaatg 64440
   gtctttcttt tctttagata ctgtttggat gaccaaaaag cattagaaag agatggggga 64500
   ttctctgaac ttcagtctcg tctcatccgt tatgaaaccc aaaccacctg cactagagaa 64560
   agttttccgg tacccactgt gttgagccct cttccatcac ctgccgtctt gtcagagcct 64620
   ggcagtgtcc ctgatggaga agctttacaa agcgagctcc gcactgaagt atctcgattg 64680
   aaaaggggat caaaagatgt gaattgcctt tatcccccaa aaaggtgcta tattaagtgc 64740
   ataaaaatag tcccttgaca gaaagtgtgt tagtccctaa ttttttttaa atttttttgc 64800
   aaattagtct ttcttatttt taagtagcat tctgtagaga tcttagatga attctttccc 64860
   atcaggaatt ttgtttttac tgtgagcagt tatgattatg cttataaatt ataaaaagaa 64920
   atgacttgga aaaaaaaaca caatttcttg ctttctgtgc taatatacag aacattagca 64980
   tgatggatta taaaattgtt aagccatgga acactagaga tcttgtccca taaattaagt 65040
   agcagagctg ctttgttgaa aaggatgtct ggggatggca gctttcctgc taggtcttcc 65100
   caccttcttg cactgctttc tgcctgtttg attttgttgg gtttatgttt tagtggatat 65160
   agttgtcaca tttagggcag aaaaatggac ctcattattg agtcacttag tctggttttg 65220
   gatttatttc tccacaatag acttgtgaag tctgaaagtt cggattctct tctttctcaa 65280
   acaagtggca atactaatcg tcgtcatcat gcagtgacat ccagaaaacc ccgagcagag 65340
   cgatcattat ctgtgacttg tccatcagtt ccagttctta gctgtgaaac ttcaaaagtc 65400
   actacaaagg ccagttcagg tcaaaaaagt gttcatggat caaaaacatc aaggcaaatt 65460
   aaggaatcaa gatcacagaa acacacacgg gtaaagattt atttcctgtc tcttagttct 65520
   acatgggaag aaactccttt atttggttca tttaaaatgt atgtcgaaaa ttttatgagt 65580
   gaaatgaaat ggtatgtata atgtacttta agataattca acataagaag gacagtgggt 65640
   aagtaggcag agggataaat gaaacaaaat tagccacaaa ttgatcctca ctgaaactgg 65700
   tttgatatca agatagggat ttgtcattaa atgttctctc tacttctcag tgtttgaaag 65760
   tttccataat aaagaataaa tgcatccctg tgtatatcct taatgtgtgt agtttttaca 65820
   atcagatttt gattcagaac ttatgatttg aattcctaag atactctagt tatttcttcc 65880
   taatagatac ctggtgacat gtatgaccat gtaaatgttc caggtctctt ttttttctca 65940
   gttattttgt cttaatgaac ttagctttca cttagttaca tgattatata attcacacaa 66000
   ctgaactcta atgatgaaat aatatgtttg taccttttcc aagaaatatt atcatatgtt 66060
   atgttctatt caagatactg aaagaagtag ttacagaaac actgaagaaa cacagcatta 66120
   ctgaggctca tggatgtttc actgcctgca gccaacgtct ctttgagatc tccaagttct 66180
   atctgaaggt actgtgtctt ctctattaag agcaaaattg aggtcatatt ttgatattct 66240
   tatctgacat aaagtaatat ttagtgagta atatttagtt tatattcatt ttataatgat 66300
   agcttataac ttgttgccag atattttttt aagtggcaat agaaaaattg gagccaaaat 66360
   attttatatc tgacttgttt gatttttgtc ctcatgttta gaaaactaca ttttaactgg 66420
   tatcttccct aacaagtctt tgacttaagt tgataataaa cttcaaaaca tgtttaatta 66480
   gaattttagt atatcgtaaa aaggcaactg gaatgggtat cagaataccc cgtttctaat 66540
   tttggatcca ttatcagcta attgtgagac attagacaga ttagccactt agatgatggt 66600
   tctgtaaaac ttaaatggtg tgggcagcca cagtggcaca gcggtttgac accgcctgca 66660
   gcctgggatg tgatcctggg gacccgggat cgagtcccac atcgggctcc ctgcatggag 66720
   cctgcttctc cctctgcctt tgtctctgcc tctctctctc tgtgtctatg aataaataat 66780
   aaaatcttaa aaaaaaaaaa acttaaatgg tgttagattg tctccagctt taagagtttt 66840
   tgactcaagt cagagttttg tgtgggttcc tggttaaaac tacagatgac cacatgcatc 66900
   caattcgcct tctttctaaa atctcaagaa aaatggtatt aaaatacaaa aataaagaag 66960
   agggggaaaa aaatggagag gtatatttgg tacattgttg aacaagacag cccatagaag 67020
   tttggtatca cagagtttaa aaagttggaa cctaaatgcc tgaccagtgg aatgctagtg 67080
   agaatcaaac catgtccaat gacacagtct ctgaggttct taggatttga agacattggg 67140
   tatcatggaa agcgggggtg tggtgtcagg tggaacatag gagagttgta taaaaatcta 67200
   cagtgaactg aggaaccccc aacttcctct ccaagtcttt ctcccacagg ggtaactacg 67260
   aagtatgctg ctactacttc ttgccctcat ctctgtgaca gtgcaggaaa tagtctctga 67320
   agcaaaaaga ggggcacaag ggaaaaacca actggcctcc taataacctg accatataaa 67380
   gccactagac agagctttca ataagatttt tacatctcag ttttaaatat gattggactt 67440
   ttgagaatta gcagaattag cagacttttg agacttttcc caaaaaaaaa gatgccaaaa 67500
   caaaggaatg aagggaactc ttaacgggca gaaagctata gaaaacttca taaaaactat 67560
   aattctctca gaaagaagat acttgcatcc ataaagctgg aacaggatgc tatttccaga 67620
   aactctatag aacatgaaag cttgaaatga aaaatatgat aaaatcaatc caatagtgac 67680
   actgaacagt tgagattaag aaagttacca ggtagggacg cctggctcag ggcgtgattc 67740
   tggaatcccg ggattgagtc ccatattgat ctccctgcat ggagcctgct tctctctctg 67800
   cttgtgtctc tgcctctctc tctctctgtg tctctcctga ataaacaaat aaaatctaaa 67860
   aaaaaaaaaa aaaaaaagtt accaggtatt aaaatcaggg catgcctgga tggctcagtt 67920
   ggttaagtgt ctgtctttgg actcaggtca tgatccccag ggtcttggga tcaagctatg 67980
   tggcgggctt ccagctcagc agggaacctg cttctccctc tgctgtcccc tgcttgtgtt 68040
   ctctctctct ctctctcaaa taaataaata aactcttaaa aaaaaaaaaa agaaattaga 68100
   atcaataaca aaaacgtgaa atataagaat cagaaaaggg gaagactaat ccagagacta 68160
   atagactaat tagactaata tggtaatatg gtaatagaag ttttaaaaat agagaataaa 68220
   ggagagcaaa ctatcaaaaa tacaacaaag tttttcccag aactgaagaa ctcaagtgcc 68280
   atgattgaaa aggcacacag cttatccatc ccagtgaatg acagagttct gcagcaagct 68340
   acattattgt ggaatttcat acttcctgaa atagcctcct aaaatcttcc caggaaagaa 68400
   agtttacatg tagagaactg agaatcagaa tggcattgta cttctccata gtaagtaatg 68460
   tatgccagaa gccagaaaag catcttccaa tctgagggaa actgatttac ctagtttttt 68520
   ataaccagtc acaaattcaa tcaagtctga ggattccatt agagatactt cagatgtaga 68580
   aagtctaaaa aagtttgtca ttcatatacc cttttaaagc atctgcaaga ggatgaagtc 68640
   catcagaata gcaactgagt caagaaataa gaactaaccc agggagcagt tagaccaaat 68700
   tggagcaggt tgtgggagaa agagcaccag gtgaaaaaat acgaataata atgaacagat 68760
   ttcttgaaaa attgaactga gaggttatcc agtatatttg aaacaacttt gctatttgta 68820
   catttaattg attgtaacat ttggtggggg agacccagaa ttacatacag ggcaaactaa 68880
   gcaaatataa ctagaggtta aacaaaatgt tacatgagaa gataattata ataacattac 68940
   ttaactcaag acagaactgt atttacatag gtataaaatg caaacatgaa ctatttacta 69000
   atcaaaactg gtgtagtagt tactaagagg aagaaggagg tggcctggtt cagagatctg 69060
   aatccacaac ctatgtagct gttaacaatg atcattgtct aaaattgcta aatcaaaaag 69120
   tagcaacata agcatattta gaaacatgaa gatataaata agagaaacag ctgaaagaac 69180
   caaaagtggc agtctgtcct gtgggaagca taacttctag ggacagcaga ggactgcttt 69240
   ttaaaataca attagatttt ttaaatttac cttgtaatct aatttgatat aatcatattt 69300
   tttaaaagtt agagatttcg gtttcttttg tcttggttgt ggagtaaaat aacccaaaag 69360
   gagagagaga aaaaaaagat attttatctg aaaaataaat acaaatgcag tatattcatt 69420
   tcccgagtac caaagcatta gtgggtactc atataatcaa agatgagact tagatttggt    69480
   cagtgctgct gagattatga ggcaagagtg acagaaattt atatatacat atatatatat    69540
   tagattagat tatatatata catacatata tattatacat acatttatat gttatatata    69600
   catatatttt taaacattta aatatacgta tatatgtata tacatatata tatataattg    69660
   ttgttagagt atcttgatat ttagggttag agttaaattg tacttgattt tttttataat    69720
   gttttctgag tcacattgat atttatagaa tgatttagga taatatcaag tttatttaat    69780
   gatgacttgc tctctgtaat ttaatgtaca actcgtgaaa tgctttatgt tgttattttt    69840
   aggatcttaa aacatcaagg ggtctattcg aagaaatgaa gaaagcagca aacaacaatg    69900
   ctgtacaggt aaataactta ttcccaagaa actacattga attaaagtgc tattttattt    69960
   tagggtagct gtgatgccat tatgtatgct gtgtaagaac tctctttaaa ataataatag    70020
   actaatgcaa acagagcgtt taggagtaca taataggctg ctctgttaac aaaagggttt    70080
   ctaaagatta tgttaagaaa gaagaaactt ttttaataaa ccaatgacct ggaaaaatta    70140
   atccattttg attagttgtt ctcaaagttt ggtttaattt ctttctagtg accctttgaa    70200
   aacaattgca aaaaaaaagt gagacaacat ggggcacctg ggtggctcag atggttaagc    70260
   agctgcctgt ggctcaggtc atgatctcag ggtcctggca tctagcccca catcaggctc    70320
   ccagctcaag gggagtctgc  cctctccctc tccctctgcc tctccccctg  cctatgctct    70380
   ctatgtctct gtctctgtct ctctctcaaa ggaataaatt taaaaatctt taaaaaaaac    70440
   taaggtgaga aaacagaatc tttattgtat ccagtgagga cagttgtact ctgcagcaga    70500
   agtctagctt cagttttatt ttcttcagtt agaaaataaa atgtgtttaa gataaaatgt    70560
   gatttaagat tttgacttgt tacttcatat tttaagtttt ctaccatgct ttttaaagtt    70620
   ttaactgtat ctttttctgt cccttctgtt ttcaatagaa ataaaccatt accgtatttt    70680
   ttttttcctt ctaggtgatt gaatgggtat tagaaaaggc aagcaagaaa taacacagaa    70740
   tccttctctt tggacagtta taaattggcc agagctatca tttgctacct ccttccttgg    70800
   ttgacaatta taagcaaatt tcaaacttta ttcaaagaat agatatttct aaagaatttc    70860
   ataaatatcc aatgtttatg tagttttgag ggtgttcagt taatgtgttt tttaattgta    70920
   tacttatttt ataatttcac tatattttca tttttaaggt taatattttt ata           70973
<210> 2
   <211> 4206
   <212> DNA
   <213> shar-pei dog
<220>
   <221> CDS
   <222> (1)..(2697)
<400> 2
   atg gat cgg tac ctg ctc ctc gtg atc tgg gga gaa gga aaa gtc ccg         48
   Met Asp Arg Tyr Leu Leu Leu Val Ile Trp Gly Glu Gly Lys Val Pro
   1               5                   10                  15
   tcc gcg gcg ggc ggg gag gcg gag tcg gcc gcc gag ggt acc gag aag         96
   Ser Ala Ala Gly Gly Glu Ala Glu Ser Ala Ala Glu Gly Thr Glu Lys
               20                  25                  30
   ctg ccc gac ctg aca gca gca aat gtt tat cat gtc ctg aaa aga agc        144
   Leu Pro Asp Leu Thr Ala Ala Asn Val Tyr His Val Leu Lys Arg Ser
           35                  40                  45
   att agt gct tca atg aat cca gaa gat agt act ttt cca gca tgt tcg        192
   Ile Ser Ala Ser Met Asn Pro Glu Asp Ser Thr Phe Pro Ala Cys Ser
       50                  55                  60
   gta ggc ggt ata cct ggt tcc aag aag tgg ttc ttt gca gta cag gca        240
   Val Gly Gly Ile Pro Gly Ser Lys Lys Trp Phe Phe Ala Val Gln Ala
   65                  70                  75                  80
   ata tat gga ttt tat cag ttt tgc agt tct gac tgg gaa gag ata tat        288
   Ile Tyr Gly Phe Tyr Gln Phe Cys Ser Ser Asp Trp Glu Glu Ile Tyr
                   85                  90                  95
   ttt ggt aca gaa aaa gat gaa att gaa gat gtt ctt caa aca aat att        336
   Phe Gly Thr Glu Lys Asp Glu Ile Glu Asp Val Leu Gln Thr Asn Ile
               100                 105                 110
   gaa gaa tgt ttg aat act gag tgt ttt gag gaa gaa gat agt aat agc        384
   Glu Glu Cys Leu Asn Thr Glu Cys Phe Glu Glu Glu Asp Ser Asn Ser
           115                 120                 125
   aga gaa tca tta tct ttg gct gag tta tat gaa gaa tct gca gaa aat        432
   Arg Glu Ser Leu Ser Leu Ala Glu Leu Tyr Glu Glu Ser Ala Glu Asn
       130                 135                 140
   ttg cat cag ttg tca gat aaa ctt ccg gct cct ggt aga gca atg ata        480
   Leu His Gln Leu Ser Asp Lys Leu Pro Ala Pro Gly Arg Ala Met Ile
   145                 150                 155                 160
   gat ata ata ctg ttg cct tct gac aaa gat cct cct aag ttg aaa gac        528
   Asp Ile Ile Leu Leu Pro Ser Asp Lys Asp Pro Pro Lys Leu Lys Asp
                   165                 170                 175
   tgt tta cct act ata gga gca tta aaa cat ttg aag gaa tgg tat tca        576
   Cys Leu Pro Thr Ile Gly Ala Leu Lys His Leu Lys Glu Trp Tyr Ser
               180                 185                 190
   gca aaa att act ata gca gga aat cat tgt gaa ata aat tgt cag aaa        624
   Ala Lys Ile Thr Ile Ala Gly Asn His Cys Glu Ile Asn Cys Gln Lys
           195                 200                 205
   att gca gaa tac ctc tct gct aat gtt gta tct tta gaa gat ctc aaa        672
   Ile Ala Glu Tyr Leu Ser Ala Asn Val Val Ser Leu Glu Asp Leu Lys
       210                 215                 220
   aat gct att gac tca aaa gaa tta tgg agg gga aaa att cag ata tgg        720
   Asn Ala Ile Asp Ser Lys Glu Leu Trp Arg Gly Lys Ile Gln Ile Trp
   225                 230                 235                 240
   gaa aga aag ttt gga gtt gaa att agt ttt cct gaa ttt tgt tta aag        768
   Glu Arg Lys Phe Gly Val Glu Ile Ser Phe Pro Glu Phe Cys Leu Lys
                   245                 250                 255
   gga gtt aca ccc aag aat ttt agt aca tct aat tta aat cct tgc att       816
   Gly Val Thr Pro Lys Asn Phe Ser Thr Ser Asn Leu Asn Pro Cys Ile
               260                 265                 270
   cta acc aac aag gta ata cca tca aag gat aag aat att ttg cca aag       864
   Leu Thr Asn Lys Val Ile Pro Ser Lys Asp Lys Asn Ile Leu Pro Lys
           275                 280                 285
   gtt tac cat tat tat ggc cct gct tta gaa ttt gtg cag atg ata aaa       912
   Val Tyr His Tyr Tyr Gly Pro Ala Leu Glu Phe Val Gln Met Ile Lys
       290                 295                 300
   tta tca gat cta ccc tcc tgc tat atg tcg gac ttc gaa ttt gag tta       960
   Leu Ser Asp Leu Pro Ser Cys Tyr Met Ser Asp Phe Glu Phe Glu Leu
   305                 310                 315                 320
   ggg ctg aca aaa aac aat acc aaa cag aat tcc gtg ttg ttg ttg gag      1008
   Gly Leu Thr Lys Asn Asn Thr Lys Gln Asn Ser Val Leu Leu Leu Glu
                   325                 330                 335
   cag att tcc tct ctg cgt ggc aag gtt ggt gca ctt ttt gta ttg ccg      1056
   Gln Ile Ser Ser Leu Arg Gly Lys Val Gly Ala Leu Phe Val Leu Pro
               340                 345                 350
   tgt act att agt aac ata att att cca cct ccc agc caa ctc agc tca      1104
   Cys Thr Ile Ser Asn Ile Ile Ile Pro Pro Pro Ser Gln Leu Ser Ser
           355                 360                 365
   aga aaa tgg aga gaa tac ata gct aaa aag cct aag aca atc agt gtt      1152
   Arg Lys Trp Arg Glu Tyr Ile Ala Lys Lys Pro Lys Thr Ile Ser Val
       370                 375                 380
   cca gat gtt gaa gtg aaa gga gag tgt tct agc tat tat ctc ctc tta      1200
   Pro Asp Val Glu Val Lys Gly Glu Cys Ser Ser Tyr Tyr Leu Leu Leu
   385                 390                 395                 400
   caa ggt aat ggt gac aga aaa tgt aaa gcc aca ttg att cat tca gcc      1248
   Gln Gly Asn Gly Asp Arg Lys Cys Lys Ala Thr Leu Ile His Ser Ala
                   405                 410                 415
   aac cag atc aat ggc tca ttt gca ctc act tta att cat gga aag atg      1296
   Asn Gln Ile Asn Gly Ser Phe Ala Leu Thr Leu Ile His Gly Lys Met
               420                 425                 430
   aaa gca agg aca gaa gaa gcc aaa ctg agc ttt cct ttt gac ttc ttg      1344
   Lys Ala Arg Thr Glu Glu Ala Lys Leu Ser Phe Pro Phe Asp Phe Leu
           435                 440                 445
   tca ctt cca cat ttt tcg ggt gag cag ctt ata cag aga gag aaa cag      1392
   Ser Leu Pro His Phe Ser Gly Glu Gln Leu Ile Gln Arg Glu Lys Gln
       450                 455                 460
   tta gct aat gtt caa gct ttg gct ttg aaa gaa tgt ctg aaa agg aga      1440
   Leu Ala Asn Val Gln Ala Leu Ala Leu Lys Glu Cys Leu Lys Arg Arg
   465                 470                 475                 480
   aag ttg gca aag cag cct gaa gca gtt tct gtt gat gaa ttc aaa agt      1488
   Lys Leu Ala Lys Gln Pro Glu Ala Val Ser Val Asp Glu Phe Lys Ser
                   485                 490                 495
   cta ttg ata ctc aca agg gaa tcc ttt tta gag cat ttt gat gct gtt      1536
   Leu Leu Ile Leu Thr Arg Glu Ser Phe Leu Glu His Phe Asp Ala Val
               500                 505                 510
   att cct aaa aca att cca ata aag aca gac aaa att aaa acc tcc aat      1584
   Ile Pro Lys Thr Ile Pro Ile Lys Thr Asp Lys Ile Lys Thr Ser Asn
           515                 520                 525
   atg tta aat gat gcc agt tca gtg gaa tct act tct tca aat cta atg      1632
   Met Leu Asn Asp Ala Ser Ser Val Glu Ser Thr Ser Ser Asn Leu Met
       530                 535                 540
   gaa acc aat gct ctg gaa tgg cca gaa agg cat gtt ctt caa aat ttg      1680
   Glu Thr Asn Ala Leu Glu Trp Pro Glu Arg His Val Leu Gln Asn Leu
   545                 550                 555                 560
   gaa agt ttt gaa aaa gct aag caa aaa atg aga act ggt tca tta cct      1728
   Glu Ser Phe Glu Lys Ala Lys Gln Lys Met Arg Thr Gly Ser Leu Pro
                   565                 570                 575
   cgt tca tct gag cag ttg ctg ggc cac aaa gaa ggg cca cgg gac tcg      1776
   Arg Ser Ser Glu Gln Leu Leu Gly His Lys Glu Gly Pro Arg Asp Ser
               580                 585                 590
   atc aca tta ttg gat gct aaa gaa tta ctg aaa ttc ttt act tca gat      1824
   Ile Thr Leu Leu Asp Ala Lys Glu Leu Leu Lys Phe Phe Thr Ser Asp
           595                 600                 605
   gga tta cca att gga gat ctt cag cct tta cag att caa aag ggg gaa      1872
   Gly Leu Pro Ile Gly Asp Leu Gln Pro Leu Gln Ile Gln Lys Gly Glu
       610                 615                 620
   aag act ttt gtt ctg aca ccg gaa ctt agt cct cgg aaa ctt cag ggt      1920
   Lys Thr Phe Val Leu Thr Pro Glu Leu Ser Pro Arg Lys Leu Gln Gly
   625                 630                 635                 640
   tta cct ttt gaa aaa gcc tca gtg tgt cat tat cat ggg att gaa tac      1968
   Leu Pro Phe Glu Lys Ala Ser Val Cys His Tyr His Gly Ile Glu Tyr
                   645                 650                 655
   tgt ttg gat gac caa aaa gca tta gaa aga gat ggg gga ttc tct gaa      2016
   Cys Leu Asp Asp Gln Lys Ala Leu Glu Arg Asp Gly Gly Phe Ser Glu
               660                 665                 670
   ctt cag tct cgt ctc atc cgt tat gaa acc caa acc acc tgc act aga      2064
   Leu Gln Ser Arg Leu Ile Arg Tyr Glu Thr Gln Thr Thr Cys Thr Arg
           675                 680                 685
   gaa agt ttt ccg gta ccc act gtg ttg agc cct ctt cca tca cct gcc      2112
   Glu Ser Phe Pro Val Pro Thr Val Leu Ser Pro Leu Pro Ser Pro Ala
       690                 695                 700
   gtc ttg tca gag cct ggc agt gtc cct gat gga gaa gct tta caa agc      2160
   Val Leu Ser Glu Pro Gly Ser Val Pro Asp Gly Glu Ala Leu Gln Ser
   705                 710                 715                 720
   gag ctc cgc act gaa gta tct cga ttg aaa agg gga tca aaa gat gtg      2208
   Glu Leu Arg Thr Glu Val Ser Arg Leu Lys Arg Gly Ser Lys Asp Val
                   725                 730                 735
   aat tgc ctt tat ccc cca aaa aga ctt gtg aag tct gaa agt tcg gat      2256
   Asn Cys Leu Tyr Pro Pro Lys Arg Leu Val Lys Ser Glu Ser Ser Asp
               740                 745                 750
   tct ctt ctt tct caa aca agt ggc aat act aat cgt cgt cat cat gca        2304
   Ser Leu Leu Ser Gln Thr Ser Gly Asn Thr Asn Arg Arg His His Ala
           755                 760                 765
   gtg aca tcc aga aaa ccc cga gca gag cga tca tta tct gtg act tgt        2352
   Val Thr Ser Arg Lys Pro Arg Ala Glu Arg Ser Leu Ser Val Thr Cys
       770                 775                 780
   cca tca gtt cca gtt ctt agc tgt gaa act tca aaa gtc act aca aag        2400
   Pro Ser Val Pro Val Leu Ser Cys Glu Thr Ser Lys Val Thr Thr Lys
   785                 790                 795                 800
   gcc agt tca ggt caa aaa agt gtt cat gga tca aaa aca tca agg caa        2448
   Ala Ser Ser Gly Gln Lys Ser Val His Gly Ser Lys Thr Ser Arg Gln
                   805                 810                 815
   att aag gaa tca aga tca cag aaa cac aca cgg ata ctg aaa gaa gta        2496
   Ile Lys Glu Ser Arg Ser Gln Lys His Thr Arg Ile Leu Lys Glu Val
               820                 825                 830
   gtt aca gaa aca ctg aag aaa cac agc att act gag gct cat gga tgt        2544
   Val Thr Glu Thr Leu Lys Lys His Ser Ile Thr Glu Ala His Gly Cys
           835                 840                 845
   ttc act gcc tgc agc caa cgt ctc ttt gag atc tcc aag ttc tat ctg        2592
   Phe Thr Ala Cys Ser Gln Arg Leu Phe Glu Ile Ser Lys Phe Tyr Leu
       850                 855                 860
   aag gat ctt aaa aca tca agg ggt cta ttc gaa gaa atg aag aaa gca        2640
   Lys Asp Leu Lys Thr Ser Arg Gly Leu Phe Glu Glu Met Lys Lys Ala
   865                 870                 875                 880
   gca aac aac aat gct gta cag gtg att gaa tgg gta tta gaa aag gca        2688
   Ala Asn Asn Asn Ala Val Gln Val Ile Glu Trp Val Leu Glu Lys Ala
                   885                 890                 895
   agc aag aaa taacacagaa tccttctctt tggacagtta taaattggcc                2737
   Ser Lys Lys
   agagctatca tttgctacct ccttccttgg ttgacaatta taagcaaatt tcaaacttta      2797
   ttcaaagaat agatatttct aaagaatttc ataaatatcc aatgtttatg tagttttgag      2857
   ggtgttcagt taatgtgttt tttaattgta tacttatttt ataatttcac tatattttca      2917
   tttttaaggt taatattttt atatatgttt ttaaattttc actcttggtt atttaaaaat      2977
   ttccaataaa tggaaaatat tatttatttt cttcttggta aatgtctttg ttgttaggcc      3037
   tatttaaatt actgaatctc aggtaagatt gccagtggct tttctcatgt gacctctcag      3097
   catacttaat tggacagtac ctggttttaa aaatattagt aaaaaaattc acacaggtag      3157
   ccttcttccc tcacccctgc cccagagcca gtactgttgg agctatcagt gtttatttgg      3217
   gattaaaaat gaagaaaggt tagaaattgt gaaaataaga tatagtaaaa attactaagt      3277
   agagactgcc agaaaaatat ggaaaatgca gaaaataaaa aacagagtat aaacaaattc      3337
   caacattgcc tcaatgtatg taaccacagt tgatatattt tttaaagatt ttatttattt      3397
   attggcagag agggaacaca agcaggggga atggtaggca gagagagagg gagaagcagc     3457
   ccaaagtagg tcttgatccc aggacctggt catgacctga gccaaaggca gacacttaac     3517
   caactgagcc acccaggcac tccaccacat taatattttt aaaatatata tgtgggatgc     3577
   ctgggtgact cagtcagtta aacattgcct tcagctcagg tcatgatcct ggagacacag     3637
   gattgagctc tacatggggc tccctgctca gcagggaatc tgagtctccc tctgtgcctc     3697
   cttctgttca tgcttgtgct ttctctctct ctctcaaata aataaataaa atctttaaaa     3757
   atatatatag gtatatataa ttattttttt aaatattttt gaagtagggg tgtacatgtg     3817
   tgtactcatt tgggtatgtg tatttatagc ctacttttca cttaacattg tatcatgaat     3877
   tttatatcat gtatcatttt aattactgca taataccaag gaaccacaat ttgtttaacc     3937
   aatccccatt gttggatata taggccccat ttttctctat tataaataaa actgtaatga     3997
   atcttccaaa tacatcactg gtcatttcct tagcataact tttcaggagt gtaatttgtg     4057
   ggttttaaaa ttaatggtaa tatctccaaa ttgccttgct gaggaattag ttggtacacc     4117
   aaccagcaga gtccctgttt cctttttgga tgactaaaaa tagtgagatg agcattttat     4177
   ttagaatata ataacccttt cagtttaat                                       4206
<210> 3
   <211> 899
   <212> PRT
   <213> shar-pei dog
<400> 3
   Met Asp Arg Tyr Leu Leu Leu Val Ile Trp Gly Glu Gly Lys Val Pro
   1               5                   10                  15
   Ser Ala Ala Gly Gly Glu Ala Glu Ser Ala Ala Glu Gly Thr Glu Lys
               20                  25                  30
   Leu Pro Asp Leu Thr Ala Ala Asn Val Tyr His Val Leu Lys Arg Ser
           35                  40                  45
   Ile Ser Ala Ser Met Asn Pro Glu Asp Ser Thr Phe Pro Ala Cys Ser
       50                  55                  60
   Val Gly Gly Ile Pro Gly Ser Lys Lys Trp Phe Phe Ala Val Gln Ala
   65                  70                  75                  80
   Ile Tyr Gly Phe Tyr Gln Phe Cys Ser Ser Asp Trp Glu Glu Ile Tyr
                   85                  90                  95
   Phe Gly Thr Glu Lys Asp Glu Ile Glu Asp Val Leu Gln Thr Asn Ile
               100                 105                 110
   Glu Glu Cys Leu Asn Thr Glu Cys Phe Glu Glu Glu Asp Ser Asn Ser
           115                 120                 125
   Arg Glu Ser Leu Ser Leu Ala Glu Leu Tyr Glu Glu Ser Ala Glu Asn
       130                 135                 140
   Leu His Gln Leu Ser Asp Lys Leu Pro Ala Pro Gly Arg Ala Met Ile
   145                 150                 155                 160
   Asp Ile Ile Leu Leu Pro Ser Asp Lys Asp Pro Pro Lys Leu Lys Asp
                   165                 170                 175
   Cys Leu Pro Thr Ile Gly Ala Leu Lys His Leu Lys Glu Trp Tyr Ser
               180                 185                 190
   Ala Lys Ile Thr Ile Ala Gly Asn His Cys Glu Ile Asn Cys Gln Lys
           195                 200                 205
   Ile Ala Glu Tyr Leu Ser Ala Asn Val Val Ser Leu Glu Asp Leu Lys
       210                 215                 220
   Asn Ala Ile Asp Ser Lys Glu Leu Trp Arg Gly Lys Ile Gln Ile Trp
   225                 230                 235                 240
   Glu Arg Lys Phe Gly Val Glu Ile Ser Phe Pro Glu Phe Cys Leu Lys
                   245                 250                 255
   Gly Val Thr Pro Lys Asn Phe Ser Thr Ser Asn Leu Asn Pro Cys Ile
               260                 265                 270
   Leu Thr Asn Lys Val Ile Pro Ser Lys Asp Lys Asn Ile Leu Pro Lys
           275                 280                 285
   Val Tyr His Tyr Tyr Gly Pro Ala Leu Glu Phe Val Gln Met Ile Lys
       290                 295                 300
   Leu Ser Asp Leu Pro Ser Cys Tyr Met Ser Asp Phe Glu Phe Glu Leu
   305                 310                 315                 320
   Gly Leu Thr Lys Asn Asn Thr Lys Gln Asn Ser Val Leu Leu Leu Glu
                   325                 330                 335
   Gln Ile Ser Ser Leu Arg Gly Lys Val Gly Ala Leu Phe Val Leu Pro
               340                 345                 350
   Cys Thr Ile Ser Asn Ile Ile Ile Pro Pro Pro Ser Gln Leu Ser Ser
           355                 360                 365
   Arg Lys Trp Arg Glu Tyr Ile Ala Lys Lys Pro Lys Thr Ile Ser Val
       370                 375                 380
   Pro Asp Val Glu Val Lys Gly Glu Cys Ser Ser Tyr Tyr Leu Leu Leu
   385                 390                 395                 400
   Gln Gly Asn Gly Asp Arg Lys Cys Lys Ala Thr Leu Ile His Ser Ala
                   405                 410                 415
   Asn Gln Ile Asn Gly Ser Phe Ala Leu Thr Leu Ile His Gly Lys Met
               420                 425                 430
   Lys Ala Arg Thr Glu Glu Ala Lys Leu Ser Phe Pro Phe Asp Phe Leu
           435                 440                 445
   Ser Leu Pro His Phe Ser Gly Glu Gln Leu Ile Gln Arg Glu Lys Gln
       450                 455                 460
   Leu Ala Asn Val Gln Ala Leu Ala Leu Lys Glu Cys Leu Lys Arg Arg
   465                 470                 475                 480
   Lys Leu Ala Lys Gln Pro Glu Ala Val Ser Val Asp Glu Phe Lys Ser
                   485                 490                 495
   Leu Leu Ile Leu Thr Arg Glu Ser Phe Leu Glu His Phe Asp Ala Val
               500                 505                 510
   Ile Pro Lys Thr Ile Pro Ile Lys Thr Asp Lys Ile Lys Thr Ser Asn
           515                 520                 525
   Met Leu Asn Asp Ala Ser Ser Val Glu Ser Thr Ser Ser Asn Leu Met
       530                 535                 540
   Glu Thr Asn Ala Leu Glu Trp Pro Glu Arg His Val Leu Gln Asn Leu
   545                 550                 555                 560
   Glu Ser Phe Glu Lys Ala Lys Gln Lys Met Arg Thr Gly Ser Leu Pro
                   565                 570                 575
   Arg Ser Ser Glu Gln Leu Leu Gly His Lys Glu Gly Pro Arg Asp Ser
               580                 585                 590
   Ile Thr Leu Leu Asp Ala Lys Glu Leu Leu Lys Phe Phe Thr Ser Asp
           595                 600                 605
   Gly Leu Pro Ile Gly Asp Leu Gln Pro Leu Gln Ile Gln Lys Gly Glu
       610                 615                 620
   Lys Thr Phe Val Leu Thr Pro Glu Leu Ser Pro Arg Lys Leu Gln Gly
   625                 630                 635                 640
   Leu Pro Phe Glu Lys Ala Ser Val Cys His Tyr His Gly Ile Glu Tyr
                   645                 650                 655
   Cys Leu Asp Asp Gln Lys Ala Leu Glu Arg Asp Gly Gly Phe Ser Glu
               660                 665                 670
   Leu Gln Ser Arg Leu Ile Arg Tyr Glu Thr Gln Thr Thr Cys Thr Arg
           675                 680                 685
   Glu Ser Phe Pro Val Pro Thr Val Leu Ser Pro Leu Pro Ser Pro Ala
       690                 695                 700
   Val Leu Ser Glu Pro Gly Ser Val Pro Asp Gly Glu Ala Leu Gln Ser
   705                 710                 715                 720
   Glu Leu Arg Thr Glu Val Ser Arg Leu Lys Arg Gly Ser Lys Asp Val
                   725                 730                 735
   Asn Cys Leu Tyr Pro Pro Lys Arg Leu Val Lys Ser Glu Ser Ser Asp
               740                 745                 750
   Ser Leu Leu Ser Gln Thr Ser Gly Asn Thr Asn Arg Arg His His Ala
           755                 760                 765
   Val Thr Ser Arg Lys Pro Arg Ala Glu Arg Ser Leu Ser Val Thr Cys
       770                 775                 780
   Pro Ser Val Pro Val Leu Ser Cys Glu Thr Ser Lys Val Thr Thr Lys
   785                 790                 795                 800
   Ala Ser Ser Gly Gln Lys Ser Val His Gly Ser Lys Thr Ser Arg Gln
                   805                 810                 815
   Ile Lys Glu Ser Arg Ser Gln Lys His Thr Arg Ile Leu Lys Glu Val
               820                 825                 830
   Val Thr Glu Thr Leu Lys Lys His Ser Ile Thr Glu Ala His Gly Cys
           835                 840                 845
   Phe Thr Ala Cys Ser Gln Arg Leu Phe Glu Ile Ser Lys Phe Tyr Leu
       850                 855                 860
   Lys Asp Leu Lys Thr Ser Arg Gly Leu Phe Glu Glu Met Lys Lys Ala
   865 870 875 880
   Ala Asn Asn Asn Ala Val Gln Val Ile Glu Trp Val Leu Glu Lys Ala
   885 890 895
   Ser Lys Lys
<210> 4
   <211> 16101
   <212> DNA
   <213> shar-pei dog
<400> 4
   gctccatgca gggagcctga tgtggcactt gatcctggga ctccaggatc atgccctgag      60
   ctgaaggcag gtgcttaccc gctgagccac ccaggcatcc caataaaatc ttttaaaaat     120
   aaataaataa aatactcttg gtgtaatgtt atgaaactgg actatttata cataatcgta     180
   tagtataaga tttaggcagt gatttctaaa ttgcccatat agtaaaaatt atatataaac     240
   tatattatat ataatatatg acatataaag aaatgttaca tatagatata gataactaga     300
   tatatgtctt cctgaatctt aatacaatga gtatttctaa atgtttacat ttgaaaaaaa     360
   tccacaggaa attatgagga acacttaaga ttgagaaaca ttgcccaaaa ggatattaat     420
   ctgcattgat atttctattg tggaataaca aaaattgaag gatgggacac attagtgtat     480
   gttaaatcac ttcgaaagat agatgcttat taaatatgtg taatagatca tgccttgcta     540
   cctgaaagtt ggaataatgc aaacttattc ctttaaataa agactattca ttacaagtaa     600
   tttttttaaa acttgacaat ggtttgatgt tagaatgctt ttaatttcaa gtaaatccat     660
   ccaacagtgg ctgaaacaaa taagggattg cttttttgtg cctgtctctt caaattacaa     720
   ggcatctgga gagagctagc tactggcagt ggttcaacag ctcagctgca tcacggtgac     780
   tgtcagatct tctcagcttt tacttacata tatacaaggt gaattgcaaa cttaggcatc     840
   atacccatgg attttaaggc agaaggaaag agcagtagct tcggggtact ctctaaagca     900
   aacgctcttc aaaaagcctc tgcttatgtt tcatttgtct agtgctgtgt taaaaagcta     960
   tccttggata aaagggaggc tggggagatc ctaaaaatcc ctaggcattg taaatgaaat    1020
   acctaaagga aataaagtgc tctacagact gcctaataga taataagact gccaaaagag    1080
   gcgttataaa accaaaaata acaacaatag caacaaaacc ccaaaaaaca aaaaactgta    1140
   ctcaggattt tcagttgtat ggtagcaata tctttcattg tatcaccatg aaaattaaat    1200
   aaaacaattt cagaaaaatt tattgagctc ttacagatat caggtagcct tggctttgga    1260
   gacaagttag aatagtacag agtctctatt cacagagctc actttctaat agggaaactg    1320
   agtgtaaatc aatagattca agaattgata aatccaggaa gaagacagaa gtaggttaaa    1380
   ttgaagaaag ggatttatgg ttgccaggga gagtggtggg taacaatggt tagggaagat 1440
   ctttctgagg cagaggaaga ccagattatt aggaagtagg acattcaaac aaaagtttta 1500
   tccaggaaag ggggttggat tttgtgtgta aaaacaaatt taagtgagtg tggctaaaaa 1560
   tcaattcata gagtaaagag tagtcatctg agccaactca ggcaaaccct gcctacaatt 1620
   aggggcctat atttgggtat agtggaacac cattggttaa tattttaatc agtggggttt 1680
   atgatttaag gaatgctttt tttttagaga taatgtttat tttattttac tttttaagat 1740
   ttttatttat ttatgagaga cacagaggga gagagagaga gaggccgaga cacaggcaga 1800
   gggagaagca ggctccattc agggagcccc atgtgggact caatcccggg tctccaggat 1860
   cacgccttgg gtcaaaggca ggcgccaaac tgctgagcca cccagggatc tctaaggaat 1920
   gctttttaaa aagataacca tagcttccct gtagaagata gaataaaaga atgcaatatt 1980
   agaggcaggg agaccaatat agctgggaaa tagaaatggc tcagattgta gaagtggtaa 2040
   taattagatt caagatatat tttgaaggta tatcctcctg gatcttctat ttattagatg 2100
   tacactatga aggaagtaga tgaattcaag ttgacttcta gattttcaga caaaacaact 2160
   gagtgaaaaa gagtgtcatt tactgagatt agtgaaagct tgtaggaaga gtcatttggg 2220
   ggcataggga ggaaagtagc tctgctcttg ccaatataat tttgatgtgc gtatagtact 2280
   cccaaaagca agcacaaaat agagggtatg catttatgac tatgcagctc aggagagatt 2340
   ttttagatta aaccattcaa accactgtag cagggcacaa aagtgaaaaa gaactgtgtg 2400
   cttgaaatgg tcctaaccat caaccattaa taaaagaaaa ggagtccaca agtctagaaa 2460
   tgtgggcaaa gattcacaac tgcagacagg ctcttcctga aatctgtagc atagaactaa 2520
   cgatgaggac acaaaggtgg tcaaccacag tgaaacccac aaaagtagaa taaaaaatgt 2580
   gtgaagtgtg tatgtaaaca cacgaaaagc aaagttgaaa gcatttttcc agtgactgtg 2640
   atagaagagt ctccagtgaa ttgctcatgt aataaagtca atggcattag aagaggaatt 2700
   gtctccgtgt tggtccaggc ctttcgtcag tttgaatagt tagtgcacaa catttcatgt 2760
   gcagttgtca tgtcgcaaag ggacaccacg tgacttgtgg gacatctcta tcaagaaacc 2820
   aacgaatcac tccacagatg ctgttcagag tgcttgcttg ctctgatggc tgccctacag 2880
   tgtctactta aaggcacctc agtggatccg ccacacaaac tgactagcta ctaaaagaat 2940
   acttgtgaat gatgtgaaaa aaaatcccaa aacttctgtt caaaccaatt tggttgatcc 3000
   tttcatattc tgaagcacac aaagaaggtt tcctgggagt aatttctgtt cagtgtccac 3060
   atttcataac atttattgga tttatgcaat tctcttgact cccccctgcc cccaaagcac 3120
   tttcatatgt gggcttactt tagactcagg acacttcacg gagatggata ggatagccat 3180
   attcatctgc ttttctgagc cagaagcttc ttcagaatgg gaaatactta aactatagcc 3240
   aaagaaacat ccattaatga cctgtggagg accggtttca gttggaacta cctgcatctg 3300
   gtcatatcct aacaccctaa aagtatgttc tttggcaaaa ttacttccaa tattagggat 3360
   ttggaattgc agccaaacat tcttacatat acagatatac tgacctggca gagatgttac 3420
   aggaaggggg tgaaatgtaa atccatacgg cagctataca atttacgtat tttgcaagat 3480
   gatgtattcc tccttaggaa attgagaaat aatagttact tcaaattatc ctcgaagatc 3540
   atactttatt aaaactaact gaacgattaa atttataata tttttctcct atacaataat 3600
   taaaagctgt ggctgcttct caaaaaaaaa aggagggggg aagaggggga agagaagaag 3660
   aaaataaatt tcaaaaagat tttgcctttg tttagatatt tttgtggtac ctttcattaa 3720
   tggccataaa taaaaacaat tcctgattat ttttcttcaa agttttaatt tttctcttta 3780
   tttaattata attagttgtg atcttcattt tctttcatat gaataagaga aagaaatttt 3840
   gaaagaaatt tttgaaattt ggaaagaaag aaaacagcac tacagagaaa ctgcatactg 3900
   catgtcttct tcttggaaat caatatttaa acaaatcaac ccatagacat atagcttgaa 3960
   aattacagat ttgtttttaa gcttcctgtt tttaagcctc taagactgag gtaccagaag 4020
   cacagttagc tattgaaaaa tctttacctc agaaaaaaaa atttattccc tgtcctcttt 4080
   tgccacacag tttgcgtgtg tttatgtgtg ttttttaatg tagagcccag ttgtgacata 4140
   tttgttttct tttgtattct cccaactggc tgaaaattag tcagatgaat aatctggcgt 4200
   cagtcttctg gtcccttttc tgaagcctgg tattagcagt ccagctcatt taaatctctc 4260
   cttcactcta acttcaagct ctctctgcag aactgtacct gagcaccatg tgctacttag 4320
   ttgcctaaaa gaaaaactct tttgcacaga aaaattacat ttattaagga acacacctaa 4380
   atactatcat ttctgataaa agtgacagtt ggaatcattt gagaaccatg cagaattttt 4440
   ctggcagcag ttacccttaa caaatgtact gcgatgctgt gtgctggatg tagattccca 4500
   tggaaaaggg agtagctcca aaatcctaat agtaccatgt ggtctggcca tgaactaaag 4560
   cttttcaaac catatggaaa ctcattactt caaagcactt ccacagtcac cttattttat 4620
   tattattttc acattagcgc tatagcataa gggtaaagtg gaaaaatatt cccagcttat 4680
   agatggggag acgaacatgc agcaatcatt gttaatttcc ttatttctcc taatgtcacc 4740
   aatattctct tggaagttta cctgttcatt actttttaat agaatcgatt gagctattac 4800
   taggatttat tctttcccct ccatttctgg cattaacatt ctagaccaca atttcttccc 4860
   acatgcctag attacttcca taactttcta gatttctccc tacctatgtt ctcttctcct 4920
   tttaggttgt gttcagaatt gatatagtta taaaagtttg gctcagtaaa aaaaacacaa 4980
   gaatgttcca aagaaaggga acatcagcct ggagtgttcc tgaggaacta caaaatgttt 5040
   agcttccctg gagattgtca tagaaatgta aagggttgtg taagatgaga ccataagata 5100
   aagtgaggag caggttatat atcggctttt acattccatt tcaaggaatg cttatgctga 5160
   aagcaaggac attttaagag ctttaggtct gaaaattgca taatcaaatg tggatgctta 5220
   caaaatcaac cacactcatg ctgtaaaatg aagaaaataa attagtccat tgggagactg 5280
   tacagtttgg gatgggaggt aattaatttg gaagcctgtc agtaatcagg ccaagtgatt 5340
   atggtattct gtactaaagt gctggcaatg gggtgagtaa aggtaagttg ctttaaaaat 5400
   atagaggaaa tagacttaaa acacatgaga agtaacctat taaaaaatca aaaataattc 5460
   ataaactcta acttaagcac tggttttttt ttaatatttt tatttattta tgatagtcac 5520
   agagagagag agaggcagag acacaggcag agggagaagc aggctccatg ccaggagcct 5580
   gatgtgggat tcgatcccgg gtctccagga tcgtgccccg ggccaaaggc aggcgccaaa 5640
   ccgctgcacc acccagggat cccaagcact ggtttttaat ggtgattttt cctcaatagc 5700
   gaacaacaag agttaggttc taacttggaa atgatgagcc tggaatgatt gggtacatcc 5760
   agatggggat gtctccagga cagtttaatg tacatgactg agggtcaaga aagatataca 5820
   cttaggtgtc atctgtataa atggagcagc tgttgaatga ttacacatat ggttgtgtgt 5880
   tatatgcagg atgttaaaaa gggtgctgtt tattttatct atcttctcta cttggggcac 5940
   caatttgggg tcaaatatat aaaacgattc tttatccaaa gtgggtaagt ttaattttac 6000
   taaagcgatg aaaaaggtgg tataaagcct aaaaattaaa tatacatagg tacttatgat 6060
   gaacaagaca ctgttcgaaa tcacttgctt tttgtgccat gtaatccact tagaaactca 6120
   acagtagtcc ccactttacc tcaaagacat aaaataaagg accatcattt acctagaagt 6180
   ccaagaatgg agccaaatca tgtatacaat ttcttgaaac agcatgatta attcacattt 6240
   attaatgatt actctatgtc tagcatgaca ctagatgctg tgtactgaag tctctttttt 6300
   agtttatatt aggttacact acataacccc aaataatgaa tggcacagtg aaatgtaaac 6360
   ataaatacat atatgcacag gtgcccaaag gaatctacaa ctcattggca gctaagctca 6420
   gtgtatttca gtccctcttt tctgagaagc acatagaagg gaaagccagc cacctgctcc 6480
   cagtgcccct gaaccctgat ccagaaacag cctgacctgg tgggatatga aaagtttatt 6540
   ctctaatagt gaacaaggtt tcaaaagagc ttcggtgaac tcctgtgatt tcctatccac 6600
   atttcattct taaggtaatt ctatttgaat ggccttttca aaatttaagt tctatattct 6660
   catgctattt cctcatctgg tttgttcagt tggacactca aaggtcttgt tctgattccc 6720
   cttacctagt tacatttgat aaattggcat ttcgccctca cttcagctag gctgaattac 6780
   actttgcatt gctgatcttt ctggggttgg gggttcatgc atttctttag tctccaggct 6840
   gctcctttgc ttctctactc tgagatccat tccccactct ccctgctttg ctctgtcatg 6900
   gaagtccgaa tgccgcacac tgcaattctg gatggtgcta attagcaaat gggaagtgtt 6960
   agcagtacga ggaggaaggg acaatctagg gtattttttc ccttcacttg gatttgggcg 7020
   ccagcctctg gggtggccgt gtctccccca ggattctaga ttttactgga tgtgctgctg 7080
   actcctgacc cctgttcttt ctatctcctt taactctcct gcccaggggt gttactagcc 7140
   ctctgctttg ttaatctctg gtttgtttca tttgtcgtct gattggcttt tcagccacca 7200
   aaacttttat gcctattttg ctgtatgcaa tgtcctccat tgaactactt ggtatgaacc 7260
   atgttttctt gatcaatccc agattagtat actttttcat ctacttctag ctgaggatct 7320
   tccagctgaa tttgaactga ataaagacaa gataatgata gtccaaaaat aagtattttc 7380
   cctatgatcc agccttgact tcttttccat gacactctct ttggttttcg ttattcttta 7440
   tattcctctc ttcttgactc ttttcttagg ttttcctcac ttattaggat tgcttttgct 7500
   catttattac tcatttgttt gttcatttgt tgccatatcc catgctcttc cattattggg 7560
   gttatgactt ttttatacca tttctagcac acatttgcca agatttttca ggctgtctta 7620
   caaaaaaggt taaaatgcag actttgtata ttcatttttc catagtagat gtcaatttta 7680
   ttttgagttc aaatatattt aatgtggaaa atacaactct taacttccat agcattccct 7740
   acaaaattgt tagctacagc tgtcagcaaa tgctcaagaa ttcaagagtg gcttttagat 7800
   ctctgctaaa agggtggcct ttattaacct ccttggcttt tggcttttgt aaagaaaaga 7860
   tgtacttcca atgaatcttg attcgtcaca cttgcacaga gtcatacaca cgcacactac 7920
   aaaaatacag caaaacaatt ttttctactt ttccttttgc attattaaat cattgtctct 7980
   gcctggtgtt tatttaacca cttttggcat atctacatat tagttataga aacatgtaac 8040
   acatcctgcc ttttaaggga tgtttatctt ttcttctttc tttcccgata aataatgaac 8100
   tttttgaata ccttaatccc acttgagtgt ctgacaatcc ttgcataaaa tttgcataca 8160
   gtaaatgtgc aataattgtt ggttaaatag aaataaatat aaaaacactt aaatgtaaaa 8220
   tgtttaaaat cagaagtaac tccttctcat tttggaatac tgttacctat tctttttttt 8280
   aatccatctt cagtagataa tgctattagg gctctgaaag taacaaatac tttcataata 8340
   ttttgcttga aaaatgcatg acccagattc cataagatta tgatggaaat atcttccagt 8400
   gcaaaacttg acgcatggca ggagatcaac aagtgaatgc tggttgaaaa aatgaattgc 8460
   cagtccctga gactgtctca tttgttttca taacttctac tttattcata tttttatccc 8520
   tttgaactct tagcaccaag tattttctca gtatgctttt acaaattgaa tgaatgagat 8580
   aactttcaca cgttgattct cacctacttt gattgctttg gagtctaaac caagtttcac 8640
   tggtggaaga atggaaaata ttataccatt acctaggaag tgttatgtgg atcctatctt 8700
   tgagtcatga attttacagc tggactttta tcatgtcatt agtagatggc tcatgatcta 8760
   attcctcctt ggagcacagg tagaggccca gtacactcca cagttatata cctcctatat 8820
   atagtttatt gtgagtattt gaaggcagaa atgtcatcct gttcatcttt gtgctttaaa 8880
   ggttcaagtc ccattacatt gcaccactgc tactctggcc ctctgaacat ttataagagt 8940
   cataggtata tagttgatat ataataaatg tttactaaat gaataattta ataaaataat 9000
   tgactcatgc aattgccctg aaaagcaggg gaatgaagca gccagcagta cttccaggaa 9060
   gcaactgaga aacacccttt ggcacccacc attcaatcaa cttaacatca gccttgaagc 9120
   ttaagaaata aaattaaggg ccaagtaagc atagctcaag ccaactactt ttgcattccc 9180
   caggccactg attgcctcac taaatctctc actttgctga atatgtgaaa tcaattatta 9240
   ttataggata caaaagacag aaactttttg gaaaattgtt ttgattatta ataaaagatg 9300
   acaatgtcaa ggcatgagtt accaccataa aactccacgt tagcattatt tgaattcagt 9360
   agaacaaaga caagtagatt tgtcaaatta caaccctgat tgtgtctcct atgcatctcc 9420
   attacccaaa ccagtgtaat gttctcaata gttgctttct tccaataggg gagcactgta 9480
   aggtcttata aaaataccta actttcaggt tcccacagaa actgagggaa tcaccccaga 9540
   tctaacctcc cactcacctt tgtcaacttc actgccattt tatttctttg atttctgccc 9600
   agtttcttgg cctctaactt cggtcctcca gaccagaaac ctttgaagca ctgtgcagac 9660
   tatctgttct tgactctgtg ccaaccttgg atattgcctt aaagatttgc ctgacttgat 9720
   ttccagcttg agaattagca tagctctact tttatcgtgg tgcccatctc tggtccttta 9780
   gaagctcaaa cttcaaccaa caaaggccca ggaccctttt gcttccccaa gaggaatgaa 9840
   tgttcaaaca gaatcatctc cttctgtaaa cagtgatctg tgtccttttc ctcaccctct 9900
   ttccccattt aaaacctctt tgtttgcata cttcatttgt ccagttctta ctgacagttc 9960
   cagactatct cagccatcaa ctcaggaagc tatactaacc aatttgtaac tgaacatctc 10020
   aagcatacct tgatatgcct ctcattttcc ccttatctcc tcctgcctca tatttgaata 10080
   atcttttaag gtataaatat gttatattaa ctctgttaat gactgtgatt atttttgaga 10140
   gctctagggg agctatatta tataattctt ggtatctctc actgtacttg cagagtgctt 10200
   tgcacacaag taaaccacag aatggtatac aaatgaatat ttaaatgttt agctgttttt 10260
   gaccaattat tcttaatatt ggcttcttat gacttattac cttgtgaaat attttgttag 10320
   ttgcatatct agaaaggttg ataaattcag atcagctcca ccattcttgt gtaattatta 10380
   ttattttcta aatgttcaat aatttaaata gtacttatgt acaattaatt cttgatttga 10440
   tctatggcat aggtctgtgc ccaggtgctt gggaaattga ctttgacatg gaggtttgga 10500
   tgcaggcggc taatagggaa ataaactagg caatagcacc agtgctagtg tgaaaaacaa 10560
   aggattcgac agaaaaagtt gaactccaat gcaagtgaaa atgaggcctt ggggcacctg 10620
   ggtggctcag gttgtgattc tggggtcctg ggatcgagtc ctacaacaga ctccctgcag 10680
   ggagcctgcc tctccctctg cttatgtctc tgcttctcct tctgtgtctc tcatgaataa 10740
   ataaataaaa tcttaaaata aataaaataa aaaatggggg ggggagggaa tggagcagcc 10800
   ataaggacta gttatatgag tgttggtttt ctcaaaagca tacttggaga aaatgttaac 10860
   tttttaatga tagatttaat atattaatat atgccaagta cttagtgtaa tgcttatcat 10920
   gtggtcactg ctcgctgaat tgaagttgcc tgaatctagc ttggaggcag gtgtgagtaa 10980
   gtcataaatt aactatgaaa tgacctttat ggctttagca actctataag atcccatagt 11040
   gaccacattc attattaagt aatctggtct taatcttgat aactgatttt gaatttatga 11100
   ctcctctttt actccctcat ctacatctct tcaactgcat tgttaatctt ttcagtcctg 11160
   tgcgtgtcat atcaacttcc tcccgttacc ttgtctcatg atgttctact ctattttctt 11220
   ccagaaagtg aactgtccct cctctctata aaatcacatc ttaaatgtcc tctctcccac 11280
   ttacataagg cttccttatg caaggattac tttgtttttt agacttcttt acctcctccg 11340
   aagtcttgca cttattaata tgtattactc ttttataaat caggattttg gagattaatt 11400
   taaatacagt aaaattcacc ttttgagttt gccaaatggc aaactgtcat tcaaccaaac 11460
   taccattaag ttgtagaaca tttcaactac catgaaaagt ttcctcataa ctatttgtgg 11520
   tcagtcccat cctcctccac cccccatacc tggtgaccac tagcggagtt tctgtcttca 11580
   cagttttgcc tttcccagaa tttcatttaa acagagtcat ataatatgtg ttttctggca 11640
   taatgctttt gaggtccatt cattgtttca tgtatcaata ggtcattcct tctaatggct 11700
   aagtagtatt ccattacatg gatataccac aatttgttta tatatttact acgttgatgg 11760
   gcctctggct tgatttcaat atttaactat tatgaataag tttatgtaaa tattaatata 11820
   cagatctttg tgtggaaaat gtttcaattt tttcccatta ataatgctga gggaaactgc 11880
   tggattatga ggtataattg tttaacatta cttgaaaatt ccaacttctt tttgtacata 11940
   tttgtactgt tttgagttat aaaaaactgt gtttctgttt aatcacaaac taattaaact 12000
   gggtaaatat ttttttaact ttagatacct acttgctata tagtaacacg tcggtgtggt 12060
   cttaattcgg attttactaa tgactaatga tgttgagcat tgcttccagc cagctatgta 12120
   tctcctttgg tggagtgtct gtgtaaatct tttgcccatt gaaaaaactg ggttattttt 12180
   tcttcttctt gattcctatg agtattcata tgttctaaat atgcctctat caaacaggta 12240
   ttttgaccat attttctctt catctgtagc ttgtctttac attctctaac agtccctttg 12300
   tgaagcagat tttatttatt tatttattta tttatttatt tatttattta ttattttttt 12360
   aaattttcat gagagacaca cagagggaga ggcagagaca ggcagaggga gagaagcagg 12420
   cttcatgcca ggagcctgat gtgggcctca atcccgggtc tctgggatca tgccctgggc 12480
   cgaaggcagg cgctaaactg ctgagctacc cagggatccc cctgtgaagc agattttaat 12540
   gttgattaag tccagttatt tttttctagg tttcctttgt gtcctattta aaatatcttt 12600
   gtctaatcaa aagtcacaaa gttgacaaag tcctcctgtt ttttctcctg gaagttgtac 12660
   agttttaatt tttatatttt taagtctgtg atacatttaa tttcttctat aaggtgtaag 12720
   gtaagggctg aggctcatat tttgcatgta gatgtgccat tgtttcagca ctctttgctg 12780
   aaaagactac ttttcccttc aaattacctg ggaagttttt tgtaaatcaa cagaatatat 12840
   acgtatggat gtataccttt cattatggat tagtgaaaag atcatgattt tggggttata 12900
   ctaacattta aatcctgtct ctatcatttt ctatgttttc taaggccatt tattatgaat 12960
   cagttcctta atctgaaaaa ataattaaaa tataaaattt ttgaggttta ataactgtaa 13020
   aataagcaag acatattaaa tttttagtgt ccaggcagta ctcaaaagtg gtatttgtgt 13080
   ttacttgttt ttttttgttg ttgttgtttt tttttttttt acttgttttt aacaattttc 13140
   aaatcttccc tttctagtta gatgagtagt ctcttaagtc agggactgtt ttgttttgtt 13200
   ttgttttttt ctaattctac aattacagcc acaatcttca gggtagatat tgtgtcctat 13260
   ggaggataag atactgaggg tggtttgaat tttcaaagaa gatgatgaag accatgacca 13320
   ctggctgtgt aagcaaatta ttaaattaat caaagttcag aggagatcaa gaatgttggg 13380
   ctatctctat gctccttaat catgtacata ctgctttgtt ttatcctctc tctgttcaat 13440
   aggacatgaa attcaacata aatcctactg atttagacca aagatcaata gcattttatt 13500
   cactataaat tgcttcatta ttttgcaggg tctccattct cataacctaa aattaaccgt 13560
   ttctttaaaa ttgcccaaat tccaaagtat aagccaaaca aaattgttga ttgctttggg 13620
   aagggtttta tgaagttaag aggagcagaa atacattgct ttcaaggaat ttgttattta 13680
   aaatgaatgc aaaaatgttt gcagaataga ttgttgaact gggagtcaaa ttagtttggt 13740
   tcaaattcca ctctgccttt gaataaaaag caagttgttg aactgcctta actgccaact 13800
   ttattatcca taaataggag ctaattcacc tcaaaaggcc tttgcatgca cgaatgagat 13860
   aataaatgtg aaagcacttt gtaatcacta acatcctata caaataaaag tcattgttat 13920
   tacgatgatt caaagccaac tctagaaaga atatgttatt tgttgatcaa tgaaaatgac 13980
   ccctggagac ctccttcccc tagtcaagga acactctact aagatgtaat actacctgtc 14040
   atttcaccat gtgcactgca ttgctttatg ttaagtaagc cctctcagat ttccttttaa 14100
   aatggcaata cctcctaaag agtaataatt aaagtcacag ctcagagtgc ataggtctca 14160
   aggactttct gttaaatacc tggcacctga gcaactttta catgtgtctt ttcagcagct 14220
   ctgactaggg cagcaaaaca taaaaacatg agtcctagaa gacttgaaaa gagtaaatgg 14280
   ggaaaaaaaa ccaacctaga aactagagaa tatgttagag agaggtagaa aagaacaaga 14340
   gtctctttcc aattgtccag ggtaagcctt gtaaatagca gacattaaag atatattgca 14400
   ctgataatta taaccagtat gtatttttta aattactgag gtataattga cataaataat 14460
   tgcttgtatt taagtggaca atgtgatgaa ttggggccta tgtagatgcc catgaaacca 14520
   ttaccacagt caagaaaatg aacaaaaccc tcaaccctct gcctactgca aatgtcatcc 14580
   ctctggttcc tcccattttc ccttccatgg cctacgtcca tcattccgta gtactttgaa 14640
   tttcttagca ttttatgaaa atagaatcat atgatatata ttctttaaaa aatctggttc 14700
   ctttttaaaa aagattttat ttatttgaga gagagaaaga gagtgcacaa gtggagagag 14760
   gagccaaggg agagggagaa gcagactccc cactgagcag gaagcttgat gggagggctc 14820
   aatcccagga cctggagacc atgacctgag ctgaaggcag acatttaacc aactgagcca 14880
   cccaggcacc caaatctggt tcctttcact ctgcacagtt attttgaggt tgatccaggt 14940
   tatactatgt atgaagttaa ttccttttta ttgctgaata gtattccatt gtatgactat 15000
   cagtttgttc ttctacatgt tgatggatgt ttggatgatg ccattgttta acttttatga 15060
   ataaagcttc tatgaacaca agcatacaac tctttctata gacacttgct ttcatttatc 15120
   aggtaactat ccaggagcaa gatagctgaa ttatatgtca agtatatttt taattttgta 15180
   agaactgttt tccaaagtgg ttacaccatt ttgcttgacc accagcagtt cattggagtt 15240
   ccaattcctc catatttctc caatacttgg tatggccagt gtttttttgt cttaaccatt 15300
   ctaataggca tgtagggata tcactatggt ttgatttaca tttcattaat gactaataat 15360
   atttagcatt atttgatgtg cttatttgcc aactatgtaa atcttctgtg gtgaaatgac 15420
   tttaaatttt tacctttttg ttggtttgtt ttatcttcac atcattggac tttgaaaatt 15480
   ccttattttg agtacaagtc tattatcagt taaatgcttt tcaaatattt tccccagtct 15540
   gtgacgtatc ttttccttct ctttgtagac tgttgaatct atttctggac tctattctgt 15600
   tcattgacct atttctctat ctttacacaa ataccacatt tttattacta tagccttaaa 15660
   ataagtctag aaatcggtag tatcagtcct ccaacatatt cctttctttc aaagtgagta 15720
   tggctatcct agattatctt tccctaagtg ttttaaatct gtttgttaat tcaacaacag 15780
   gaggaggagg aagagaattc tggtggagtt ttggttgtcg tttctttgaa tatttagatc 15840
   aatttggggg agaagtgaca taatgttgaa tcttccaatc taactatttc tgagcaataa 15900
   gtaaggcagt atgctatcgt ttagttttct tgtttcttgt gcttgaggtt cattgagttt 15960
   cttatatctg tgagttaata atttccatca aatttggaaa atttgcaggc attattcctt 16020
   caaatacatt ttctcctctc tccttcaagg gatccagttt tatgtatatt aaaccatttg 16080
   aaattgtccc actacttgct g 16101
<210> 5
   <211> 27
   <212> DNA
   <213> artificial sequence
<220>
   <223> primer
<400> 5
   atcttaaaac atcaaggggt ctattcg 27
<210> 6
   <211> 28
   <212> DNA
   <213> artificial sequence
<220>
   <223> primer
<400> 6
   gatcttaaaa catcaagggg tctattca 28
<210> 7
   <211> 29
   <212> DNA
   <213> artificial sequence
<220>
   <223> primer
<400> 7
   cagcattgtt gtttgctgct ttcttcatt 29

## Description

In a first aspect, the present invention relates to a method for analyzing a shar-pei dog for a genetic predisposition to develop Shar-Pei auto inflammatory disease (SPAID) or for identifying dogs suffering from SPAID. Namely, the present invention relates to identifying the missense mutation MTBP:g.19383758G>A (CanFam 3.1, CFA13:19383758) as a marker for genetic predisposition to develop SPAID or for identifying dogs suffering from SPAID. Further, the present invention relates to the use of said mutation for determining the genetic predisposition or for determining SPAID in said dog as well as to isolate nucleic acid sequences and probes useful in these methods. In addition, a kit or test system is provided for analyzing a genetic predisposition of a Shar-Pei dog to develop SPAID or for identifying dogs suffering from SPAID based on mutations in the MTBP gene, namely, determining the mutation of MTBP:g.19383758G>A.

### Prior Art

High-throughput technologies have been shown to play an increasingly important role in clinical research and diagnostics. In whole genome sequencing data various causative variants for disease traits have been detected in human as well as other mammals like cattle, horse and dogs. It has been shown that disease causing mutations can be breed or population specific and are often linked or promoted by characteristic features or phenotypic traits supported in breeding programs (Metzger J, et al., PLoS ONE 2015, 10(10):e0141514). In Shar-Pei dogs the development of a characteristically strongly wrinkled skin stabilized by deposits of hyaluronic acid were suggested to be involved in inflammatory processes resulting in a periodic fever (Olsson M, et al., PLoS Genet 2011, 7(3):e1001332). It was proposed that a 16.1 Kb duplication upstream of the hyaluronan synthase 2 (HAS2) on chromosome (CFA) 13 was associated with skin wrinkling and periodic fever in Shar-Pei dogs, see also EP2 397 565 B1. However, a direct relation of specific copy numbers detected by real-time PCR to Shar-Pei fever could not be confirmed (Metzger J, Distl O, Anim Genet 2014, 45.5: 763-764). Due to the variable type of clinical manifestation, the recurrent bouts of fever in Shar-Pei were supposed to represent only one of various clinical signs designated as a syndrome referred to as Shar-Pei autoinflammatory disease (SPAID). Additional signs of inflammation could be shown to be arthritis, dermatitis, otitis and systemic amyloidosis. It was proposed that genetic risk factors on CFA 13 and CFA 14 might trigger these inflammatory processes. Further droplet digital PCR (ddPCR) analysis of the 16.1 Kb copy number variation (CNV) upstream of HAS2 revealed stable CNV alleles in this region in contrast to continuous alleles previously detected by real-time PCR and suggested an association not only with fever but also with SPAID in general (Olsson M, et al., BMC Genomics 2016, 17(1):299). This CNV was proposed to explain 25% of the genetic heritability whereas the influence of further potential causative variants and triggers for SPAID was still unclear.

Shar-Pei are known to have a strong predisposition for the auto inflammatory disease clinically resembling some hereditary periodic fever syndromes in humans, such as Familial Mediterranean Fever (FMF). Both diseases are auto inflammatory, characterized by seemingly unprovoked episodes of inflammation. Fever symptoms in both FMF and SPAID are characterized by short (12-72 hours) recurrent bouts of high fever, accompanied by localized inflammation usually involving the joints, like the ankles in humans and the corresponding hock joint in dogs.

Patients with FMF and Shar-Pei with SPAID can be free of symptoms between the episodes that can occur as often as every few weeks. However, since acute phase reactants endure between episodes, a subclinical state and chronic autoinflammation might persist.

Recently, EP 2 397 565 B1 identifies a method for risk assessment of the development of Familial Shar-Pei fever (FSF) based on determining the copy number of a 16.1 duplication present upstream of the hyaluronic acid synthase 2 (HAS2) gene.

However, the approach described therein is expensive and further methods and tests are desired identifying specific markers accordingly.

Thus, an object of the present invention is the provision of a method allowing determination of the genetic predisposition to develop SPAID as well as a method for identifying dogs already suffering from SPAID accordingly.

### Summary of the present invention

In a first aspect, the present invention relates to a method for analyzing a Shar-Pei dog for a genetic predisposition to develop Shar-Pei auto inflammatory disease (SPAID) or for identifying dogs suffering from SPAID comprising obtaining sequence information from the Shar-Pei MTBP gene to determine a mutation on at least one allele wherein the mutation is indicative of the risk that the dog will develop or has SPAID as defined in claim 1.

The present inventors recognized that the specific gene, namely, the MTBP gene and the mutation MTBP:g.19383758G>A corresponding to a mutation of G to A at position 69870 (g.69870G>A) of SEQ ID No. 1 represents a suitable marker for identifying genetically predisposed Shar-Pei dogs.

In a further aspect, the present invention relates to the use of a mutation of the MTBP gene of the Shar-Pei dog, namely, of the mutation MTBP:g.19383758G>A for determining a genetic predisposition of developing SPAID in said dog, or for identifying SPAID in said dog.

Further, an isolated nucleic acid is provided said isolated nucleic acid molecule is for use in determining a genetic predisposition of Shar-Pei dogs in developing SPAID or for use in identifying dogs suffering from SPAID in a method according to any one of claims 1 to 8, characterized in that the isolated nucleic acid sequence includes the mutation to determined, with, the position 69870 (g.69870G>A) of SEQ ID No.1.

Moreover, in a further embodiment, an isolated probe is provided comprising a nucleic acid sequence of the MTBP gene of the Shar-Pei dog comprising the mutation MTBP:g.19383758G>A.

This isolated probe is useful for analyzing the genetic predisposition of a Shar-Pei dog for developing SPAID and/or for identifying Shar-Pei dogs suffering from SPAID and/or for identifying Shar-Pei dogs representing healthy dogs, in particular useful for breeding.

Finally, the present invention provides a kit or test system as well as its use for analyzing the genetic predisposition or for identifying the disease in a Shar-Pei dog.

### Detailed description of the present invention

In a first aspect, the present invention relates to a method for analyzing a Shar-Pei dog for a genetic predisposition to develop Shar-Pei auto inflammatory disease (SPAID) or for identifying dogs suffering from SPAID comprising obtaining sequence information from the Shar-Pei MTBP gene to determine a mutation on at least one allele thereof, wherein the mutation is indicative of the risk that the dog will develop or has SPAID as defined in claim 1.

The term "analyzing a genetic predisposition" or "determining a genetic predisposition" which are used herein interchangeably, identifies that the DNA of the dog is obtained and analyzed e.g. by sequencing, thus determining the nucleic acid sequence of a predetermined region or gene. The term "determining a mutation" identifies that based on the physical analysis of the sequence, the mutation is identified.

The term "mutation" and "alteration" is used herein synonymously as long as not identified otherwise.

The term "comprise" or "comprising" as well as the term "contain" or "containing" includes the embodiments of "consist of" or "consisting of".

The term "genetic marker" identifies a DNA sequence with a known localization on a chromosome which can be used for identifying individual or species. Typically, the genetic marker is a short DNA sequence, like a sequence having a sequence showing an exchange of a nucleotide base pair (semi-based pair exchange, SMP) or any other type of mutation or alteration.

The term "probe" refers to a nucleic acid molecule, typically, an oligonucleotide or polynucleotide, having a complementary base sequence of the bases to a region of nucleic acid and, thus, is able to hybridize to said region accordingly.

The term "nucleic acid molecule" refers to a molecule that is composed of single bases containing nucleotides. This term includes DNA as well as RNA molecules but also other molecules having these kind of bases but having other types of binding including PNA, LNA, TNA, and so on.

The term "oligonucleotide" refers to a nucleic acid molecule being of size in between 6 and 40 bases, like 10 to 30 bases. The term "polynucleotide" refers to a nucleic acid molecule containing more than 40 bases.

The present invention allows analyzing a Shar-Pei dog for a genetic predisposition to develop Shar-Pei auto inflammatory disease (SPAID) or for identifying dogs suffering from SPAID. That is, it has been recognized by the present inventors that a mutation in the Shar-Pei MTBP gene, namely, the mutation MTBP:g.19383758G>A, is a mutation being indicative of the risk that the dog will develop or has SPAID.

A mutation on at least one of the alleles of said gene represents Shar-Pei dogs having a genetic predisposition to develop SPAID or may already suffer from SPAID accordingly.

In contrast, Shar-Pei dogs not having a mutation in the MTBP gene, namely, not having the mutation MTBP:g.19383758G>A, can be regarded as healthy dogs, thus, representing healthy dogs with respect to SPAID.

The presence of said mutation on either copies of the MTBP gene, namely, of the mutation MTBP:g.19383758G>A on either alleles of the MTBP gene is an indicator for having a predisposition for developing SPAID or identifies dogs suffering from SPAID.

The mutation is the missense mutation MTBP:g.19383758G>A corresponding to a mutation of G to A at position 69870 (g.69870G>A) of SEQ ID No. 1.

SEQ ID No. 1 represents the genomic sequence of the MTBP gene while SEQ ID No. 2 represents the cDNA sequence encoding the MTBP protein accordingly. The missense mutation MTBP:g.19383758G>A is located at position 69870 (g.69870G>A) of SEQ ID No.1 or position 2623 (c.2623G>A) of SEQ ID No. 2.

The specific missense mutation MTBP:g.19383758G>A is also identified in the dog genome SNP database. In particular, the specific mutation MTBP:g.19383758G>A is identified further as rsnp_cf13019383758 while the transcript of the MTBP molecule is identified as ENSCAFT00000001477 and the gene is identified as EN-SCAFG00000000951.

Based on the method according to the present invention it is possible to classify the dogs into dogs having a predisposition to develop SPAID and dogs which represent healthy animals or healthy carriers with respect to SPAID. These healthy animals are particularly useful for breeding purposes. That is, with the method according to the present invention it is possible to identify dogs useful for breeding while avoiding the use of Shar-Pei dogs having a predisposition to develop SPAID. This is particularly useful in view of the fact that SPAID may develop at older ages. That is, the average age of developing SPAID is 2.7 years whereby only after 6 years all animals being predisposed to SPAID developed the same.

The skilled person is well aware of suitable methods for analyzing the Shar-Pei dogs accordingly. These methods include typical molecular biology based methods including PCR based methods and/or methods based on sequencing. Alternatively, restriction fragment length polymer polymorphism (RFLP) may be used. In an embodiment of the present invention, the analysis is conducted in a way that the genomic DNA or cDNA is amplified and, thereafter, sequenced by known methods. A particularly preferred embodiment of the method of the present invention includes the use of next generation sequencing (NGS) or droplet digital PCR (ddPCR). The skilled person is well aware of these methods and applying these methods for the analysis of the mutation in the MTBP gene accordingly.

Further, an isolated probe is provided comprising the mutation to be determined, the mutation MTBP:g.19383758G>A. The probe comprises the nucleic acid sequence as well as other molecules including marker like fluorescence molecules, radioactive molecules or other known labels useful for determining said probes in a sample accordingly. The skilled person is well aware of suitable marker molecules including 6-carboxyfluoresceine, carboxy tetramethyl-rhodamine, carboxy-x-rhodamine and Biotin.

Table S1 shows appropriate probe and primer sequences, annealing temperature and number of cycles for validation of MTBP:g.19383758G>A.

In addition, the present invention relates to a method according to the present invention further comprising the step of obtaining sequence information from the region of the nucleic acid located upstream of the Hyaluronic Acid Synthase 2 (HAS2) gene comprising the nucleic acid sequence consisting of bp 22,937,592 to bp 24,414,650 on the canine chromosome 13 according to CanFam 2.0 assembly (CanFam 2.0 Chr13: 22,937,592-24,414,650) and 19,900,518-21,377,996 according to CanFam 3.1 assembly (CanFam 3.1 Chr13:19,900,518-21,377,996) to determine the copy number of a 16.1 kb duplication consisting of the nucleic acid sequence of SEQ ID No. 4 or a sequence having more than 95% identity to SEQ ID No. 4 present in this region.
That is, in an embodiment both, the marker according to the present invention and the region described in EP 2 397 565 B1.

In another aspect, the present invention relates to the use of a mutation of the MTBP gene of the Shar-Pei dog, the mutation MTBP:g.19383758G>A (CanFam 3.1, CFA13: 19383758) for determining a genetic predisposition of developing SPAID in said dog, or for identifying SPAID in said dog.

The material or the sample to be used for analyzing the predisposition may be any sample obtained from the dog. For example, the sample may be blood or may be hair, hair root or semen or tissue containing DNA of said animal.

The DNA is obtained by suitable methods known to the skilled person. That is, the DNA is obtained for example by extraction known in the art and analyzed further e.g. by amplification and sequencing of the DNA of interest, namely, the MTBP gene.

Of course, other methods may be used for determining any mutation in the MTBP gene accordingly.

Further, an isolated nucleic acid molecule for use in determining a genetic predisposition of Shar-Pei dogs in developing SPAID or for use in identifying dogs suffering from SPAID in a method according to the present invention, characterized in that the isolated nucleic acid sequence includes the mutation to be determined, namely, the position 69870 (g.69870G>A) of SEQ ID No.1 is provided as well as a probe comprising a nucleic acid sequence of the MTBP gene of the Shar-Pei dog comprising the mutation MTBP:g.19383758G>A.

In another embodiment, the use of said isolated probe, in particular, in form of an isolated oligonucleotide is described. This probe is a probe comprising a nucleic acid sequence of the MTBP gene of the Shar-Pei dog comprising the mutation MTBP:g.19383758G>A containing at least 12 consecutive nucleotides of the MTBP gene, the MTBP cDNA or the complementary sequence thereto, for analyzing a genetic predisposition of Shar-Pei dogs for developing SPAID and/or for identifying Shar-Pei dogs suffering from SPAID and/or for identifying Shar-Pei dogs representing healthy dogs in particular useful for breeding.

The probe or isolated nucleic acid according to the present invention may be a probe containing the at least 12 consecutive nucleotides comprising the mutation to be determined, the mutation MTBP:g.19383758G>A.

The nucleic acid sequence may be a nucleic acid sequence containing consecutively at least 14 nucleotides, like 15, 16, 17, 18, 19, 20, 21, 21 or more nucleotides.

The primers to be used according to the present invention are primer pairs with primer sizes known in the art. For example, the primers are primers comprising at least 12 consecutive nucleotides of the sequence, like having a primer size of 13, 14, 15, 26, 17, 18, 19, 20, 21, 22 or more nucleotides.

In addition, the present invention provides a method for breeding Shar-Pei dogs comprising the analysis of the breeding animals for a genetic predisposition of SPAID according to the present invention. In case of the presence of at least a mutation in at least one of the alleles of the breeding animal, this animal is excluded from breeding while the breeder selects a suitable animal which has no mutation on both alleles of in the MTBP gene, namely, has not the mutation MTBP:g.19383758G>A in any one of the two alleles.

In an embodiment of the method of breeding, the offsprings of said breeding are analyzed for any genetic predisposition for SPAID. In case of a mutation in the MTBP gene as described herein, these animals are restricted in or excluded from any breeding programs.

These methods are suitable in particular for the breeding programs of the Shar-Pei dog whereby the analysis of the dogs may be achieved by using a sample derived from blood, hair, hair root or from semen of said dog.

The invention will be described further by way of examples without restricted thereto.

### Materials and Methods

### Animals

The genomic DNA of 164 Shar-Pei dogs and 162 dogs from different breeds as controls was isolated from EDTA-blood samples and adjusted to 10ng/µl. The health status of all Shar-Pei dogs was determined by clinical examination performed by accredited veterinarians (28 Shar-Pei) or by the owners observations recorded in a questionnaire.

In addition, in 132 study participants a supplementary form was filled in by dog owners and their veterinarians to specify the types of inflammatory processes. Specific parameters like the occurrence of fever, arthritis, skin vesicles, erythema, thickened and pasty skin, otitis, eye inflammation, recurrent intestinal inflammation and mast cell tumor were assessed in this form. Two of SPAID-affected Shar-Pei with severe clinical signs including recurrent bouts of fever and arthritis were chosen for whole-genome sequencing.

### Whole-genome sequencing

For library preparation genomic DNA of two Shar-Pei affected with recurrent bouts of fever was isolated from blood samples using Invisorb Spin Blood Mini Kit (STRATEC biomedical, Birkenfeld, Germany). DNA samples were enzymatically tagmented and fragmented using Nextera Library Preparation Kit (Illumina, San Diego, CA) and quality controlled on the Bioanalyzer High Sensitivity DNA Kit (Agilent, Santa Clara, California). NGS was performed on the Illumina MiSeq for 2x300 bp reads in paired-end mode. In total two runs with v3 Reagent Kits (15 Gb, Illumina) were carried out for each SPAID-affected Shar-Pei. We performed quality control using fastqc 0.11.5, mapping to the reference genome CanFam 3.1 (Ensembl) using BWA 0.7.13 and variant calling using SAMtools 1.3.1, Picard tools (http://picard.sourceforge.net, version 2.3.0) and GATK 3.5. Five fastq files from a Korean Jindo Dog (DRR001566), an Afghan Hound (SRR1061643), two German Shepherd dogs (SRR1130247, SRR1124304) and a Border Collie (SRR654728) derived from Sequence Read Archive (NCBI) were included in this study as controls. Variants with a read depth of 3-999 and quality values >20 were chosen for further analysis and investigated for their potential effects using SNPEff version 4.1 g.

### Selection of variants

Variants derived from whole genome sequencing analysis were filtered for homozygous mutant genotypes exclusively found in SPAID-affected Shar-Pei using SAS/Genetics, version 9.4 (Statistical Analysis System, Cary, NC). Variants were investigated for their potential effects on the protein using SIFT and PolyPhen-2. Those SNPs which were predicted to be deleterious by SIFT and also predicted to be probably damaging by PolyPhen-2 as well as two INDEL were chosen for validation.

### Validation

All nine variants were genotyped in all 102 SPAID-affected, 62 SPAID-unaffected Shar-Pei and 162 dogs of 11 different breeds. For six SNPs Kompetitive Allele Specific PCR (KASP, assays (LGC Genomics, Middlesex, UK) were designed and run on an ABI7300 real-time system for 96 well plates). Further three variants were genotyped by gel electrophoresis on an acrylamide gel using the LI-COR 4300 DNA Analyzer (LI-COR, Lincoln, Nebraska). Allele and genotype distributions and their association with SPAID were investigated applying the CASECONTROL procedure from SAS/Genetics. In addition, results from CNV (CNV_16.1) detection derived from previous analysis using an ABI 7300 real-time system were analyzed for correlations with MTBP:g.19383758G>A genotypes and SPAID status with PROC GLM procedure (SAS/Genetics).

### Sanger sequencing

Skin tissues for RNA isolation were available from an SPAID-affected Shar-Pei that was euthanized due to signs of severe renal dysfunction including sickness, diarrhea, fever and swollen joints. In addition skin tissues from an Irish Wolfhound and English Springer Spaniel which were euthanized due to orthopedic problems were used as reference samples (additional file 7). RNA was extracted according to standard protocols for the RNeasy Mini Kit (QIAGEN, Maryland, USA). Tissues were homogenized in QIAzol Lysis Reagent (QUIAGEN) using the Precellys homogenizer (Bertin Technologies, Montigny le Bretonneux, France). The obtained RNA was transcribed into cDNA using the Maxima First Strand cDNA Synthesis Kit (Fermentas, Thermo Fisher Scientific, Waltham, Massachusetts, USA). For Sanger sequencing primers were designed by Primer3 software (version 0.4.0, http://bioinfo.ut.ee/primer3-0.4.0/) and PCR reactions were run on a thermocycler TProfessional 96 (Biometra, Göttingen, Germany).

### ddPCR

Detection of CNV_16.1 was performed in 31 Shar-Pei using QX200 Droplet Digital PCR (ddPCR) System (BIO RAD, München, Germany). Primers, probes and reference gene were used according to previous analysis (Olsson M, et al., PLoS Genet 2011, 7(3):e1001332). The DNA was digested using Haelll restriction enzyme (New England Bio Labs, Ipswich, Massachusetts).

Table S1 shows appropriate probe and primer sequences, annealing temperature and number of cycles for validation of MTBP:g.19383758G>A by Kompetitive allele specific PCR (KASP).

### Results

### Phenotype

Clinical investigations and patient histories recorded by a questionnaire resulted in 102 Shar-Pei suspected to be affected with SPAID and 62 potentially unaffected dogs of all types (Table 1). In total 46 of the SPAID-suspected Shar-Pei and 11 SPAID-unaffected dogs could be shown to be older than six years and were therefore proposed to represent a reliable SPAID-phenotype. First signs of the disease could be observed in a range of one to twelve years in the individual dogs whereas the average onset of the disease was 2.7 years. Only three Shar-Pei developed first signs of inflammation at an age of older than six years. Specific investigations of SPAID signs revealed all kinds of combinations of the inflammatory types fever, arthritis, skin vesicles, erythema, thickened and pasty skin, otitis, eye inflammation and recurrent intestinal inflammations. In addition, in eight of nine Shar-Pei with signs of inflammation, mast cell tumors could also be found.

### Whole genome sequencing

Two severely SPAID-affected Shar-Pei with recurrent bouts of fever and acute joint inflammations were sequenced to investigate the whole genome for SPAID-associated variants in comparison to data from five reference dogs of different breeds from sequence read achieve (SRA). In total 31 missense variants, two disruptive in-frame insertions and deletions, one non coding transcript exon variant, two frameshift variants and one splice acceptor variant could be detected in filtering analysis for homozygous mutant genotypes exclusively found in SPAID-affected Shar-Pei. Six SNPs that were predicted to be deleterious (SIFT) as well as probably damaging (PolyPhen-2) and three insertions/deletions (INDEL) were chosen for further investigation of their association with SPAID, see table 2.

### Investigation of candidate variants

Genotyping of all nine potentially deleterious variants detected in whole genome sequencing data in 102 SPAID-affected and 62 SPAID-unaffected Shar-Pei revealed a slightly significant P-value for TGFBR3:g.57204844A>G (P=0.050), but a highly significant P-value for *MTBP*:g.19383758G>A (P=2.664E-06) (Table 2). Further validation of all variants in 162 dogs of 11 different breeds showed that none of the variants could be found in any other tested dog breed (data not shown). The missense mutation *MTBP*:g.19383758G>A was predicted to result in a deleterious (0.01; SIFT) as well as probably damaging (0.979; PolyPhen-2) substitution of glutamic acid to lysine located in the MDN2-binding protein, C-terminal domain. Further investigation of the genotypes in a subgroup of Shar-Pei older than six years revealed the disease associated genotype A/A exclusively in SPAID-affected dogs as well as the wild type genotype G/G only in SPAID-unaffected Shar-Pei (Table 3).

*MTBP*:g.19383758G>A was found to be located 1 Mb proximal of the copy number variation CNV_16.1 on CFA13. Further detection of CNV_16.1 by ddPCR revealed stable alleles of one or five copies. These copy numbers were in perfect cosegregation with the genotypes of *MTBP*:g.19383758G>A (Table 4).

### Candidate gene sequencing

Sanger sequencing results of *MTBP* (ENSCAFT00000001477 (see dog genome SNP database) in one SPAID-affected Shar-Pei and two control dogs revealed the candidate SNP *MTBP*:g.19383758G>A also known as rsnp_cf13019383758 in the complementary DNA in the affected Shar-Pei and confirmed the *MTBP gene* model predicted by Ensembl. In addition, we detected four SNPs which could not be exclusively found in the affected Shar-Pei, but also in SPAID-unaffected Shar-Pei.

### Discussion

Analysis of NGS data from two SPAID-affected Shar-Pei in comparison to five reference dogs resulted in 37 variants which could be exclusively found homozygous for the mutant allele in affected Shar-Pei. Further investigation of these variants revealed *MTBP*:g.19383758G>A on CFA 13 to be highly associated with SPAID in Shar-Pei. All SPAID-affected dogs harbored at least one mutant A-allele of this missense mutation. In SPAID-unaffected Shar-Pei only 23 dogs harbored the homozygous mutant genotype (A/A). These dogs were all younger than six years whereas none of the older dogs showed the A/A genotype. It is submitted that due to the variable onset of the disease SPAID-unaffected Shar-Pei with homozygous mutant genotype, which are younger than six years, might still develop signs of the disease. Shar-Pei at an age of at least seven years with no signs of the disease were proposed to be most likely healthy with regard to SPAID. It is submitted that a homozygous mutant genotype probably has a damaging effect on MTBP protein by the substitution of an acidic to an alkaline amino acid (like E -> K) in the MDN2-binding protein domain, and thus triggers SPAID. A heterozygous *MTBP*:g.19383758G>A genotype might increase the risk for the development of an autoinflammatory processes but might be compensated by other protein effects. MTBP was shown to play an important role in binding MDM2, an antagonist of p53 and mediator of inflammatory processes. It was shown that MTBP stabilizes MDM2 by preventing it from autoubiquitination and subsequently promotes an activation of the innate immune system. After the acute inflammatory response, MTBP induces apoptotic processes by p53-inhibition and therefore acts as a precursor for epithelial healing. A modified MTPB protein might increase MDM2 proinflammatory effect and thus support wrongly promoted excessive inflammatory reactions. It is submitted that this missense mutation is the potential causative trigger for SPAID. It is located near CNV_16.1 which has previously been suggested to increase SPAID-risk in Shar-Pei dogs and showed perfect coseggregation with *MTBP*:g.19383758G>A. Nevertheless, no gene could be found to be located in this CNV region which might be affected by an increased copy number. It was suggested that it might alter the expression of *hyaluronic acid 2* (*HAS2*) although no significant correlation of serum HA levels with the CNV could be found (Olsson M, et al., PLoS genet 2011, 7(3):e1001332.

**Table 1. Phenotypes of 164 Shar-Pei investigated for SPAID. The type, coat and intensity of wrinkles is shown in dogs of ≤6 years and >6 years.**

| Age | | ≤6 years | | >6 years | |
|---|---|---|---|---|---|
| Suspected SPAID- type | | SPAID-affected | SPAID-unaffected | SPAID-affected | SPAID-unaffected |
| Breed specific phenotype | | | | | |
| Type | bone mouth | 10 | 10 | 7 | 5 |
| | meat mouth | 46 | 41 | 39 | 6 |
| | | | | | |
| Coat | horse | 10 | 9 | 11 | 5 |
| | brush | 40 | 31 | 28 | 4 |
| | bear | 1 | 1 | 2 | 0 |
| | unknown | 5 | 10 | 5 | 2 |
| | | | | | |
| Wrinkles | few | 11 | 25 | 17 | 10 |
| | moderate | 40 | 25 | 23 | 1 |
| | pronounced | 5 | 1 | 6 | 0 |
| Total number of individuals | | 56 | 51 | 46 | 11 |

**Table 2. Case control test results for candidate variants for SPAID. SPAID-associated variants with predicted high or moderate effects (SNPEff) were genotyped for 102 SPAID-affected and 62 SPAID-unaffected Shar-Pei.**

| CFA | Polymorphism | MAF total | MAF controls | MAF cases | Allele od ds ratio | Chi-Square Genotype (Probability) | Chi-Square Allele (Probability) | Chi-Square Trend (Probability) |
|---|---|---|---|---|---|---|---|---|
| 1 | *CD79A*:g.112413114insGTGATG | 0.149 | 0.137 | 0.152 | 1.128 | 2.287 (P=0.319) | 0.136 (P=0.712) | 0.156 (P=0.693) |
| 6 | *C16orf96*:g.36821482G >T | 0.149 | 0.169 | 0.142 | 1.230 | 2.445 (P=0.295) | 0.442 (P=0.507) | 0.438 (P=0.508) |
| 6 | *EN-SCAFG00000024344*:g. 40648375C>T | 0.334 | 0.387 | 0.305 | 0.695 | 4.450 (P=0.108) | 2.311 (P=0.128) | 2.470 (P=0.116) |
| 6 | *RPAP2*:g.56637047del ACAA | 0.301 | 0.307 | 0.299 | 1.036 | 0.436 (P=0.804) | 0.020 (P=0.887) | 0.020 (P=0.887) |
| 6 | *TGFBR3:g.57204844A* >G | 0.455 | 0.436 | 0.465 | 0.886 | 6.180 (P=0.050) | 0.276 (P=0.599) | 0.267 (P=0.605) |
| 13 | *MTBP*:g.19383758G>A | 0.247 | 0.395 | 0.157 | 3.517 | 25.671 (P=2.664E -06) | 23.550 (P=1.217E-06) | 23.551 (P=1.217E-06) |
| 15 | *HCFC2*:g.42583230A> C | 0.408 | 0.395 | 0.422 | 0.896 | 0.193 (P=0.908) | 0.222 (P=0.637) | 0.192 (P=0.661) |
| 22 | *CLN5*:g.30574626C>T | 0.214 | 0.266 | 0.177 | 1.692 | 3.787 (P=0.151) | 3.732 (P=0.053) | 3.633 (P=0.057) |
| 37 | *OSGEPL1*:g.502225delCTTGTGCAA | 0.485 | 0.500 | 0.466 | 0.872 | 1.341 (P=0.511) | 0.364 (P=0.546) | 0.388 (P=0.533) |

**Table 3. Genotypic distribution of SPAID-associated missense mutation MTBP:g.19383758G>A. None of the affected Shar-Pei older than six years showed the wild type genotype G/G whereas none of the unaffected Shar-Pei of this group harboured the disease associated genotype A/A. Regardless of the muzzle type both genotypes G/G or A/A could be found in meat mouth and bone mouth Shar-Pei.**

| Phenotype | Number of Shar Pei (n) | Genotype G/G | Genotype G/A | Genotype A/A |
|---|---|---|---|---|
| **Age group (>6 years)** | | | | |
| SPAID affected Shar-Pei | 46 | 0 | 17 | 29 |
| SPAID unaffected Shar-Pei | 11 | 3 | 8 | 0 |

| **All age groups (1-14 years)** | | | | |
|---|---|---|---|---|
| SPAID affected Shar-Pei | 102 | 0 | 32 | 70 |
| SPAID unaffected Shar-Pei | 62 | 10 | 28 | 24 |

| **Muzzle types (>6 years)** | | | | |
|---|---|---|---|---|
| meat mouth | 45 | 1 | 17 | 27 |
| bone mouth | 12 | 2 | 8 | 2 |

| **Muzzle types (1-14 years)** | | | | |
|---|---|---|---|---|
| meat mouth | 132 | 3 | 41 | 88 |
| bone mouth | 32 | 7 | 19 | 6 |

**Table 4. Comparison of MTBP:g.19383758G>A genotypes with CNV_16.1. The results of CNV detection by digital droplet PCR show perfect cosegregation with MTBP:g.19383758G>A.**

| **All age groups (1-14 years)** | | | |
|---|---|---|---|
| SPAID affected Shar-Pei | 17 | 3 | 0 |
| SPAID unaffected Shar-Pei | 3 | 6 | 2 |
| *MTBP*:g.19383758G>A | A/A | A/G | G/G |
| CNV mean (RT-PCR) | 10.05 | 5.22 | 2.0 |
| CNV (ddPCR) | 10 | 6 | 2 |

| **Age group >6 years** | | | |
|---|---|---|---|
| SPAID affected Shar-Pei | 8 | 3 | 0 |
| SPAID unaffected Shar-Pei | 0 | 3 | 1 |
| *MTBP*:g.19383758G>A | A/A | A/G | G/G |
| CNV mean (RT-PCR) | 9.5 | 5.0 | 2.0 |
| CNV (ddPCR) | 10 | 6 | 2 |

**Table S1. Primer sequences and assays for the validation of MTBP:g.19383758G>A. Kompetitive Allele Specific PCR (KASP) assays were used for detection of genotypes. Primer pairs, amplicon size, annealing temperature and number of PCR cycles are shown.**

| CFA | Polymorphism | Forward primers) (5'-3') | Reverse primer (5'-3') | Validation type | amplicon size (bp) | annealing temperature (°C) | Nu mber of cycles |
|---|---|---|---|---|---|---|---|
| 13 | *MTBP*:g.19383758G >A | | | KASP | 50 | 61 | 26 |

## Claims

1. A method for analysing a Shar-Pei dog for a genetic predisposition to develop Shar-Pei auto inflammatory disease (SPAID) or for identifying dogs suffering from SPAID comprising determining the presence or absence of a mutation on at least one allele in the Shar-Pei MTBP gene, wherein the MTBP gene has the sequence of SEQ ID No. 1 and the mutation is the missense mutation MTBP:g.19383758G>A corresponding to a mutation of G to A at position 69870 (g.69870G>A) of SEQ ID No. 1 and wherein the presence of said mutation is indicative for the risk of developing or having SPAID.

2. The method according to claim 1 wherein the mutation is present on both alleles of the MTBP gene.

3. The method according to any one of the preceding claims wherein the presence of the mutation is indicative for a genetic disposition of developing SPAID at an age above 6 years.

4. A method according to any one of the preceding claims wherein the absence of the missense mutation MTBP:g.19383758G>A is indicative for a non-predisposition of developing SPAID.

5. The method according to any one of the preceding claims for selecting non-predisposed dogs for breeding.

6. The method according to any one of the preceding claims wherein the mutation is determined by PCR.

7. The method according to claim 6 wherein the mutation is determined by next generation sequencing or droplet digital PCR (ddPCR).

8. A method according to any one of the preceding claims further comprising the step of obtaining sequence information from the region of the nucleic acid located upstream of the Hyaluronic Acid Synthase 2 (HAS2) gene comprising the nucleic acid sequence consisting of bp 22,937,592 to bp 24,414,650 on the canine chromosome to determine the copy number of a 16.1 kb duplication consisting of the nucleic acid sequence of SEQ ID No. 4 or a sequence having more than 95% identity to SEQ ID No. 4. present in this region.

9. Use of a mutation of the MTBP gene of the Shar-Pei dog, namely, of the mutation MTBP:g.19383758G>A for determining a genetic predisposition of developing SPAID in said dog, or for determining SPAID in said dog, by the method of any of claims 1-8.

10. Probe containing at least 12 consecutive nucleotides of the MTBP gene, the MTBP cDNA or the complementary sequence thereto comprising a nucleic acid sequence of the MTBP gene of the Shar-Pei dog comprising the mutation MTBP:g.19383758G>A.

11. Use of a probe according to claim 10, for analyzing a genetic predisposition of Shar-Pei dogs comprising the mutation MTBP:g.19383758G>A for developing SPAID and/or for identifying Shar-Pei dogs suffering from SPAID and/or for identifying Shar-Pei dogs representing healthy dogs in particular useful for breeding.

12. The use of a kit or test system for analyzing a genetic predisposition of a Shar-Pei dog to develop SPAID or for identifying dogs suffering from SPAID, said kit comprising at least means for determining a genetic variation in the MTBP gene of said dog, whereby this genetic variation is at least the mutation of MTBP:g.19383758G>A, said means comprising a probe having a nucleic acid sequence of the MTBP gene with at least 12 consecutive nucleotides comprising the mutation MTBP:g.19383758G>A.

## Patentansprüche

1. Verfahren zum Analysieren eines Shar-Pei Hundes auf eine genetische Veranlagung zur Entwicklung einer Shar-Pei autoinflammatorischen Erkrankung (SPAID) oder zum Identifizieren von Hunden, die an SPAID leiden, umfassend die Bestimmung des Vorhandenseins oder des Fehlens einer Mutation auf mindestens einem Allele im Shar-Pei MTBP Gen, wobei das MTBP-Gen die Sequenz der SEQ ID Nr. 1 hat und die Mutation, die Missense-Mutation MTBP:g. 19383758G>A entsprechend einer Mutation von G zu A an der Position 69870 (g.69870G>A) der SEQ ID Nr. 1 ist, und wobei das Vorhandensein dieser Mutation indikativ für ein Risiko zur Entwicklung oder zum Vorliegen von SPAID ist.

2. Das Verfahren nach Anspruch 1, wobei die Mutation auf beiden Allelen des MTBP Gens vorhanden ist.

3. Das Verfahren nach einem der vorherigen Ansprüche, wobei das Vorhandensein dieser Mutation indikativ ist für eine genetische Disposition zur Entwicklung von SPAID in einem Alter von über sechs Jahren.

4. Ein Verfahren nach einem der vorherigen Ansprüche, wobei das Fehlen dieser Missense-Mutation (MTBP:g. 19383758G>A) indikativ ist für eine Nichtdisposition zur Entwicklung von SPAID.

5. Das Verfahren nach einem der vorherigen Ansprüche für die Auswahl nicht prädisponierter Hunde für die Zucht.

6. Das Verfahren nach einem der vorherigen Ansprüche, wobei die Mutation mittels PCR bestimmt wird.

7. Das Verfahren nach Anspruch 6, wobei die Mutation bestimmt wird durch next generation-Sequenzierung oder digitale Tröpfchen-PCR (ddPCR).

8. Ein Verfahren nach einem der vorherigen Ansprüche weiter umfassend den Schritt des Erhalts der Sequenzinformation aus der Region der Nukleinsäure, die sich stromaufwärts des Hyaluronsäure-Synthase 2 Gens (HAS2) liegt, umfassend die Nukleinsäure-Sequenz bestehend aus bp 22,937,592 bis bp 24,414,650 auf dem Chromosom des Hundes, um die Kopienzahl einer 16.1 kb Duplikation, bestehend aus der Nukleinsäure-Sequenz der SEQ ID Nr. 4 oder einer Sequenz die mehr als 95 % Identität zu SEQ ID Nr. 4 aufweist, die in dieser Region vorhanden ist, zu bestimmen.

9. Verwendung einer Mutation des MTBP Gens des Shar-Pei Hundes, nämlich der Mutation MTBP:g. 19383758G>A, zur Bestimmung einer genetischen Prädisposition zur Entwicklung von SPAID in diesem Hund oder zur Bestimmung von SPAID in diesem Hund durch ein Verfahren nach einem der Ansprüche 1 - 8.

10. Sonde enthaltend mindestens 12 aufeinander folgende Nukleotide des MTBP Gens, der MTBP cDNA oder der komplementären Sequenz davon, umfassend eine Nukleinsäure-Sequenz des MTBP Gens des Shar-Pei Hundes, umfassend die Mutation MTBP:g. 19383758G>A.

11. Verwendung einer Sonde nach Anspruch 10 zur Analyse einer genetischen Prädisposition von Shar-Pei Hunden umfassend die Mutation MTBP:g. 19383758G>A zur Entwicklung von SPAID und/oder zur Identifizierung von Shar-Pei Hunden die an SPAID leiden und/oder zur Identifizierung von Shar-Pei-Hunden die gesunde Hunde darstellen, insbesondere für die Zucht nützliche Hunde.

12. Die Verwendung eines Kit- oder Test-Systems zur Analyse einer genetischen Prädisposition eines Shar-Pei Hundes zur Ausbildung von SPAID oder zur Identifizierung von Hunden, die an SPAID leiden, dieses Kit umfasst mindestens ein Mittel zur Bestimmung einer genetischen Variation in dem MTBP-Gen dieses Hundes, wobei diese genetische Variation mindestens die Mutation MTBP:g. 19383758G>A ist, diese Mittel umfassend eine Sonde mit einer Nukleinsäure-Sequenz des MTBP-Gens mit mindestens 12 aufeinander folgenden Nukleotiden, umfassend die Mutation MTBP:g. 19383758G>A.

## Revendications

1. Procédé pour analyser un chien Shar-Pei pour une prédisposition génétique à développer la maladie auto-inflammatoire du Shar-Pei (SPAID) ou pour identifier des chiens souffrant de SPAID comprenant la détermination de la présence ou l'absence d'une mutation sur au moins un allèle dans le gène MTBP du Shar-Pei, dans lequel le gène MTBP a la séquence de SEQ ID N° 1 et la mutation est la mutation faux sens MTBP:g.19383758G>A correspondant à une mutation de G en A à la position 69870 (g.69870G>A) de SEQ ID N° 1 et dans lequel la présence de ladite mutation est indicative du risque de développer ou d'être atteint de SPAID.

2. Procédé selon la revendication 1, dans lequel la mutation est présente sur les deux allèles du gène MTBP.

3. Procédé selon l'une quelconque des revendications précédentes, dans lequel la présence de la mutation est indicative d'une disposition génétique au développement de SPAID à un âge supérieur à 6 ans.

4. Procédé selon l'une quelconque des revendications précédentes, dans lequel l'absence de la mutation faux sens MTBP:g.19383758G>A est indicative d'une non-prédisposition au développement de SPAID.

5. Procédé selon l'une quelconque des revendications précédentes, pour sélectionner des chiens non prédisposés pour la reproduction.

6. Procédé selon l'une quelconque des revendications précédentes, dans lequel la mutation est déterminée par PCR.

7. Procédé selon la revendication 6, dans lequel la mutation est déterminée par séquençage de nouvelle génération ou PCR numérique à gouttelette (ddPCR).

8. Procédé selon l'une quelconque des revendications précédentes comprenant en outre l'étape d'obtention d'informations de séquence de la région de l'acide nucléique situé en amont du gène d'acide hyaluronique synthase 2 (HAS2) comprenant la séquence d'acide nucléique constituée de pb 22 937 592 à pb 24 414 650 sur le chromosome canin pour déterminer le nombre de copies d'une duplication de 16,1 kb constituée de la séquence d'acide nucléique de SEQ ID N° 4 ou d'une séquence ayant plus de 95 % d'identité avec SEQ ID N° 4 présente dans cette région.

9. Utilisation d'une mutation du gène MTBP du chien Shar-Pei, à savoir, de la mutation MTBP:g.19383758G>A pour déterminer une prédisposition génétique à développer SPAID chez ledit chien, ou pour déterminer SPAID chez ledit chien, par le procédé selon l'une quelconque des revendications 1 à 8.

10. Sonde contenant au moins 12 nucléotides consécutifs du gène MTBP, de l'ADNc MTBP ou de la séquence complémentaire de celle-ci comprenant une séquence d'acide nucléique du gène MTBP du chien Shar-Pei comprenant la mutation MTBP:g.19383758G>A.

11. Utilisation d'une sonde selon la revendication 10, pour analyser une prédisposition génétique de chiens Shar-Pei comprenant la mutation MTBP:g.19383758G>A pour développer SPAID et/ou pour identifier des chiens Shar-Pei souffrant de SPAID et/ou pour identifier des chiens Shar-Pei représentant des chiens sains, en particulier utiles pour la reproduction.

12. Utilisation d'un kit ou d'un système d'essai pour analyser une prédisposition génétique d'un chien Shar-Pei à développer SPAID ou pour identifier des chiens souffrant de SPAID, ledit kit comprenant au moins un moyen pour déterminer une variation génétique dans le gène MTBP dudit chien, où cette variation génétique est au moins la mutation MTBP:g.19383758G>A,
ledit moyen comprenant une sonde ayant une séquence d'acide nucléique du gène MTBP avec au moins 12 nucléotides consécutifs comprenant la mutation MTBP:g.19383758G>A.
